# EUROPÄISCHE PATENTSCHRIFT

(11) **EP 2 398 787 B1**
(45) Veröffentlichungstag und Bekanntmachung des Hinweises auf die Patenterteilung: **30.01.2013**
(21) Anmeldenummer: 10703415.9
(22) Anmeldetag: 03.02.2010
(51) Int. Cl.: C07D 333/12, C09K 19/34, C09K 19/44, C09K 19/46, G02F 1/1337

(54) **Thiophenderivate und diese enthaltende FK-Medien**
Thiophene derivates and liquid crystal media comprising the same
Dérivés de thiophène et milieux de cristaux liquides les contenant

(30) Priorität: 19.02.2009 DE 102009009630
(43) Veröffentlichungstag der Anmeldung: 28.12.2011
(73) Patentinhaber: Merck Patent GmbH, 64293 Darmstadt (DE)
(72) Erfinder: JANSEN, Axel, 64293 Darmstadt (DE); PAULUTH, Detlef, 64372 Ober-Ramstadt (DE); HIRSCHMANN, Harald, 64291 Darmstadt (DE); CZANTA, Markus, 64285 Darmstadt (DE)
(86) Internationale Anmeldenummer: PCT/EP2010/000636
(87) Internationale Veröffentlichungsnummer: WO 2010/099853

(56) Entgegenhaltungen:
- EP-A1- 0 625 513
- EP-A2- 0 467 260
- DATABASE WPI Week 200767 Thomson Scientific, London, GB; AN 2007-711534 XP002585579 -& JP 2007 084487 A (ASAHI DENKA KOGYO KK) 5. April 2007 (2007-04-05) in der Anmeldung erwähnt

## Beschreibung

Die vorliegende Erfindung betrifft Thiophenderivate, Verfahren und Zwischenprodukte zu ihrer Herstellung, ihre Verwendung für optische, elektrooptische und elektronische Zwecke, insbesondere in Flüssigkristall (FK)-Medien und FK-Anzeigen, sowie diese enthaltende FK-Medien und FK-Anzeigen.

Flüssige Kristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur. Daneben gibt es auch Zellen, die mit einem elektrischen Feld parallel zur Substrat- und Flüssigkristallebene arbeiten, wie beispielsweise die IPS-Zellen ("in-plane switching"). Vor allem die TN-, STN- und IPS- Zellen, insbesondere die TN-, STN- und IPS-Zellen, sind derzeit kommerziell interessante Einsatzgebiete für die erfindungsgemäßen Medien.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur eine geeignete Mesophase besitzen, beispielsweise für die oben genannten Zellen eine nematische Mesophase. Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nicht-linearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität und geringem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFT's aus Verbindungshalbleitern wie z.B. CdSe oder TFT's auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays für Rechneranwendungen (Laptop) und im Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei low-volt-Mischungen war es bisher nicht möglich, sehr hohe spezifische Widerstände zu realisieren. Weiterhin ist es wichtig, dass der spezifische Widerstand eine möglichst geringe Zunahme bei steigender Temperatur sowie nach Temperatur- und/oder UV-Belastung zeigt. Besonders nachteilig sind auch die Tieftemperatureigenschaften der Mischungen aus dem Stand der Technik. Gefordert wird, dass auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Neben Flüssigkristallanzeigen, die eine Hintergrundbeleuchtung verwenden, also transmissiv und gegebenenfalls transflektiv betrieben werden, sind besonders auch reflektive Flüssigkristallanzeigen interessant. Diese reflektiven Flüssigkristallanzeigen benutzen das Umgebungslicht zur Informationsdarstellung. Somit verbrauchen sie wesentlich weniger Energie als hintergrundbeleuchtete Flüssigkristallanzeigen mit entsprechender Größe und Auflösung. Da der TN-Effekt durch einen sehr guten Kontrast gekennzeichnet ist, sind derartige reflektive Anzeigen auch bei hellen Umgebungsverhältnissen noch gut abzulesen. Dies ist bereits von einfachen reflektiven TN-Anzeigen, wie sie in z. B. Armbanduhren und Taschenrechnern verwendet werden, bekannt. Jedoch ist das Prinzip auch auf hochwertige, höher auflösende Aktiv-Matrix angesteuerte Anzeigen wie z. B. TFT-Displays anwendbar. Hier ist wie bereits bei den allgemeinen üblichen transmissiven TFT-TN-Anzeigen die Verwendung von Flüssigkristallen mit niedriger Doppelbrechung (Δn) nötig, um eine geringe optische Verzögerung (d · Δn) zu erreichen. Diese geringe optische Verzögerung führt zu einer meist akzeptablen geringen Blickwinkelabhängigkeit des Kontrastes (vgl. DE 30 22 818). Bei reflektiven Anzeigen ist die Verwendung von Flüssigkristallen mit kleiner Doppelbrechung noch wichtiger als bei transmissiven Anzeigen, da bei reflektiven Anzeigen die effektive Schichtdicke, die das Licht durchquert, ungefähr doppelt so groß ist wie bei transmissiven Anzeigen mit derselben Schichtdicke.

Für TV- und Videoanwendungen werden MFK-Anzeigen mit geringen Schaltzeiten benötigt. Solche geringen Schaltzeiten lassen sich besonders dann realisieren, wenn Flüssigkristallmedien mit geringen Werten für die Viskosität, insbesondere der Rotationsviskosität γ₁ verwendet werden. Verdünnende Zusätze verringern jedoch in der Regel den Klärpunkt und damit den Arbeitstemperaturbereich des Mediums.

Es besteht somit immer noch ein großer Bedarf nach MFK-Anzeigen mit sehr hohem spezifischen Widerstand bei gleichzeitig großem Arbeitstemperaturbereich, kurzen Schaltzeiten auch bei tiefen Temperaturen und niedriger Schwellenspannung, die diese Nachteile nicht oder nur in geringerem Maße zeigen.

Bei TN-(Schadt-Helfrich)-Zellen sind Medien erwünscht, die folgende Vorteile in den Zellen ermöglichen:
- erweiterter nematischer Phasenbereich (insbesondere zu tiefen Temperaturen)
- Schaltbarkeit bei extrem tiefen Temperaturen (out-door-use, Automobil, Avionik)
- erhöhte Beständigkeit gegenüber UV-Strahlung (längere Lebensdauer)
- kleine Schwellenspannung

Mit den aus dem Stand der Technik zur Verfügung stehenden Medien ist es nicht möglich, diese Vorteile unter gleichzeitigem Erhalt der übrigen Parameter zu realisieren.

Bei höher verdrillten Zellen (STN) sind Medien erwünscht, die eine höhere Multiplexierbarkeit und/oder kleinere Schwellenspannungen und/oder breitere nematische Phasenbereiche (insbesondere bei tiefen Temperaturen) ermöglichen. Hierzu ist eine weitere Ausdehnung des zur Verfügung stehenden Parameterraumes (Klärpunkt, Übergang smektisch-nematisch bzw. Schmelzpunkt, Viskosität, dielektrische Größen, elastische Größen) dringend erwünscht.

Bei FK-Anzeigen für TV- und Videoanwendungen (z.B. LCD-TV, Monitore, PDAs, Notebooks, Spielkonsolen) ist eine deutliche Verringerung der Schaltzeiten gewünscht. Es besteht deshalb ein Bedarf an Verbindungen für FK-Medien, die eine Verringerung der Schaltzeiten ermöglichen, ohne gleichzeitig die anderen Eigenschaften des FK-Mediums, wie beispielsweise den Klärpunkt, die dielektrische Anisotropie Δε oder die Doppelbrechung Δn zu verschlechtern. Hierfür sind insbesondere niedrige Rotationsviskositäten wünschenswert.

Bei Anwendungen für FK-Medien mit positiver dielektischer Anisotropie sind generell schnelle Schaltzeiten gefragt. Es ist bekannt, dass eine Verringerung der Schichtdicke d des FK-Mediums in der FK-Zelle theoretisch zu einer Verringerung der Schaltzeiten führt. Deshalb werden hierfür FK-Medien mit höheren Doppelbrechungswerten Δn benötigt, um eine ausreichende optische Verzögerung d·Δn zu gewährleisten. Andererseits zeigen jedoch FK-Medien mit höheren Doppelbrechungswerten typischerweise auch höhere Werte der Rotationsviskosität, was wiederum zu längeren Schaltzeiten führt. Die durch Verringerung der Schichtdicke erzielte Verkürzung der Schaltzeit wird somit durch die höhere Rotationsviskosität des verwendeten FK-Mediums zumindest teilweise wieder kompensiert. Es besteht deshalb ein dringender Bedarf an FK-Medien, welche gleichzeitig hohe Doppelbrechungswerte und niedrige Rotationsviskositäten aufweisen.

Der Erfindung liegt die Aufgabe zugrunde, Medien insbesondere für derartige MFK-, TN-, STN- oder IPS-Anzeigen bereitzustellen, welche die oben angegebenen gewünschten Eigenschaften besitzen und die oben angegebenen Nachteile nicht oder nur in geringerem Maße zeigen. Insbesondere sollten die FK-Medien schnelle Schaltzeiten und niedrige Rotationsviskositäten bei gleichzeitig hoher dielektrische Anisotropie und hoher Doppelbrechung aufweisen. Darüber hinaus sollten die FK-Medien einen hohen Klärpunkt, einen breiten nematischen Phasenbereich und eine niedrige Schwellenspannung aufweisen.

Es wurde nun gefunden, dass diese Aufgabe gelöst werden kann, wenn man erfindungsgemäße Thiophenderivate in FK-Medien, insbesondere in FK-Medien mit positiver dielektischer Anisotropie, und in MFK-, TN-, STN- und IPS-Anzeigen, verwendet. Diese Thiophenderivate führen zu FK-Medien mit den oben angegebenen gewünschten Eigenschaften.

In der JP 2007-084487 A1 werden Thiophenderivate offenbart, bei denen eine Thiophen-2,5-diyleinheit direkt oder über Brücken mit einer 2,3-Difluorphenylen-1,4-diyleinheit verknüpft ist. Diese Verbindungen besitzen jedoch eine negative dielektrische Anisotropie, und sind damit ungeeignet für Anwendungen in dielektrisch positiven FK-Medien. Weiterhin führt eine 2,3-Difluorphenylen-1,4-diyleinheit in der Regel zu vergleichsweise höheren Rotationsviskositäten als weniger fluorierte Phenyleneinheiten oder 2,5-Difluorphenylen-1,4-diyleinheiten.

Die EP 0 467 260 A2 offenbart Verbindungen mit einer Thiophen-2,5-diyleinheit, die direkt mit einer 2-und/oder 3-substiuierten Phenylen-1,4-diyleinheit verknüpft ist. Die der EP 0 467 260 A2 zugrundeliegende Aufgabe war jedoch die Entwicklung neuer Materialien für die Anwendung in ferroelektrischen FK-Anzeigen. Deshalb sind auch insbesondere ferroelektrische FK-Medien und ferroelektrische FK-Substanzen, sowie allgemein Verbindungen, die besonders für den Einsatz in ferroelektrischen FK-Anzeigen geeignete Eigenschaften aufweisen, Gegenstand der EP 0 467 260 A2.

Besonders FK-Medien mit einer chiralen smektisch C Phase (SmC*) sind für die Verwendung in ferroelektrischen FK-Anzeigen geeignet. Für die Herstellung solcher FK-Medien werden im Allgemeinen mesogene Verbindungen mit smektischen Phasen benötigt. Smektische Phasen werden bevorzugt dann ausgebildet, wenn das Grundgerüst der Flüssigkristallstruktur mit besonders langen Alkyl- oder auch Alkoxyseitenketten ausgestattet ist, wie in den besonders bevorzugten Ausführungsformen der EP 0 467 260 A2 offenbart. Smektische Phasen werden auch dann stabilisiert, wenn polare Carbonylfunktionen wie -C(O)-in der Seitenkette vorhanden sind, wie in einigen bevorzugten Ausführungsformen der EP 0 467 260 A2 offenbart. Zur Induktion einer SmC*-Phase werden chirale mesogene Verbindungen benötigt. Daher besitzen die Verbindungen der EP 0 467 260 A2 vorzugsweise verzweigte Alkylketten mit einem Chiralitätszentrum, das auch mit einer Esterfunktion kombiniert sein kann, wie z.B. in der Beispielverbindung I-40 auf Seite 20 der EP 0 467 260 A2:

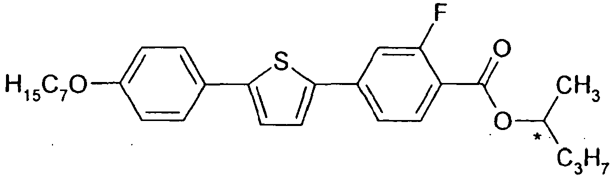

Ferroelektrische FK-Anzeigen sind heutzutage jedoch nur noch von untergeordneter Bedeutung. Für moderne Displayanwendungen werden fast ausschließlich nematische Flüssigkristallmedien verwendet. Die in der EP 0 467 260 A2 beschriebenen Verbindungen sind für solche modernen Displayanwendungen jedoch ungeeignet, da fast ausschließlich smektische Flüssigkristalle beschrieben werden.

Wie in den untenstehenden Vergleichsbeispielen gezeigt wird, besitzen auch die anderen Verbindungen aus der EP 0 467 260 A2, die keine Chiralitätszentren, Carbonyl- oder Carboxylfunktionen in der Seitenketten, sowie direkt verknüpfte Ringe aufweisen, nicht die für die Verwendung in erfindungsgemäßen FK-Anzeigen erforderlichen günstigen Eigenschaften.

So ist die Beispielverbindung I-1 auf Seite 12 der EP 0 467 260 A2 nur in einem sehr begrenzten Temperaturbereich (K 115 N 119 I) nematisch:

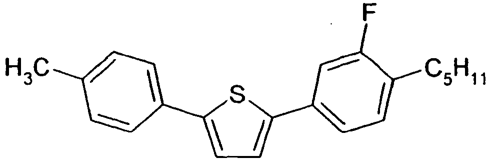

Zudem zeigt diese Verbindung schlechte Werte für den Klärpunkt (145 °C) und die Rotationsviskosität (196 mPa·s), was durch die aufgrund der zentralen Thiopheneinheit insgesamt gewinkelte Struktur des Moleküls erklärt werden kann.

Die Beispielverbindung I-134 auf Seite 38 der EP 0 467 260 A2 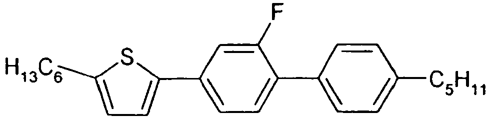 weist einen breiten Bereich smektischer Phasen auf (K 44 Sm (6) SmB (38) SmC 68 SmA 75 N 93,5 I).

Die Beispielverbindung I-136 auf Seite 39 der EP 0 467 260 A2 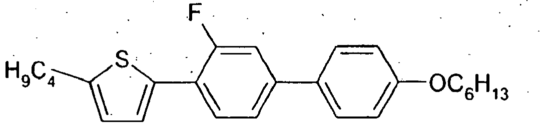 besitzt ebenfalls einen breiten Bereich smketischer Phasen (K 66 SmE 89 SmC 131 SmA 144 N 146,4 I) und ein ungünstiges Rotationsviskosität zu Klärpunkt-Verhältnis (Clp.: 179 °C, γ₁: 262 mPa·s).

Weitere Beispielverbindungen der EP 0 467 260 A2 sind z.B. aufgrund des Vorhandenseins von Keto- und / oder Esterfunktionen ungeeignet für den Einsatz in modernen MFK-Anzeigen, da diese funktionellen Gruppen zu einer schlechten Reliability, insbesondere zu einer schlechten Voltage Holding Ratio (VHR) führen. Zudem sind Verzweigungen, z.B. innerhalb einer Alkylseitenkette wie sie für chirale smektische Flüssigkristalle notwendig sind, für nematische Flüssigkristalle generell unerwünscht, da dies zu einer höheren Rotationsviskosität und somit zu schlechten Schaltzeiten führt.

In A. S. Matharu et al., Liquid Crystals 2007, 34, 489-506 werden lateral fluorierte Flüssigkristalle mit 2,2'-Bithiopheneinheiten beschrieben, daruner auch Verbindungen der folgenden Formeln 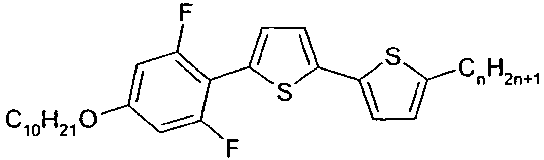 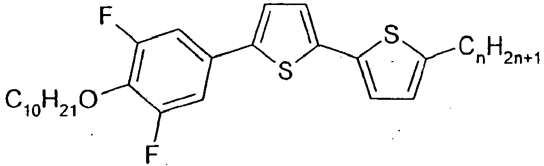 wobei Substanzen mit n = 6-10 synthetisiert und untersucht wurden. Die Substanzen weisen aufgrund der Länge der Alkyl- bzw. Alkoxyseitenketten und der zentralen Thiopheneinheit schlechte Phaseneigenschaften auf. So werden bei niedrigen Klärpunkten lediglich schmale monotrope smektisch A-Phasen ausgebildet (z.B. für n = 6: K 30,1 SmA (24,8) I).

In der EP 0 499 252 A1 und einer Reihe ähnlicher Veröffentlichungen EP 0 459 406 A1, EP 0 476 567 A1, EP 0 500 072 A1, JP 06-025668 A werden Thiophenderivate der folgenden Formel 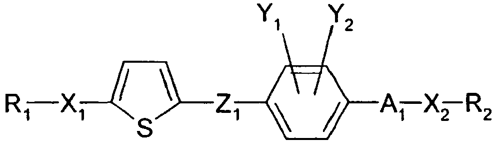 beschrieben, wobei Z₁ COO, CH₂O oder CH=C(CN) bedeutet, und Y₁ und Y₂ neben anderen Bedeutungen auch F bedeuten können. Als konkrete Beispiele werden folgende Verbindungen beschrieben (siehe z.B. EP 0 499 252 A1, Verbindungen I-110 auf Seite 32 und I-130 auf Seite 35):

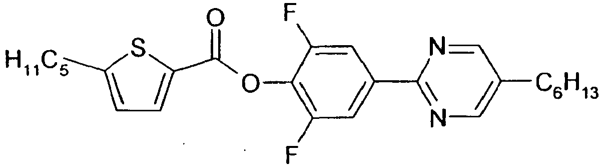

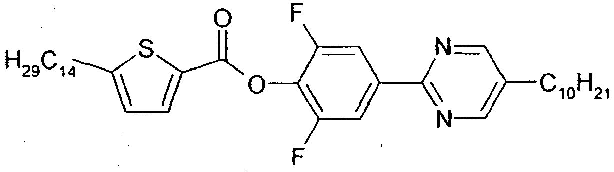

Auch diese Dokumente zielen auf die Entwicklung neuer Materialien für die Anwendung in ferroelektrischen FK-Anzeigen ab. Damit weisen auch die darin beschriebenen Verbindungen die oben erwähnten Nachteile (z.B. smektische Phasen, schlechte VHR durch Estergruppen) in Bezug auf Ihre Verwendung in modernen, nematischen Flüssigkristallmedien auf.

In der GB 2 388 841 A werden spezielle polymerisierbare Mesogene der folgenden Formel 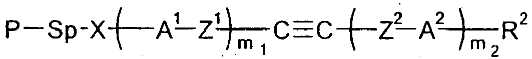 beschrieben, wobei P eine polymerisierbare Gruppe, X eine Einfachbindung und Sp eine Abstandsgruppe oder eine Einfachbindung bedeutet, und -A¹-Z¹- unter Anderem auch folgende Gruppe 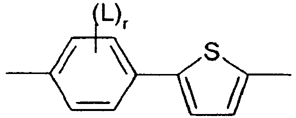 bedeuten kann, worin L unter Anderem F und r 0, 1, 2, 3 oder 4 bedeutet. Konkrete Verbindungen mit Thiophenringen werden jedoch nicht beschrieben. Weiterhin ist die Möglichkeit zur Verwendung dieser Materialien in Flüssigkristallmischungen für Displays (Active Matrix Displays, TN, STN, IPS, VA etc.) erwähnt. Die Verwendung monoreaktiver Mesogene in Flüssigkristallmedien moderner Displays erfolgt jedoch eher untergeordnet.

In der GB 2 229 179 A werden Alkylthiophene vom Typ der Formel 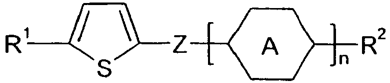 beschrieben, wobei R¹, R² unter Anderem Alkylreste mit bis zu 16 Kohlenstoffatomen oder CN oder F bedeuten können, Z eine COO-, OCO-, CH₂CH₂- oder CH₂O-Brücke bedeutet, A unter Anderem ein fluorierter Phenylrest sein kann, und n 1 oder 2 bedeuten kann. Die Aufgabe der dort beschriebenen Erfindung war es jedoch, Verbindungen mit moderater bzw. niedriger Doppelbrechung zu erhalten. Dazu wurde die Konjugation zwischen der Thiopheneinheit und aromatischen Einheiten A bewusst durch Einführung von nicht zur Konjugation befähigten Brückengliedern unterbrochen. Für moderne TN-TFT-Displayanwendungen werden allerdings bevorzugt Komponenten benötigt, die eine höhere Doppelbrechung mit einem guten Rotationsviskosität zu Klärpunktverhältnis vereinen.

Wie zuvor schon erläutert wurde, wirken sich weiterhin die hier besonders bevorzugten Estergruppen negativ auf die Reliability von Flüssigkristallen aus. Beispiele für Komponenten mit Ethylenbrücken bzw. CH₂O-Gruppen zwischen einer Thiopheneinheit und einem weiteren aromatischen Ringelement werden nicht offenbart. Weiterhin wird nicht auf das Fluorierungsmuster eines mit der Thiopheneinheit verbrückten aromatischen Ringsystems eingegangen.

In der WO 2004/029697 A1 und WO 02/093244 A2 werden Flüssigkristallmischungen enthaltend Verbindungen der Formel 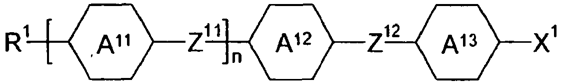 beschrieben, wobei die Ringelemente A¹¹, A¹² und A¹³ unabhängig voneinander z.B. 1,4-Phenylen, 3,5-Difluor-1,4-phenylen oder 2,6-Difluor-1,4-phenylen bedeuten können und A¹² Thiophen-2,5-diyl bedeuten kann. X¹ ist eine polare Gruppe F, OCF₃, OCF₂H, Cl, CN, C≡C-CN, NCS, bevorzugt CN, C≡C-CN, NCS. Diese Dokument richtet sich jedoch insbesondere auf die Verwendung hochpolarer Einzelverbindungen in Flüssigkristallmischungen die insbesondere für die ältere STN-Technologie geeignet sind. Als konkretes Beispiel wird die folgende Verbindung genannt (siehe WO 2004/029697 A1, Seite 47 bzw. 58, Formel I-2c-16 mit n=4): 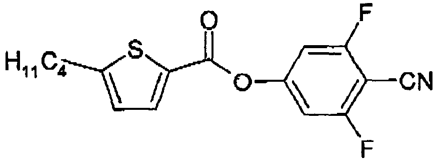 Jedoch sind sowohl die bevorzugten polaren Gruppen als auch die Estergruppe nicht geeignet, um Materialien mit ausreichender Reliability zu erhalten. Zudem ist der für diese Verbindung beschriebene Klärpunkt (Tg -54 X1 -35 X2 -7 I, X ist eine nicht weiter bestimmte flüssigkristalline Phase) äußerst niedrig.

In der WO 2001/064814 A1 werden auf Seite 29 (Formel IB4) Verbindungen der folgenden Formel 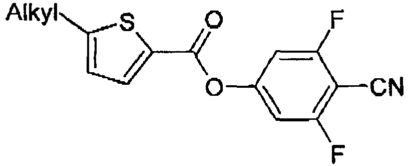 beschrieben, wiederum für die Verwendung in STN-Mischungen.

In der EP 0 625 513 A1 werden Thiophenderivate als Komponente von flüssigkristallinen Mischungen offenbart, die neben dem Thiophenring einen optional substituierten, gegebenenfalls auch fluorierten Benzolring aufweisen,

Es war deshalb für den Fachmann aufgrund des Standes der Technik nicht zu erwarten, dass die Verwendung von erfindungsgemäßen Thiophenderivaten in achiralen nematischen FK-Medien mit einer inhärent unverdrillten Phase, insbesondere in FK-Medien mit positiver dielektischer Anisotropie, und in MFK-, TN-, STN- und IPS-Anzeigen, zu einer Verbesserung der Eigenschaften, insbesondere zu schnellen Schaltzeiten und niedrigen Rotationsviskositäten bei gleichzeitig hoher dielektrischer Anisotropie, hoher Doppelbrechung und hohem spezifischen Widerstand, führen kann.

Gegenstand der vorliegenden Erfindung sind somit Verbindungen der Formel I 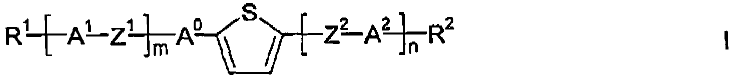 worin die einzelnen Reste folgende Bedeutung besitzen
- R¹ und R²: H, F, Cl, Br, -CN, -SCN, -NCS, SF₅ oder geradkettiges oder verzweigtes Alkyl mit 1 bis 12 C-Atomen, worin auch eine oder mehrere nicht benachbarte CH₂-Gruppen jeweils unabhängig voneinander durch -CH=CH-, -C≡C-, -O-, -CO-, -CO-O-, -O-CO-, -O-CO-O- so ersetzt sein können, dass O-Atome nicht direkt miteinander verknüpft sind, und worin auch ein oder mehrere H-Atome durch F, Cl oder Br ersetzt sein können, oder P-Sp-,
- P: eine polymerisierbare Gruppe,
- Sp: eine Abstandsgruppe oder eine Einfachbindung,
- A⁰: 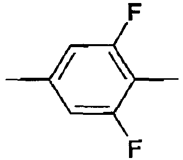
- A¹ und A²: jeweils unabhängig voneinander Phenylen-1,4-diyl, worin auch eine oder zwei CH-Gruppen durch N ersetzt sein können und ein oder mehrere H-Atome durch Halogen, CN, CH₃, CHF₂, CH₂F, OCH₃, OCHF₂ oder OCF₃ ersetzt sein können, Cyclohexan-1,4-diyl, worin auch eine oder zwei nicht benachbarte CH₂ -Gruppen unabhängig voneinander durch O und/oder S ersetzt sein können, und ein oder mehrere H-Atome durch F ersetzt sein können, Cyclohexen-1,4-diyl, Bicyclo[1.1.1]pentan-1,3-diyl, Bicyclo[2.2.2]octan-1,4-diyl, Spiro[3.3]heptan-2,6-diyl, Tetrahydropyran-2,5-diyl, oder 1,3-Dioxan-2,5-diyl,
- Z¹ und Z²: jeweils unabhängig voneinander -CF₂O- -OCF₂-, -CH₂O- -OCH₂-, -CO-O-, -O-CO-, -C₂H₄- -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- oder eine Einfachbindung,
- m und n: jeweils unabhängig voneinander 0, 1, 2 oder 3.

Ein weiterer Gegenstand der Erfindung ist ein FK-Medium, vorzugsweise ein FK-Medium welches bei Raumtemperatur eine, vorzugsweise achirale, nematische Phase aufweist, enthaltend eine oder mehrere Verbindungen der Formel I.

Weiterer Gegenstand der Erfindung sind neue Verfahren zur Herstellung von Verbindungen der Formel I, sowie darin erhaltene bzw. verwendete Zwischenprodukte.

Weiterer Gegenstand der Erfindung ist die Verwendung von Verbindungen der Formel I und erfindungsgemäßen FK-Medien in elektrooptischen Anzeigen, insbesondere FK-Anzeigen.

Weiterer Gegenstand der Erfindung ist eine FK-Anzeige enthaltend eine oder mehrere Verbindungen der Formel I oder ein erfindungsgemäßes FK-Medium, insbesondere eine MFK-, TN-, STN- oder IPS-Anzeige.

FK-Medien, die ohne Gegenwart von chiralen Dotierstoffen eine achirale FK-Phase aufweise, sowie Verbindungen der Formel I, worin die Reste Z^{1,2}, A^{1,2}, R^{1,2} kein Chiralitätszentrum aufweisen, sind generell bevorzugt.

Ferner bevorzugt sind Verbindungen der Formel I, worin m und n 0, 1 oder 2, besonders bevorzugt 0 oder 1 bedeuten. Besonders bevorzugt sind Verbindungen der Formel I, worin n 0 bedeutet. Weiterhin bevorzugt sind Verbindungen der Formel I, worin m 0, 1 oder 2, vorzusgweise 1 oder 2, ganz besonders bevorzugt 1 bedeutet.

A¹ und A² in Formel I bedeuten besonders bevorzugt Phenylen-1,4-diyl, welches auch ein oder mehrfach durch F substituiert sein kann, ferner Cyclohexan-1,4-diyl, Tetrahydropyran-2,5-diyl, oder 1,3-Dioxan-2,5-diyl.

Z¹ und Z² in Formel I bedeuten besonders bevorzugt CF₂O oder eine Einfachbindung, insbesondere eine Einfachbindung.

Besonders bevorzugt bedeuten A¹ und A² in Formel I 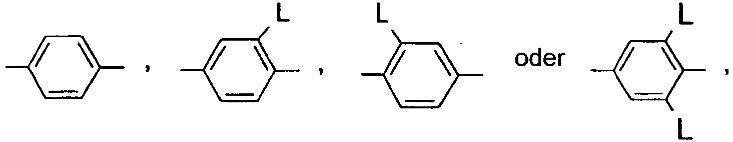 bevorzugt unsustitueirtes 1,4-Phenylen,
worin L Halogen, CF₃ oder CN, vorzugsweise F bedeutet.

Ferner bevorzugt sind Verbindungen der Formel I, worin R¹ und R² jeweils unabhängig voneinander H, F, Cl, Br, -CN, -SCN, -NCS, SF₅, P-Sp-, Halogen oder optional durch Halogen, insbesondere durch F, substituiertes Alkyl, Alkenyl oder Alkinyl mit 1 bis 8, vorzugsweise 1 bis 5 C-Atomen bedeuten.

Besonders bevorzugte Reste R¹ und R² bedeuten H, Halogen, oder, optional durch Halogen, insbesondere durch F, substituiertes Alkyl, Alkenyl, Alkinyl oder Alkoxy mit 1 bis 12, vorzugsweise 1 bis 8 C-Atomen, besonders bevorzugt H, F, Alkyl, Alkenyl oder Alkinyl mit 1 bis 8 C-Atomen. Ganz besonders bevorzugt ist R¹ gleich Alkyl. Ferner bevorzugt ist R² H, Alkyl oder Fluor. Ganz besonders bevorzugt ist R¹ Alkyl und R² H oder Alkyl. R¹, R² bedeuten jeweils unabhängig voneinander ganz besonders bevorzugt unverzweigtes Alkyl mit 1-5 C-Atomen. Falls R¹ und R² substituiertes Alkyl, Alkoxy, Alkenyl oder Alkinyl bedeuten, beträgt die Gesamtzahl der C-Atome in beiden Gruppen R¹ und R² vorzugsweise weniger als 10.

Bevorzugte Alkylgruppen sind beispielsweise Methyl, Ethyl, n-Propyl, n-Butyl, n-Pentyl, n-Hexyl, n-Heptyl und n-Octyl.

Bevorzugte Alkenylgruppen sind beispielsweise Ethenyl, Propenyt, Butenyl und Pentenyl.

Bevorzugte Alkinylgruppen sind beispielsweise Ethinyl, Propinyl, Butinyl, Pentinyl, Hexinyl, Heptinyl und Octinyl.

Bevorzugte Alkoxygruppen sind beispielsweise Methoxy, Ethoxy, n-Propoxy, n-Butoxy, n-Pentoxy, n-Hexoxy, n-Heptoxy, n-Octoxy.

Halogen bedeutet vorzugsweise F oder Cl.

Die polymerisierbare Gruppe P ist eine Gruppe, die für eine Polymerisationsreaktion, wie beispielsweise die radikalische oder ionische Kettenpolymerisation, Polyaddition oder Polykondensation, oder für eine polymeranaloge Umsetzung, beispielsweise die Addition oder Kondensation an eine Polymerhauptkette, geeignet ist. Besonders bevorzugt sind Gruppen für die Kettenpolymerisation, insbesondere solche enthaltend eine C=C-Doppelbindung oder -C≡C-Dreifachbindung, sowie zur Polymerisation unter Ringöffnung geeignete Gruppen wie beispielsweise Oxetan- oder Epoxygruppen

Bevorzugte Gruppen P sind ausgewählt aus CH₂=CW¹-COO-, CH₂=CW¹-CO-, 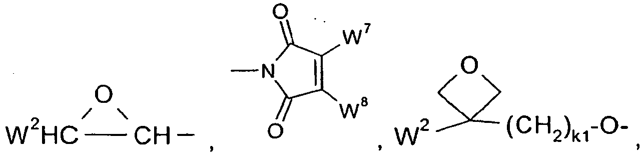 CH₂=CW²-(O)ₖ₃-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, CH₂=CH-(CO)ₖᵢ-Phe-(O)ₖ₂-, Phe-CH=CH-, HOOC-, OCN-, und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, welches optional mit ein oder mehreren Resten L wie oben definiert ist, k₁, k₂ und k₃ jeweils unabhängig voneinander 0 oder 1 bedeuten, k₃ vorzugsweise 1 bedeutet.

Besonders bevorzugte Gruppen P sind CH₂=CW¹-COO-, insbesondere CH₂=CH-COO-, CH₂=C(CH₃)-COO- und CH₂=CF-COO-, ferner CH₂=CH-O-, (CH₂=CH)₂CH-OCO-, (CH₂=CH)₂CH-O-, 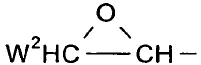 und

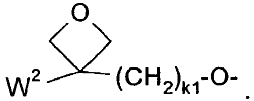

Ganz besonders bevorzugte Gruppen P sind Vinyloxy, Acrylat, Methacrylat, Fluoracrylat, Chloracrylat, Oxetan und Epoxy.

Der Begriff "Abstandsgruppe" (engl. "spacer" oder "spacer group"), vor- und nachstehend auch als "Sp" bezeichnet, ist dem Fachmann bekannt und in der Literatur beschrieben, siehe beispielsweise Pure Appl. Chem. 73(5), 888 (2001) und C. Tschierske, G. Pelzl, S. Diele, Angew. Chem. 2004, 116, 6340-6368. Falls nicht anders angegeben, bezeichnet der Begriff "Abstandsgruppe" bzw. "Spacer" vor- und nachstehend eine flexible Gruppe, die in einer polymerisierbaren flüssigkristallinen oder mesogenen Verbindung die mesogene Gruppe und die polymerisierbare(n) Gruppe(n) miteinander verbindet.

Bevorzugte Abstandsgruppen Sp sind ausgewählt aus der Formel Sp'-X', so dass der Rest P-Sp- der Formel P-Sp'-X'- entspricht, wobei
- Sp': Alkylen mit 1 bis 20, vorzugsweise 1 bis 12 C-Atomen bedeutet, welches optional durch F, Cl, Br, I oder CN ein- oder mehrfach substituiert ist, und worin auch eine oder mehrere nicht benachbarte CH₂-Gruppen jeweils unabhängig voneinander so durch -O-, -S-, -NH-, -NR⁰-, -SiR⁰⁰R⁰⁰⁰-, -CO-, -COO-, -OCO-, -OCO-O-, -S-CO-, -CO-S-, -NR⁰⁰-CO-O-, -O-CO-NR⁰⁰-, -NR⁰⁰-CO-NR⁰⁰-, -CH=CH- oder -C≡C- ersetzt sein können, dass O- und/oder S-Atome nicht direkt miteinander verknüpft sind,
- X': -O-, -S-, -CO-, -COO-, -OCF-, -O-COO-, -CO-NR⁰⁰-, -NR⁰⁰-CO-; _ -NR⁰⁰-CO-NR⁰⁰-, -OCH₂-, -CH₂O-, -SCH₂-, -CH₂S-, -CF₂O-, -OCF₂-, -CF₂S-, -CF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-COO-, -OCO-CH=CH- oder eine Einfachbindung bedeutet,
- R⁰⁰ und R⁰⁰⁰: jeweils unabhängig voneinander H oder Alkyl mit 1 bis 12 C-Atomen bedeuten, und
- Y² und Y³: jeweils unabhängig voneinander H, F, Cl oder CN bedeuten.

X' ist vorzugsweise -O-, -S -CO-, -COO-, -OCO-, -O-COO-, -CO-NR⁰-, -NR⁰-CO-, -NR⁰-CO-NR⁰- oder eine Einfachbindung.

Typische Abstandsgruppen Sp' sind beispielsweise -(CH₂)ₚ₁-, -(CH₂CH₂O)_{q1}-CH₂CH₂-, -CH₂CH₂-S-CH₂CH₂-, -CH₂CH₂-NH-CH₂CH₂- oder -(SiR⁰⁰R⁰⁰-O)ₚ₁-, worin p1 eine ganze Zahl von 1 bis 12 ist, q1 eine ganze Zahl von 1 bis 3 ist, und R⁰⁰ und R⁰⁰⁰ die oben angegebenen Bedeutungen besitzen.

Besonders bevorzugte Gruppen -X'-Sp'- sind -(CH₂)ₚ₁-, -O-(CH₂)ₚ₁-, -OCO-(CH₂)ₚ₁-, -OCOO-(CH₂)ₚ₁-.

Besonders bevorzugte Gruppen Sp' sind beispielsweise jeweils geradkettiges Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen und Butenylen.

Besonders bevorzugte Verbindungen der Formel I sind solche ausgewählt aus folgenden Unterformeln 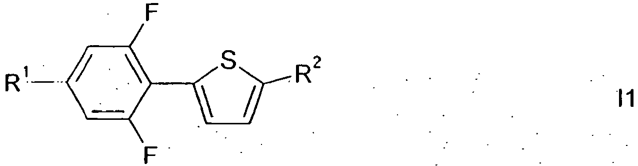 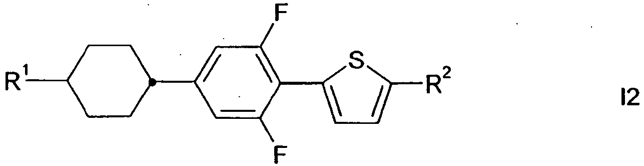 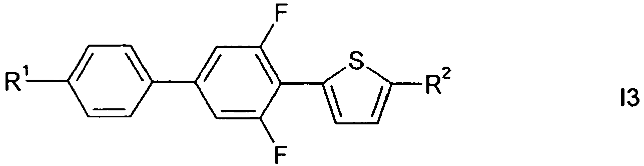 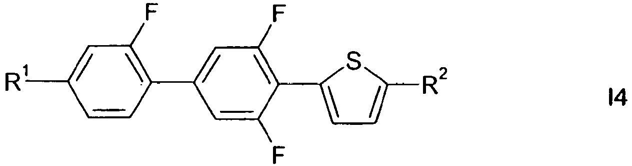 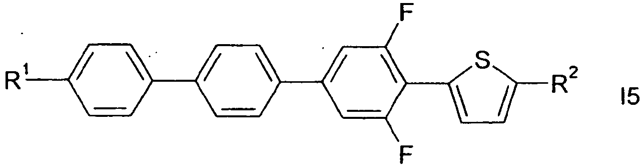 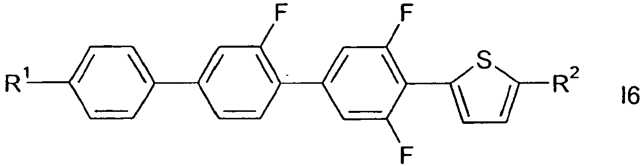 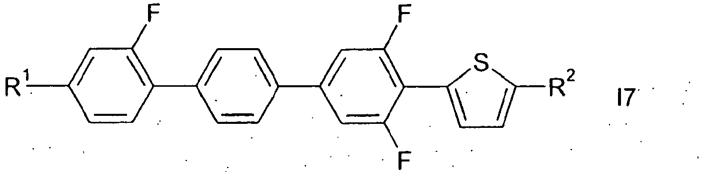 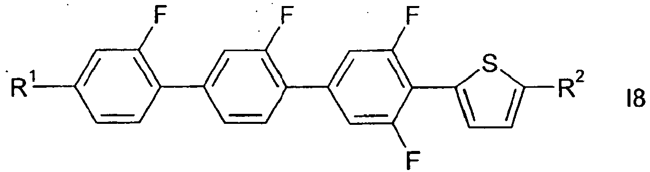 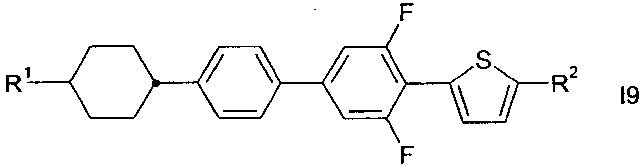 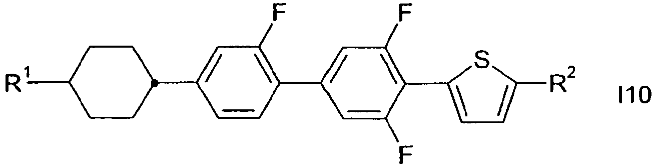 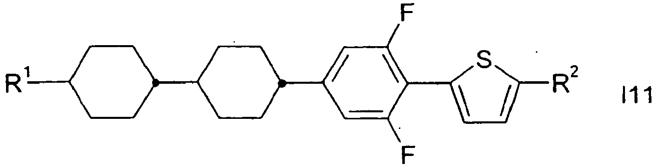 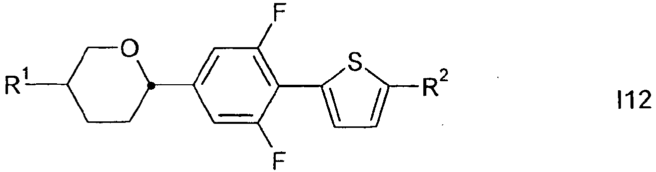 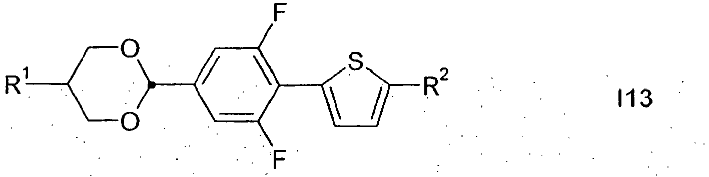 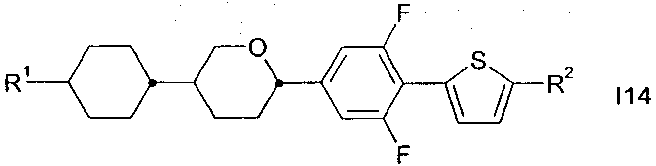 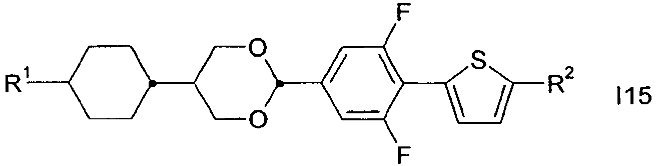 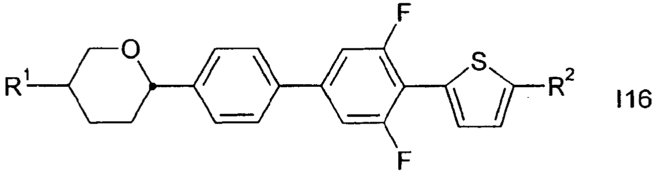 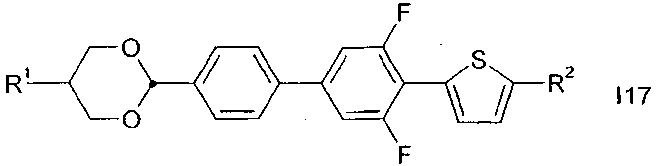 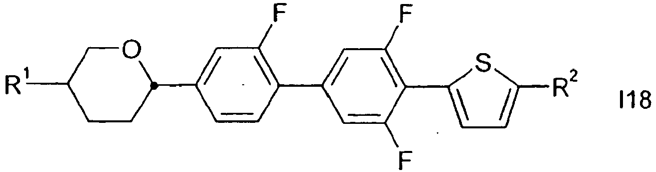 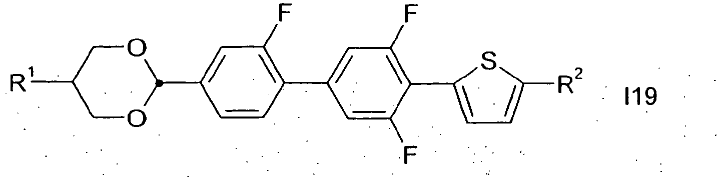 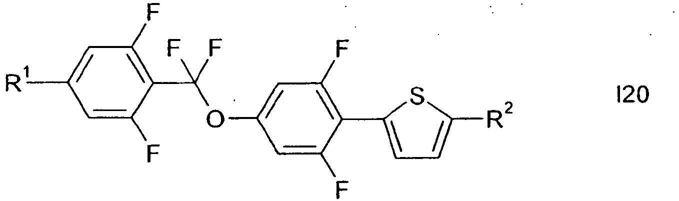 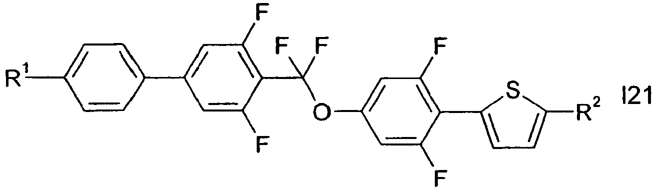 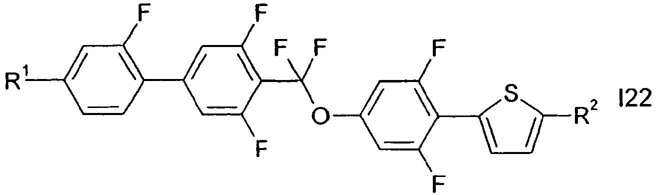 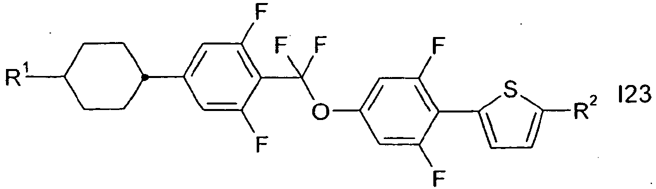 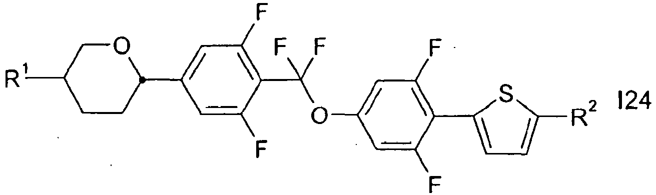 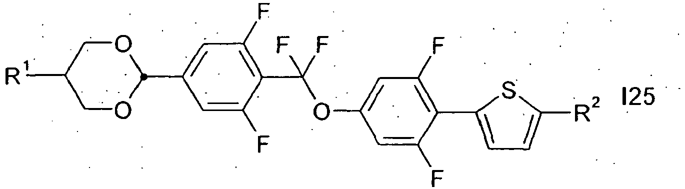 worin R¹ und R² die vor- und nachstehend angegebenen Bedeutungen besitzen. R¹ und R² bedeuten darin vorzugsweise optional fluoriertes Alkyl, Alkenyl, Alkinyl oder Alkoxy mit 1 bis 12 C-Atomen, besonders bevorzugt optional fluoriertes Alkyl, Alkenyl oder Alkinyl mit 1 bis 5 C-Atomen. Die Verbindungen der Formel I3 sind ganz besonders bevorzugt.

Die Verbindungen der Formel I können in Analogie zu dem Fachmann bekannten und in Standardwerken der organischen Chemie beschriebenen Verfahren, wie beispielsweise in Houben-Weyl, Methoden der organischen Chemie, Thieme-Verlag, Stuttgart, hergestellt werden.

Besonders bevorzgte Verfahren zur Herstellung von Verbindungen der Formel I sind im Nachfolgenden beschrieben. Dabei haben die einzelnen Reste R^{1,2}, A^{0,1,1}, Z^{1,2}, m und n die in Formel I angegebene Bedeutung.

Vorzugsweise erfolgt die Synthese der Verbindungen der Formel I, wie beispielhaft im nachfolgenden Schema 1 gezeigt, ausgehend von entsprechend substituierten Bromthiophenen **2**. Diese werden in einer Palladium-vermittelten Suzuki-Kreuzkupplung mit entsprechenden Boronsäuren **1** oder Boronsäureestern zu den Verbindungen der Formel I umgesetzt.

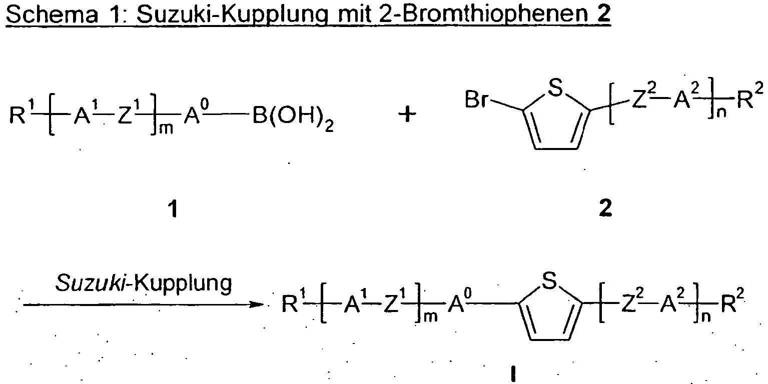

Die Synthese der Bromthiophene **2** erfolgt durch Bromierung von Thiophenen **3**. Dies kann durch direkte Bromierung mit Brom oder NBS (z.B. *Methoden A* und *B* in Schema 2) oder durch Bromierung einer Lithiothiophenzwischenstufe erfolgen (*Methode C*).

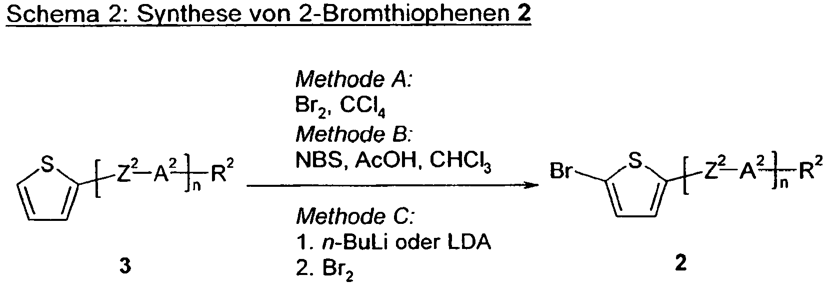

Wird für die obige *Suzuki*-Kupplung Bromthiophen **2a** (**2** mit n = 0 und R² = H) verwendet, so werden Verbindungen **Ib** (**I** mit n = 0 und R² = H) erhalten, die weiter funktionalisiert werden können, wie beispielhaft in Schema 3 gezeigt. Diese Funktionalisierung kann beispielsweise über eine lithiierte Zwischenstufe erfolgen. Diese kann mit zahlreichen Elektrophilen umgesetzt werden. Aus einer Reaktion mit Brom oder lod gehen Verbindungen **Ic** (**I** mit n = 0 und R² = Br oder I) hervor die durch Kreuzkupplungsreaktionen (z.B. *Suzuki*-, *Kumada-* oder *Sonogashira-*Kupplungen) weiter funktionalisiert werden können.

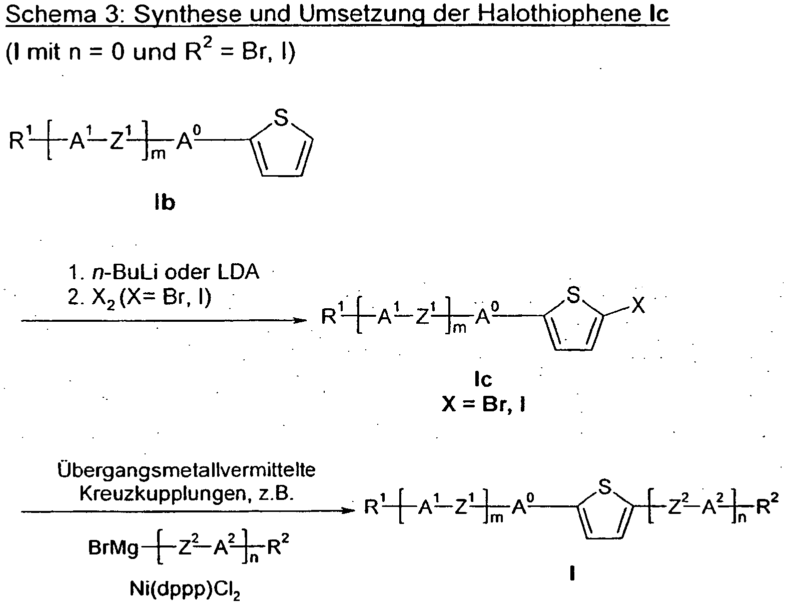

Ein Zugang zu den besonders bevorzugten Verbindungen **I** in denen R² einen Alkylrest bedeutet, besteht in der Alkylierung der aus **Ib** hervorgehenden lithiierten Verbindung **4** mit z.B. Alkylhalogeniden oder Alkylsulfaten, wie beispielhaft in Schema 4 gezeigt.

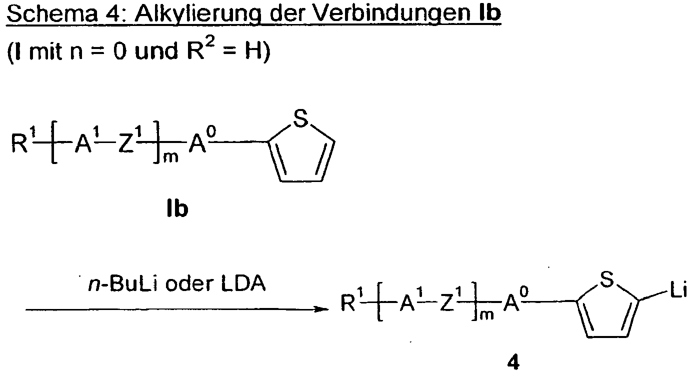

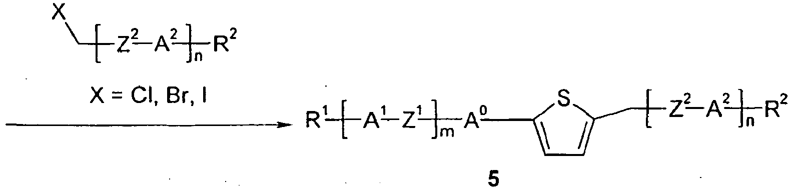

In einigen Fällen verlaufen diese Alkylierungen jedoch unter Verbleib von nicht umgesetzten Ausgangsmaterialien **Ib** deren Abtrennung sich schwierig gestalten kann. Daher sind die Zwischenstufen **6** bevorzugte Materialien, um zu den bevorzugten Verbindungen der Formel **I** mit Alkenyl- und Alkylsubstituenten zu gelangen. Die Thiophencarbaldehyde **6** werden, wie beispielhaft in Schema 5 gezeigt, durch Umsetzung der Verbindungen **4** mit DMF oder *N*-Formylmorpholin erhalten. Die Aldehyde können dann in einer *Wittig*-Reaktion zu alkenylsubstituierten Thiophenen **7** umgesetzt werden. Letztere Verbindungen können zu Alkylthiophenderivaten **8** hydriert werden.

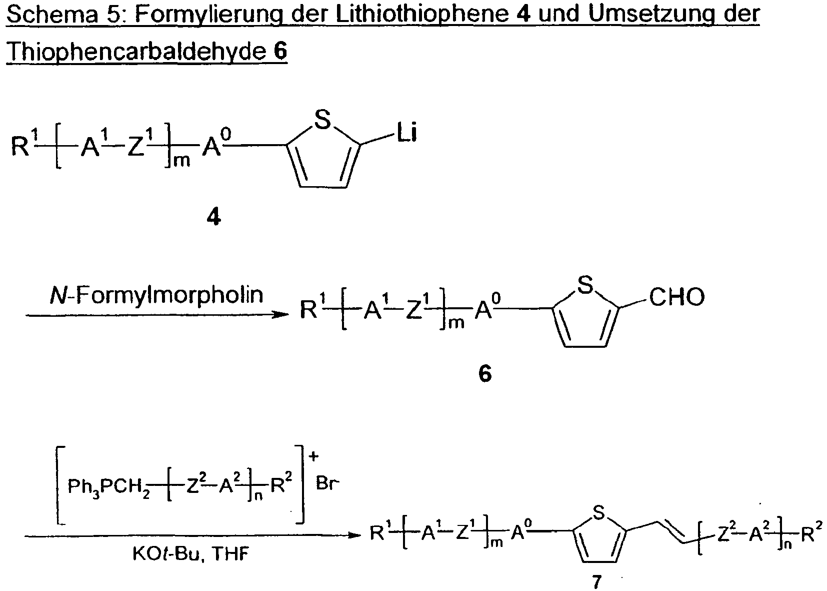

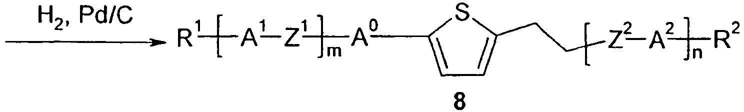

Für die Synthese der Verbindungen I bei denen R²[A²Z²]- gleich Alkyl- oder Alkenyl ist, ist auch 5-Bromthiophen-2-carbaldehyd 9 ein bevorzugtes Ausgangsmaterial, wie beispielhaft in Schema 6 gezeigt.

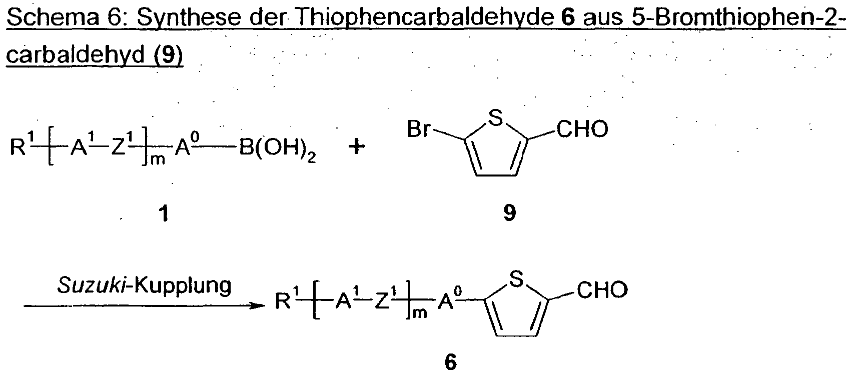

Weiterhin können in den Kreuzkupplungen aus Schema 1 die Rollen von Elektrophil und Nucloephil vertauscht werden, indem Verbindungen vom Typ 10 mit Thiophenboronsäuren oder Thiophenhydroxyboronsäuresalzen 11 umgesetzt werden, wie beispielhaft in Schema 7 gezeigt. Letztere Verbindungen lassen sich besonders einfach synthtisieren und isolieren [A. N. Cammidge, V. H. M. Goddard, H. Gopee, N. L. Harrison, D. L. Hughes, C. J. Schubert, B. M. Sutton, G. L. Watts, A. J. Whitehead, Org. Lett. 2006, 8, 4071-4074.]

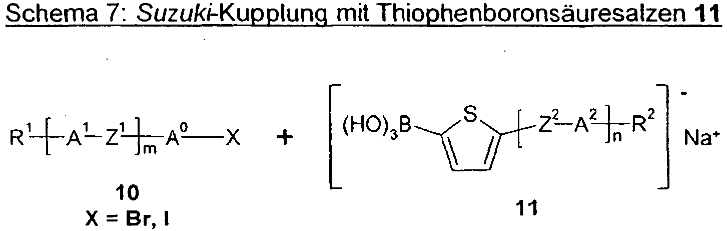

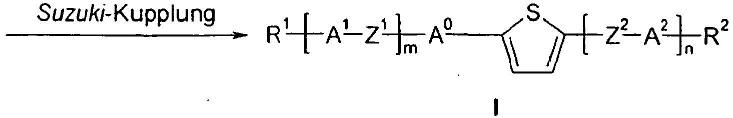

Weiterhin bevorzugt sind Verbindungen in denen n = 0 bedeutet und R² bevorzugt einen Alkoxysubstituenten darstellt. Zu deren Synthese werden 5-Bromthiophen-2-ylether **2c** (**2** mit n = 0 und R² = OR²², wobei R²² die gleichen Bedeutungen sowie die gleichen bevorzugten Bedeutungen wie unter Formel I definiert annehmen kann, wobei O-Atome nicht direkt miteinander verknüpft sein sollen und R²² bevorzugt Alkyl bedeutet) benötigt. Diese werden ausgehend von Bromthiophen **2a** (**2** mit n = 0 und R² = Br) hergestellt, wie beispielhaft in Schema 8 gezeigt. Zuerst erfolgt Einführung der Alkoxygruppe durch Umsetzung mit einem Alkoholat in Gegenwart von Kupfer(I)-bromid [M. A. Keegstra, T. H. A. Peters, L. Brandsma, Tetrahedron 1992, 48, 3633-3652.]. Die so erhaltenen Thiophene **12** werden dann zu den Verbindungen **2c** (**2** mit n = 0 und R² = OR²², wobei R²² die gleichen Bedeutungen sowie die gleichen bevorzugten Bedeutungen wie unter Formel **I** definiert annehmen kann, wobei O-Atome nicht direkt miteinander verknüpft sein sollen und R²² bevorzugt Alkyl bedeutet) bromiert und wie in Schema 1 dargestellt weiter umgesetzt.

### Schema 8: Synthese von 5-Bromthiophen-2-ylethern 2c

(**2** mit n = 0 und R² = OR²², wobei R²² die gleichen Bedeutungen sowie die gleichen bevorzugten Bedeutungen wie unter Formel **I** definiert annehmen kann, wobei O-Atome nicht direkt miteinander verknüpft sein sollen und R²² bevorzugt Alkyl bedeutet)

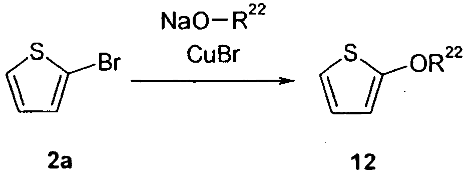

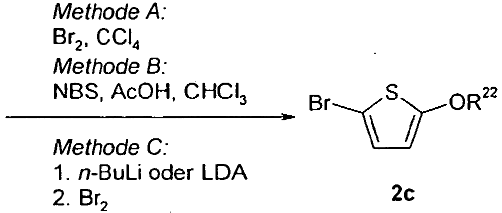

Weiterhin bevorzugt sind Verbindungen vom Typ I in denen -[Z₂-A₂]ₙ-R₂ Fluor bedeutet (= **Id**). Diese werden vorteilhaft aus den Verbindungen **Ib** (**I** mit n = 0 und R² = H) hergestellt, wie beispielhaft in Schema 9 gezeigt. Dazu wird die zunächst gebildete lithiierte Zwischenstufen **4** mit *N*-Fluorbenzolsulfonamid abgefangen.

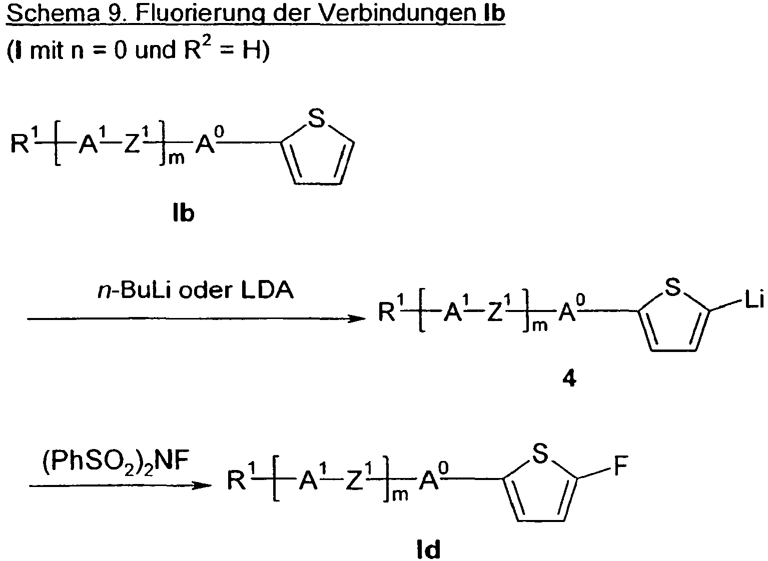

Die vor- und nachstehend beschriebenen Verfahren und die darin erzeugten bzw. verwendeten neuen Zwischenprodukte, sowie deren Verwendung, zur Herstellung erfindungsgemäßer Verbindungen der Formel I, sind ein weiterer Gegenstand der vorliegenden Erfindung.

Besonders bevorzugte erfindungsgemäße FK-Medien werden im Folgenden genannt:
- FK-Medium, welches zusätzlich eine oder mehrere Verbindungen der Formel II und/oder III enthält: 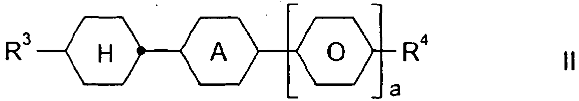 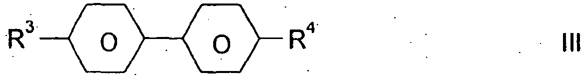 worin
   - A: 1,4-Phenylen oder trans-1,4-Cyclohexylen bedeutet,
   - a: 0 oder 1 ist,
   - R³: Alkenyl mit 2 bis 9 C-Atomen bedeutet, und
   - R⁴: Alkyl mit 1 bis 12 C-Atomen, wobei auch eine oder zwei nicht benachbarte CH₂-Gruppen durch -O-, -CH=CH-, -CO-, -OCO- oder -COO- so ersetzt sein können, dass O-Atome nicht direkt miteinander verknüpft sind, und vorzugsweise Alkyl mit 1 bis 12 C-Atomen oder Alkenyl mit 2 bis 9 C-Atomen bedeutet.

Die Verbindungen der Formel II sind vorzugsweise ausgewählt aus der Gruppe bestehend aus folgenden Formeln: 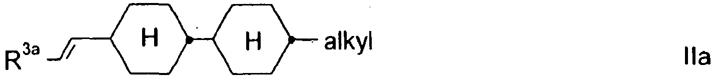 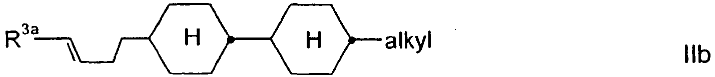 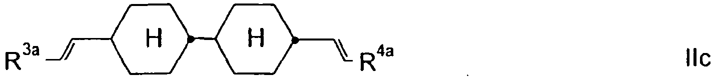 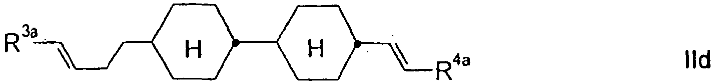 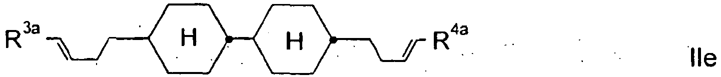 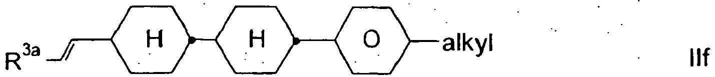 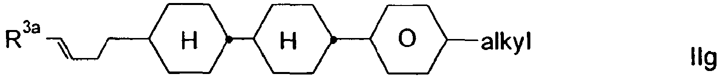 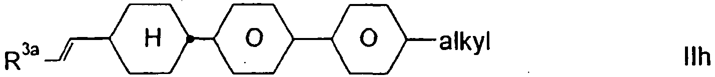 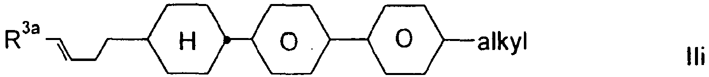 worin R^{3a} und R^{4a} jeweils unabhängig voneinander H, CH₃, C₂H₅ oder C₃H₇ bedeuten, und "alkyl" eine geradkettige Alkylgruppe mit 1 bis 8, vorzugsweise 1, 2, 3, 4 oder 5 C-Atomen bedeutet. Besonders bevorzugt sind Verbindungen der Formel IIa und IIf, insbesondere worin R^{3a} H oder CH₃, vorzugsweise H, bedeutet, und Verbindungen der Formel IIc, insbesondere worin R^{3a} und R^{4a} H, CH₃ oder C₂H₅ bedeuten.

Die Verbindungen der Formel III sind vorzugsweise ausgewählt aus der Gruppe bestehend aus den folgenden Formeln: 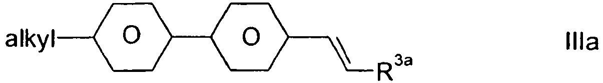 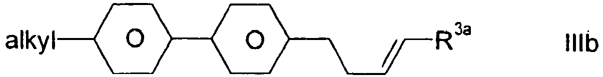 worin "alkyl" und R^{3a} die oben angegebenen Bedeutungen haben und R^{3a} vorzugsweise H oder CH₃ bedeutet. Besonders bevorzugt sind Verbindungen der Formel IIIb;
- FK-Medium, welches zusätzlich eine oder mehrere Verbindungen ausgewählt aus der Gruppe bestehend aus den folgenden Formeln enthält: 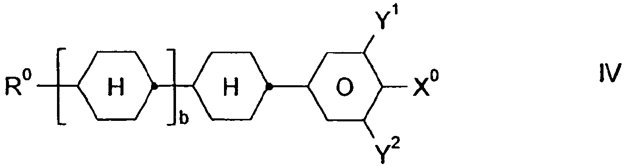 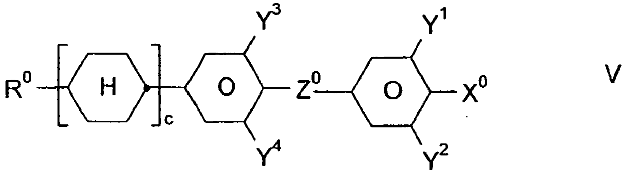 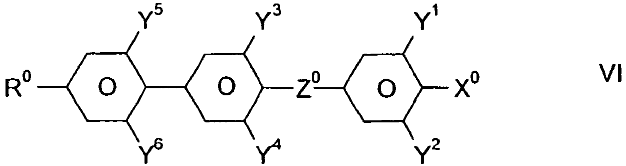 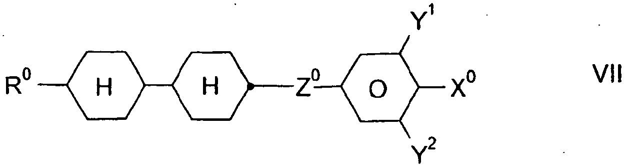 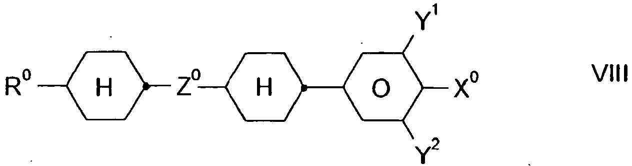 worin
   - R⁰: einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH- , 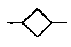 , 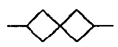 , -O-, -CO-O- oder -O-CO- so ersetzt sein können, dass O-Atome nicht direkt miteinander verknüpft sind, und worin auch ein oder mehrere H-Atome durch Halogen ersetzt sein können,
   - X⁰: F, Cl, CN, SF₅, SCN, NCS, einen halogenierten Alkylrest, halogenierten Alkenylrest, halogenierten Alkoxyrest oder halogenierten Alkenyloxyrest mit jeweils bis zu 6 C-Atomen,
   - Y¹⁻⁶: jeweils unabhängig voneinander H oder F,
   - Z⁰: -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O oder -OCF₂-, in den Formeln V und VI auch eine Einfachbindung, und
   - b und c: jeweils unabhängig voneinander 0 oder 1
   bedeuten.

In den Verbindungen der Formel IV bis VIII bedeutet X⁰ vorzugsweise F oder OCF₃, ferner OCHF₂, CF₃, CF₂H, Cl, OCH=CF₂. R⁰ ist vorzugsweise geradkettiges Alkyl oder Alkenyl mit jeweils bis zu 6 C-Atomen.

Die Verbindungen der Formel IV sind vorzugsweise ausgewählt aus der Gruppe bestehend aus den folgenden Formeln: 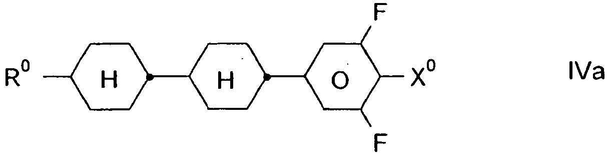 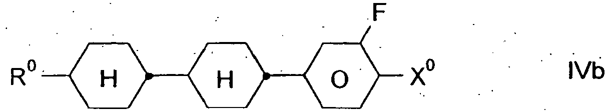 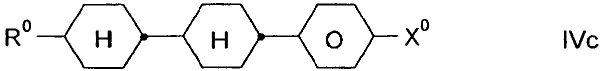 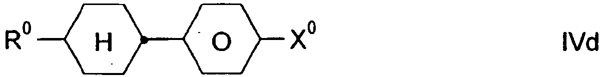 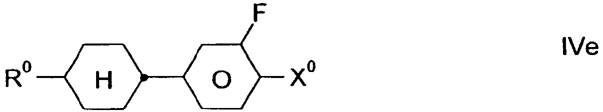 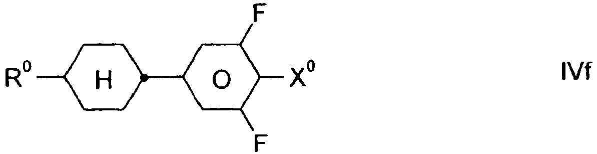 worin R⁰ und X⁰ die oben angegebenen Bedeutungen haben.

Vorzugsweise bedeutet in Formel IV R⁰ Alkyl mit 1 bis 8 C-Atomen und X⁰ F, Cl, OCHF₂ oder OCF₃, ferner OCH=CF₂. In der Verbindung der Formel IVb bedeutet R⁰ vorzugsweise Alkyl oder Alkenyl. In der Verbindung der Formel IVd bedeutet X⁰ vorzugsweise Cl, ferner F.

Die Verbindungen der Formel V sind vorzugsweise ausgewählt aus der Gruppe bestehend aus den folgenden Formeln: 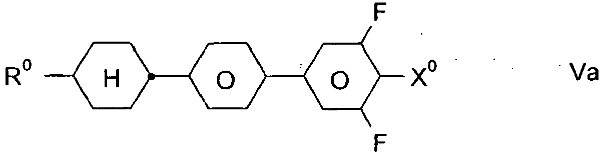 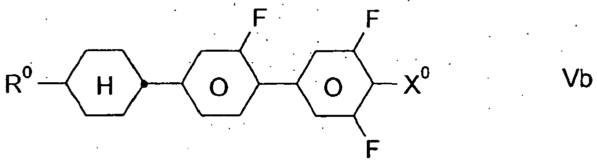 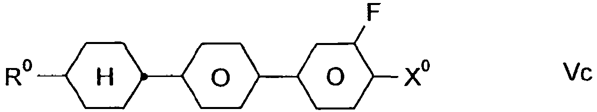 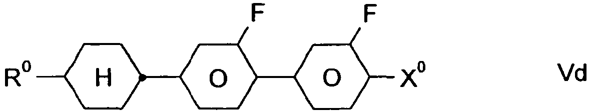 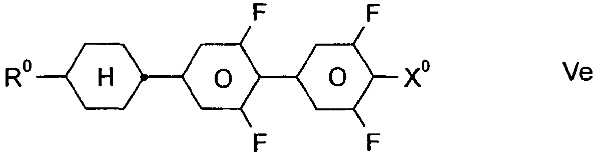 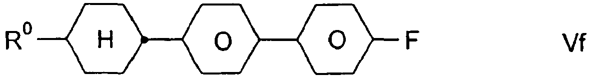 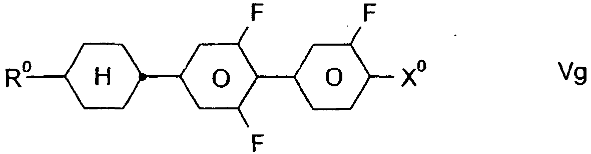 worin R⁰ und X⁰ die oben angegebenen Bedeutungen haben. Vorzugsweise bedeutet R⁰ in Formel V Alkyl mit 1 bis 8 C-Atomen und X⁰ F;
- FK-Medium, welches eine oder mehrere Verbindungen der Formel VI-1 enthält: 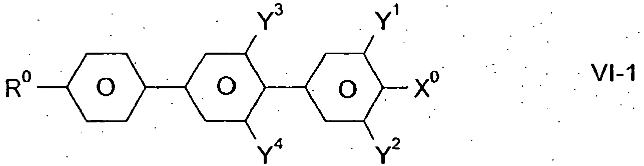 besonders bevorzugt solche ausgewählt aus der Gruppe bestehend aus den folgenden Formeln: 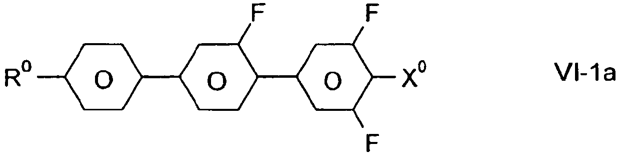 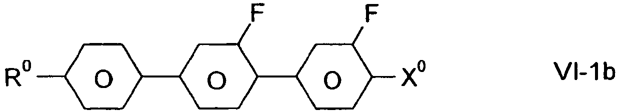 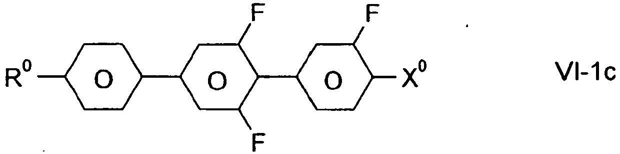 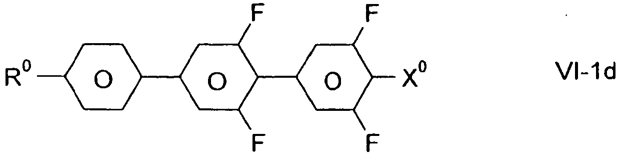 worin R⁰ und X⁰ die oben angegebenen Bedeutungen haben. Vorzugsweise bedeutet R⁰ in Formel VI Alkyl mit 1 bis 8 C-Atomen und X⁰ F, ferner OCF₃.
- FK-Medium, welches eine oder mehrere Verbindungen der Formel VI-2 enthält: 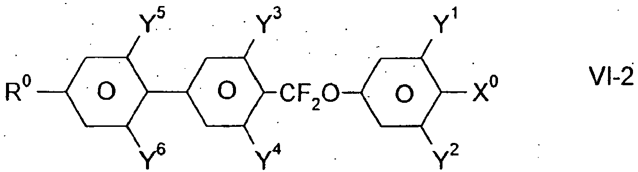 besonders bevorzugt solche ausgewählt aus der Gruppe bestehend aus den folgenden Formeln: 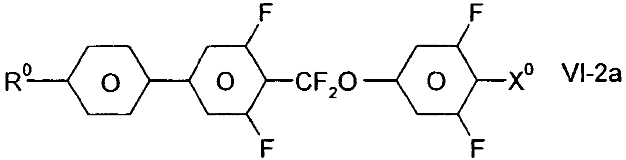 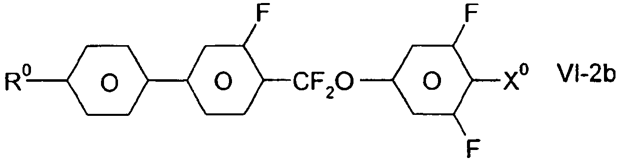 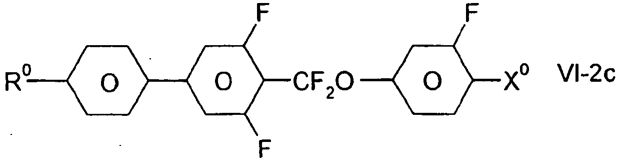 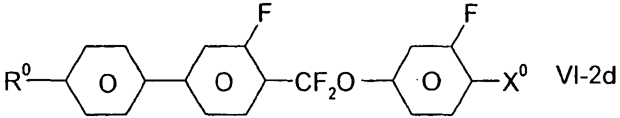 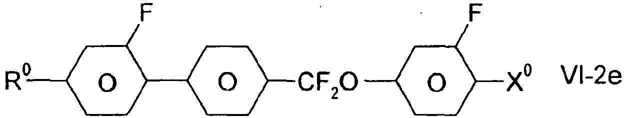 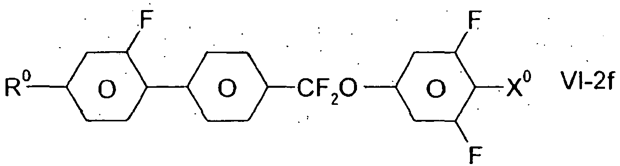 worin R⁰ und X⁰ die oben angegebenen Bedeutungen haben.

Vorzugsweise bedeutet R⁰ in Formel VI Alkyl mit 1 bis 8 C-Atomen und X⁰ F;
- FK-Medium, welches vorzugsweise eine oder mehrere Verbindungen der Formel VII, worin Z⁰ -CF₂O- -CH₂CH₂ oder -COO-, bedeutet, enthält, besonders bevorzugt solche ausgewählt aus der Gruppe bestehend aus den folgenden Formeln: 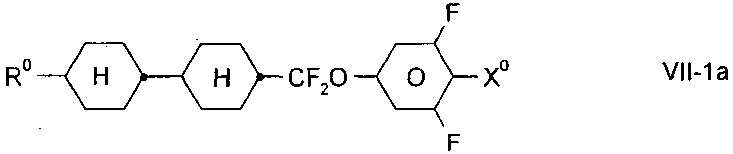 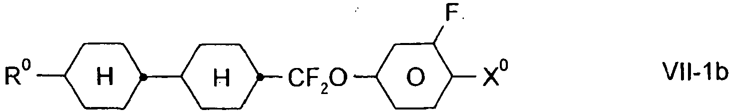 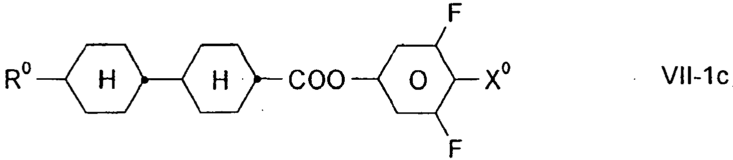 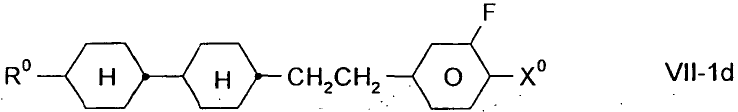 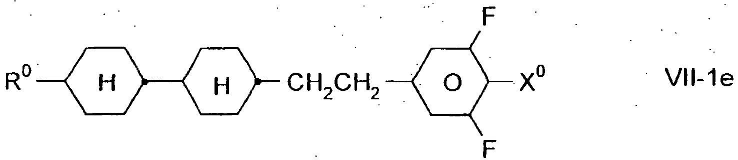
worin R⁰ und X⁰ die oben angegebenen Bedeutungen haben. Vorzugsweise bedeutet R⁰ in Formel VII Alkyl mit 1 bis 8 C-Atomen und X⁰ F, ferner OCF₃.

Die Verbindungen der Formel VIII sind vorzugsweise ausgewählt aus der Gruppe bestehend aus den folgenden Formeln: 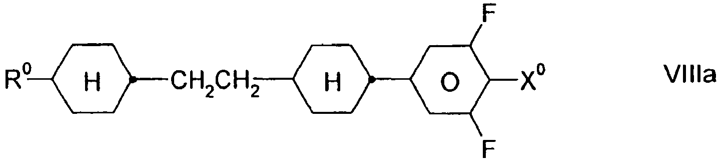 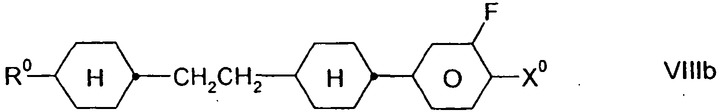 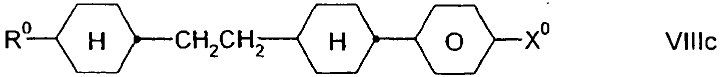 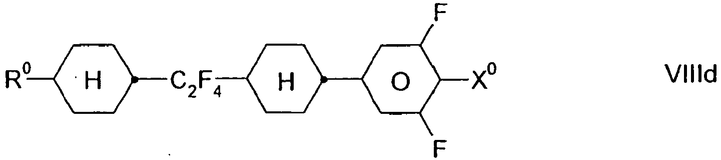 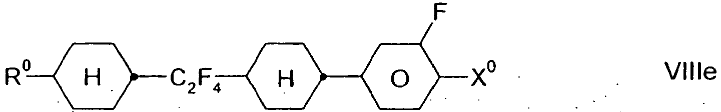 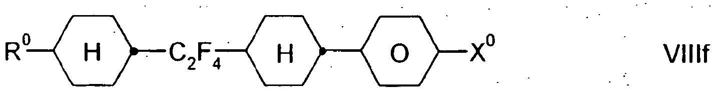 worin R⁰ und X⁰ die oben angegebenen Bedeutungen haben. Vorzugsweise bedeutet R⁰ einen geradkettigen Alkylrest mit 1 bis 8 C-Atomen. X⁰ bedeutet vorzugsweise F.
- FK-Medium, welches zusätzlich eine oder mehrere Verbindungen der folgenden Formel enthält: 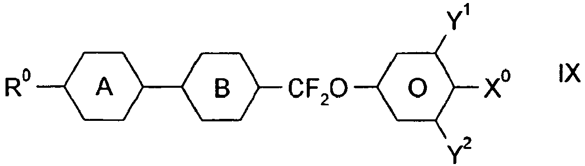 worin R⁰, X⁰, Y¹ und Y² die oben angegebene Bedeutung besitzen, und 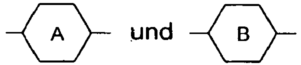 jeweils unabhängig voneinander 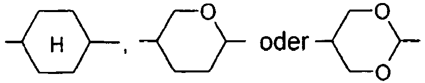
bedeuten, wobei die Ringe A und B nicht beide gleichzeitig Cyclohexylen bedeuten;

Die Verbindungen der Formel IX sind vorzugsweise ausgewählt aus der Gruppe bestehend aus den folgenden Formeln: 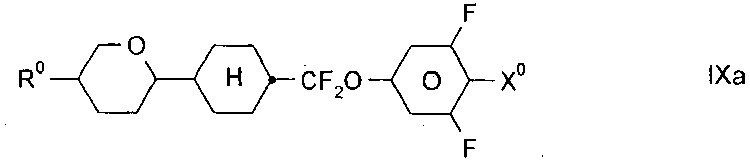 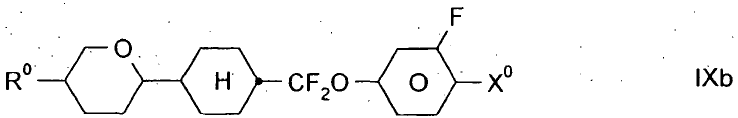 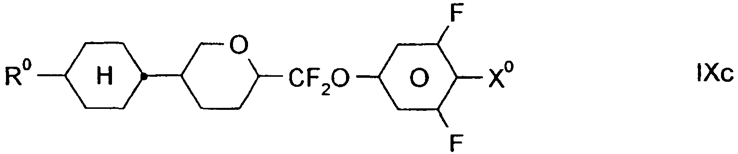 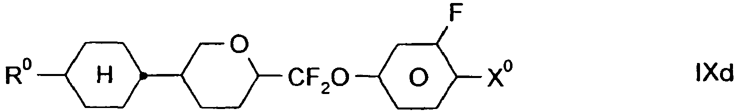 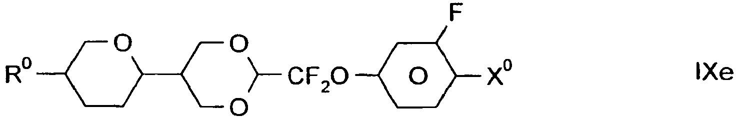 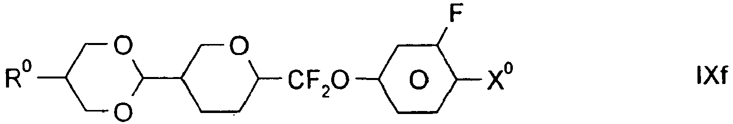 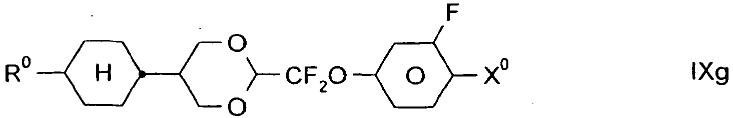 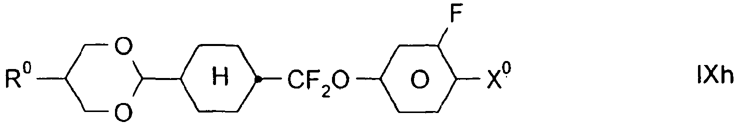 worin R⁰ und X⁰ die oben angegebenen Bedeutungen haben. Vorzugsweise bedeutet R⁰ Alkyl mit 1 bis 8 C-Atomen und X⁰ F. Besonders bevorzugt sind Verbindungen der Formel IXa;
- FK-Medium, welches zusätzlich eine oder mehrere Verbindungen ausgewählt aus der Gruppe bestehend aus den folgenden Formeln enthält: 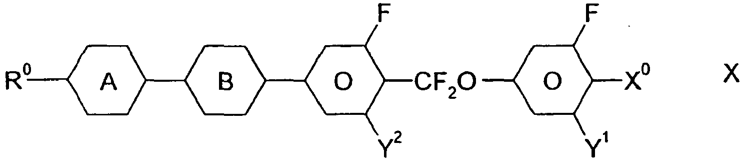 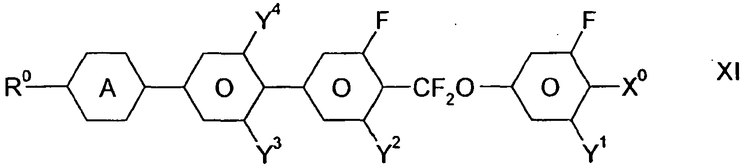 worin R⁰, X⁰ und Y¹⁻⁴ die oben angegebenen Bedeutungen besitzen, und 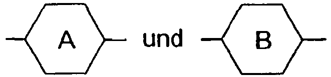 jeweils unabhängig voneinander 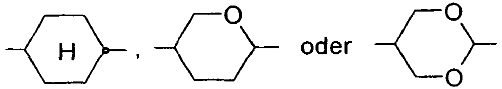 bedeuten;

Die Verbindungen der Formeln X und XI sind vorzugsweise ausgewählt aus der Gruppe bestehend aus den folgenden Formeln: 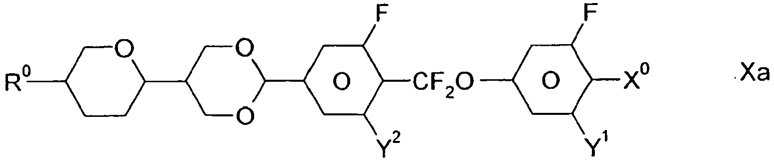 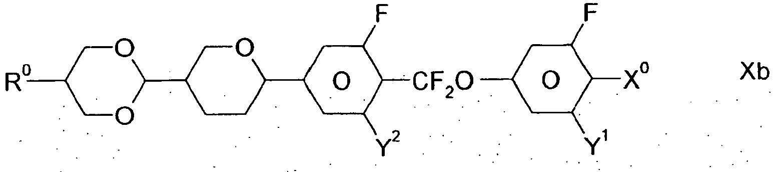 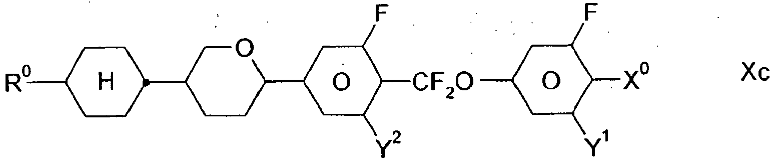 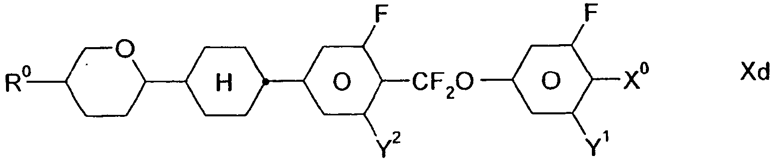 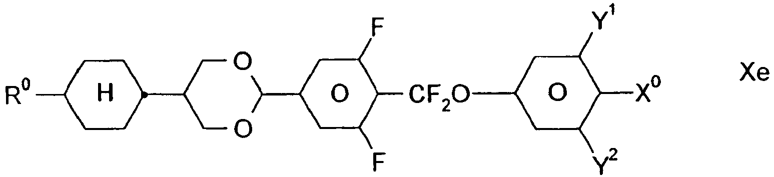 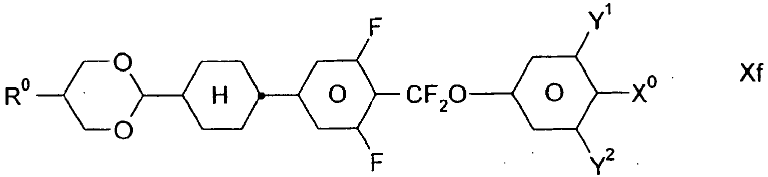 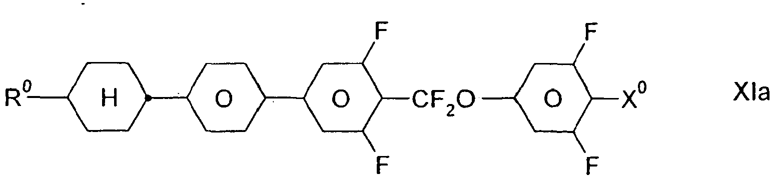 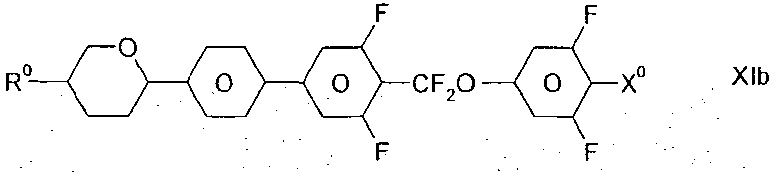 worin R⁰ und X⁰ die oben angegebenen Bedeutungen haben. Vorzugsweise bedeutet R⁰ Alkyl mit 1 bis 8 C-Atomen und X⁰ F. Besonders bevorzugte Verbindungen sind solche, worin Y¹ F und Y² H oder F, vorzugsweise F, bedeuten;

FK-Medium, welches zusätzlich eine oder mehrere Verbindungen der folgenden Formel enthält: 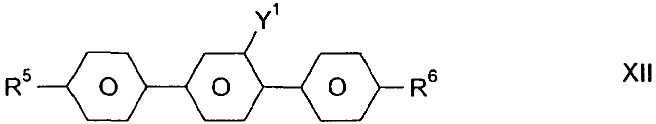 worin R⁵ und R⁶ jeweils unabhängig voneinander n-Alkyl, Alkoxy, Oxaalkyl, Fluoralkyl oder Alkenyl mit jeweils bis zu 9 C-Atomen bedeuten, und vorzugsweise jeweils unabhängig voneinander Alkyl mit 1 bis 8 C-Atomen bedeuten. Y¹ bedeutet H oder F.

Bevorzugte Verbindungen der Formel XII sind solche ausgewählt aus der Gruppe bestehend aus den folgenden Formeln: 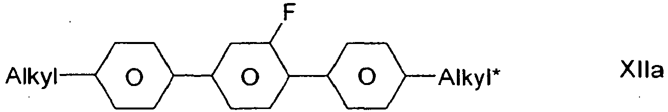 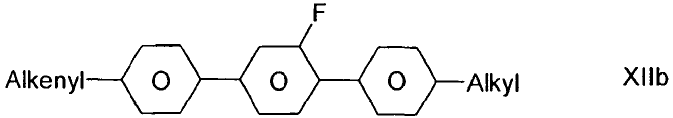 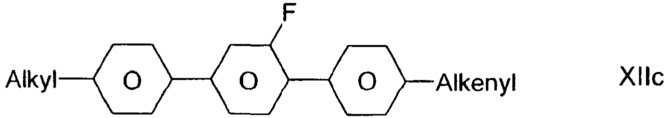 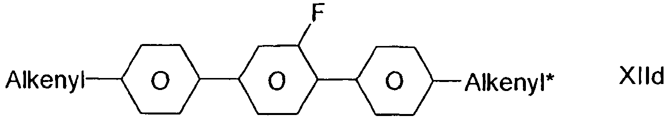 worin

| | |
|---|---|
| Alkyl und Alkyl* | jeweils unabhängig voneinander einen geradkettigen Alkylrest mit 1 bis 6 C-Atomen, und |
| Alkenyl und Alkenyl* | jeweils unabhängig voneinander einen geradkettigen Alkenylrest mit 2 bis 6 C-Atomen |

bedeuten. Ganz besonders bevorzugt sind Verbindungen der folgenden Formel 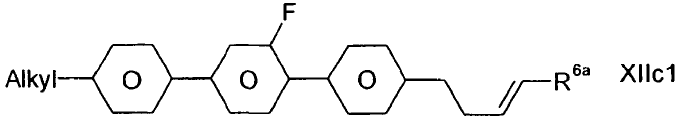 worin Alkyl die oben angegebene Bedeutung hat und R^{6a} H oder CH₃ bedeutet.
- FK-Medium, welches zusätzlich eine oder mehrere Verbindungen ausgewählt aus der Gruppe bestehend aus den folgenden Formeln enthält: 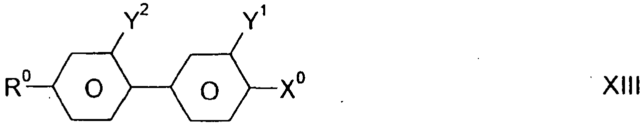 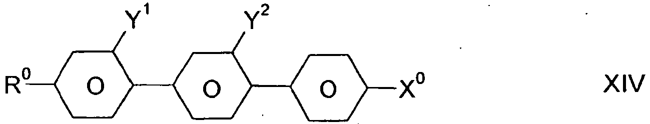 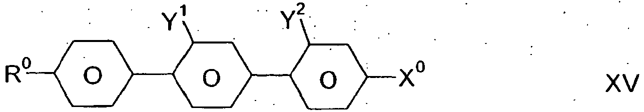 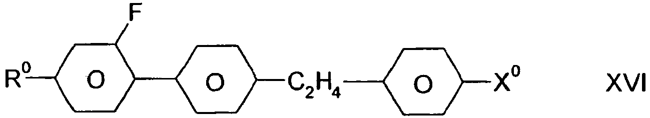 worin R⁰, X⁰, Y¹ und Y² die oben angegebenen Bedeutungen haben. Vorzugsweise bedeutet R⁰ Alkyl mit 1 bis 8 C-Atomen und X⁰ F oder Cl;

Die Verbindungen der Formeln XIII und XIV sind vorzugsweise ausgewählt aus der Gruppe bestehend aus den folgenden Formeln: 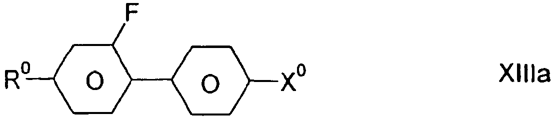 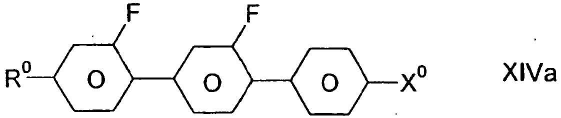 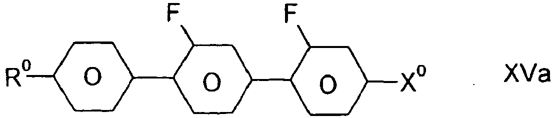 worin R⁰ und X⁰ die oben angegebenen Bedeutungen haben. Vorzugsweise bedeutet R⁰ Alkyl mit 1 bis 8 C-Atomen. In den Verbindungen der Formel XIII bedeutet X⁰ vorzugsweise F oder Cl.

FK-Medium, welches zusätzlich eine oder mehrere Verbindungen der Formel D1 und/oder D2 enthält: 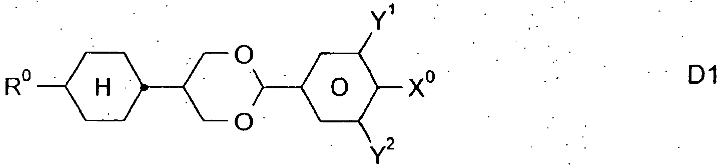 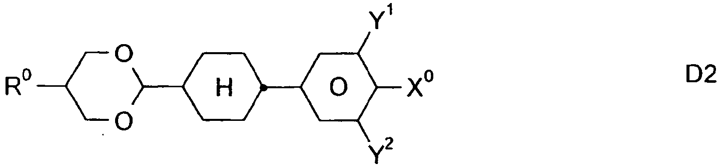 worin Y¹, Y², R⁰ und X⁰ die oben angegebene Bedeutung besitzen. Vorzugsweise bedeutet R⁰ Alkyl mit 1 bis 8 C-Atomen und X⁰ F.

Besonders bevorzugt sind Verbindungen der folgenden Formeln: 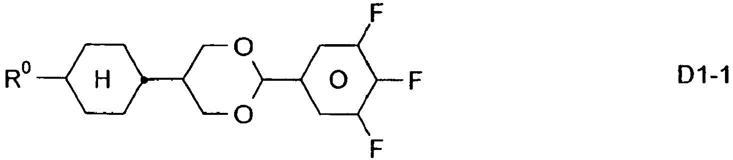 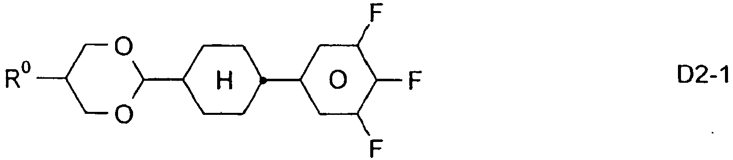 worin R⁰ die oben angegebenen Bedeutungen hat und vorzugsweise geradkettiges Alkyl mit 1 bis 6 C-Atomen, insbesondere C₂H₅, n-C₃H₇ oder n-C₅H₁₁ bedeutet.

FK-Medium, welches zusätzlich eine oder mehrere Verbindungen der folgenden Formel enthält: 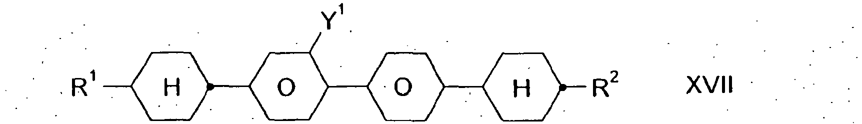 worin Y¹, R¹ und R² die oben angegebene Bedeutung besitzen. R¹ und R² bedeuten vorzugsweise jeweils unabhängig voneinander Alkyl mit 1 bis 8 C-Atomen;
- FK-Medium, welches zusätzlich eine oder mehrere Verbindungen der folgenden Formel enthält: 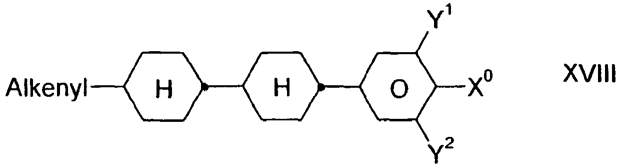 worin X⁰, Y¹ und Y² die oben angegebenen Bedeutungen besitzen und "Alkenyl" C₂₋₇-Alkenyl bedeutet. Besonders bevorzugt sind Verbindungen der folgenden Formel, 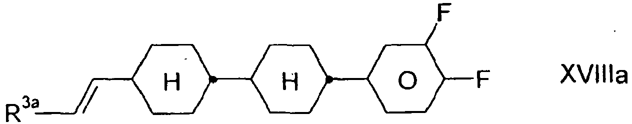 worin R^{3a} die oben angegebene Bedeutung hat und vorzugsweise H bedeutet;
- FK-Medium, welches zusätzlich eine oder mehrere Vierkern-Verbindungen ausgewählt aus der Gruppe bestehend aus den Formeln XIX bis XXV enthält: 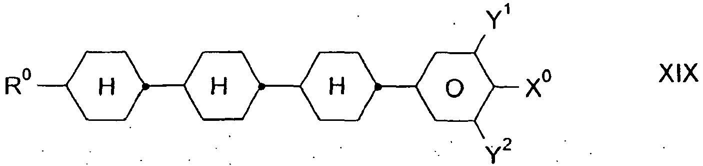 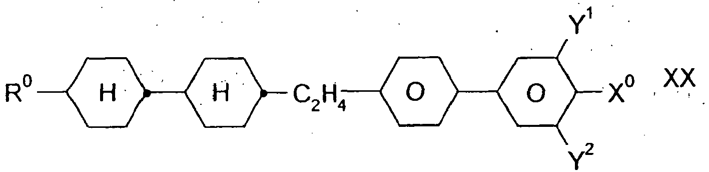 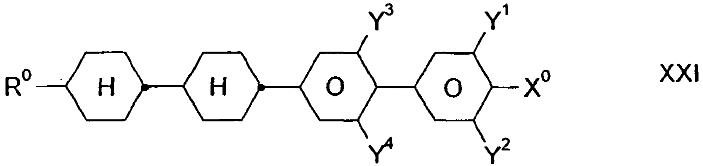 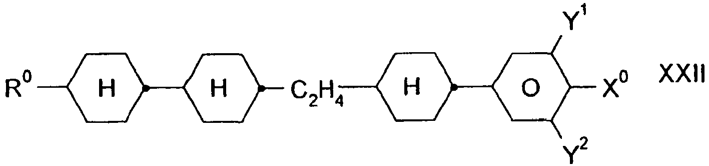 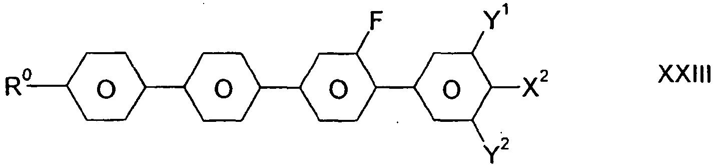 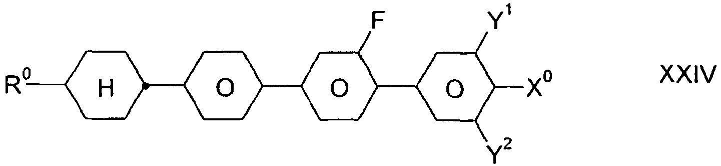 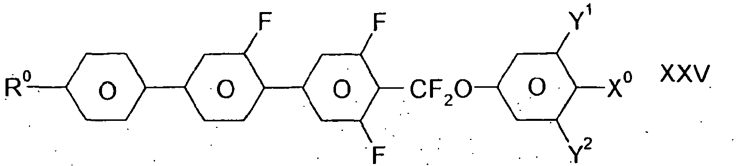 worin Y¹⁻⁴, R⁰ und X⁰ jeweils unabhängig voneinander eine der oben angegebenen Bedeutungen haben. X⁰ ist vorzugsweise F, Cl, CF₃, OCF₃ oder OCHF₂. R⁰ bedeutet vorzugsweise Alkyl, Alkoxy, Oxaalkyl, Fluoralkyl oder Alkenyl mit jeweils bis zu 8 C-Atomen.
- FK-Medium, welches zusätzlich eine oder mehrere Verbindungen der folgenden Formel enthält: 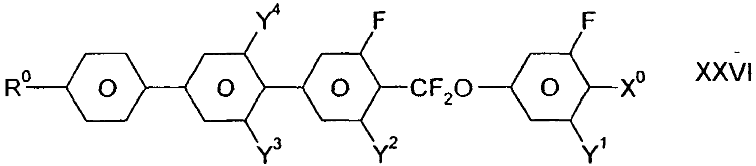 worin R⁰, X⁰ und Y¹⁻⁴ die oben angegebenen Bedeutungen besitzen. Besonders bevorzugt sind Verbindungen der folgenden Formel: 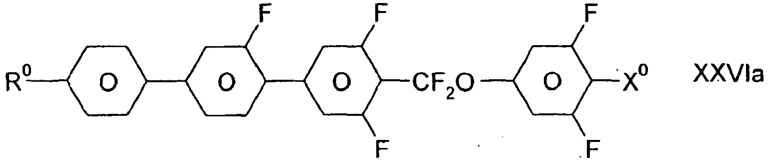
- FK-Medium, welches zusätzlich eine oder mehrere Verbindungen der folgenden Formel enthält: 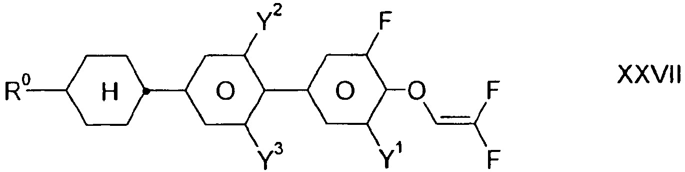 worin R⁰ und Y¹⁻³ die oben angegebenen Bedeutungen besitzen. Besonders bevorzugt sind Verbindungen der folgenden Formeln: 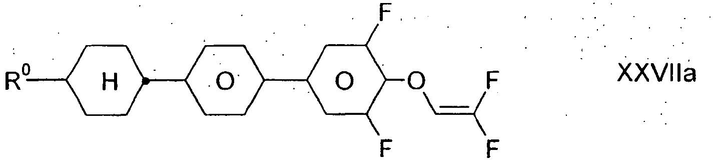 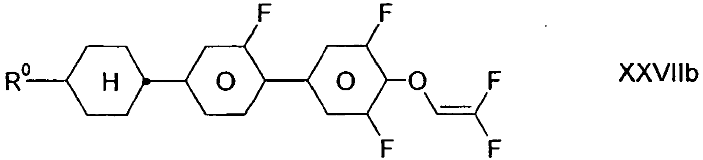 worin R⁰ die oben angegebene Bedeutung besitzt und vorzugsweise Alkyl, Alkoxy, Oxaalkyl, Fluoralkyl oder Alkenyl mit jeweils bis zu 8 C-Atomen bedeutet.
- FK-Medium, welches zusätzlich eine oder mehrere Verbindungen der folgenden Formel enthält: 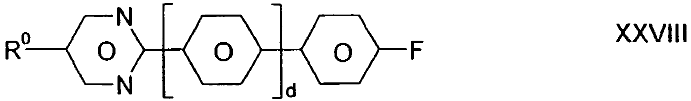 worin R⁰ die oben angegebene Bedeutung besitzt und vorzugsweise geradkettiges Alkyl mit 2-5 C-Atomen ist, und d 0 oder 1, vorzugsweise 1 bedeutet. Bevorzugte Mischungen enthalten 3 - 30 Gew.%, insbesondere 5 - 20 Gew.% dieser Verbindung(en).
- FK-Medium, welches zusätzlich eine oder mehrere Verbindungen der folgenden Formel enthält: 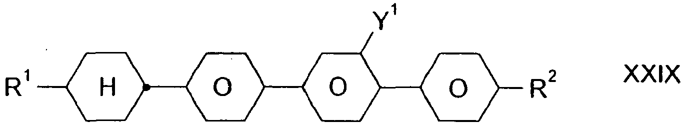 worin Y¹, R¹ und R² die oben angegebene Bedeutung besitzen. R¹ und R² bedeuten vorzugsweise jeweils unabhängig voneinander Alkyl mit 1 bis 8 C-Atomen. Y¹ bedeutet vorzugsweise F. Bevorzugte Mischungen enthalten 1 - 15 Gew.%; insbesondere 1 - 10 Gew.% dieser Verbindungen. 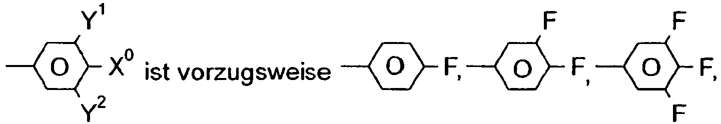 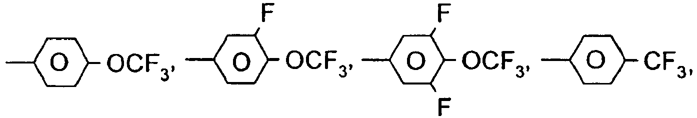 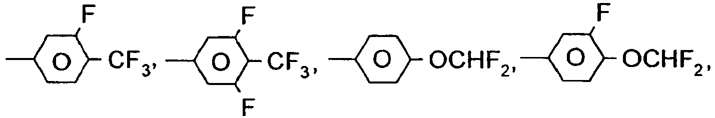 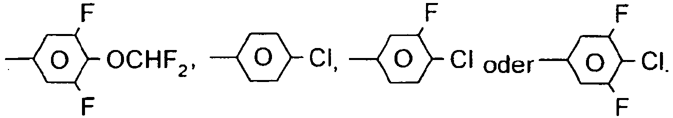
- R⁰ ist vorzugsweise geradkettiges Alkyl oder Alkenyl mit 2 bis 7 C-Atomen;
- X⁰ ist vorzugsweise F, ferner OCF₃, Cl oder CF₃;
- Das Medium enthält vorzugsweise eine, zwei oder drei Verbindungen der Formel I;
- Das Medium enthält vorzugsweise eine oder mehrere Verbindungen ausgewählt aus der Gruppe der Verbindungen der Formeln I, II, III, VI-2, XI, XII, XIII, XIV, XXIV, XXV, XXVI, XXVII und XXIX.
- Das Medium enthält vorzugsweise jeweils eine oder mehrere Verbindungen der Formel VI-2, XI und XXVI;
- Das Medium enthält vorzugsweise 1-25 Gew.%, bevorzugt 1-20 Gew.% an Verbindungen der Formel I;
- Der Anteil an Verbindungen der Formeln II-XXVIII im Gesamtgemisch beträgt vorzugsweise 20 bis 99 Gew.%;
- Das Medium enthält vorzugsweise 25-80 Gew.%, besonders bevorzugt 30-70 Gew.% an Verbindungen der Formel II und/oder III;
- Das Medium enthält vorzugsweise 20-70 Gew.%, besonders bevorzugt 25-60 Gew.% an Verbindungen der Formel IIa, insbesondere worin R^{3a} H bedeutet;
- Das Medium enthält vorzugsweise 2-20 Gew.%, besonders bevorzugt 3-15 Gew.% an Verbindungen der Formel VI-2;
- Das Medium enthält 2-20 Gew.%, besonders bevorzugt 3-15 Gew.% an Verbindungen der Formel XI;
- Das Medium enthält vorzugsweise 1-20 Gew.%, besonders bevorzugt 2-15 Gew.% an Verbindungen der Formel XXIV;
- Das Medium enthält vorzugsweise 1-25 Gew.%, besonders bevorzugt 2-20 Gew.% an Verbindungen der Formel XXVI;
- Das Medium enthält vorzugsweise 1-35 Gew.%, besonders bevorzugt 5-30 Gew.% an Verbindungen der Formel XXVII;
- Das Medium enthält vorzugsweise eine oder mehrere Verbindungen ausgewählt aus der Gruppe der Verbindungen der Formeln VI-2, VII-1a, VII-1b, IX, X, XI und XXV (CF₂O-verbrückte Verbindungen).

Es wurde gefunden, dass bereits ein relativ geringer Anteil an Verbindungen der Formel I im Gemisch mit üblichen Flüssigkristallmaterialien, insbesondere jedoch mit einer oder mehreren Verbindungen der Formeln II bis XXIX zu einer beträchtlichen Erhöhung der Lichtstabilität und zu niedrigen Werten für die Doppelbrechung führt, wobei gleichzeitig breite nematische Phasen mit tiefen Übergangstemperaturen smektisch-nematisch beobachtet werden, wodurch die Lagerstabilität verbessert wird. Gleichzeitig zeigen die Mischungen sehr niedrige Schwellenspannungen und sehr gute Werte für die VHR bei UV-Belastung

Der Ausdruck "Alkyl" bzw. "Alkyl*" umfasst in dieser Anmeldung geradkettige und verzweigte Alkylgruppen mit 1-7 Kohlenstoffatomen, insbesondere die geradkettigen Gruppen Methyl, Ethyl, Propyl, Butyl, Pentyl, Hexyl und Heptyl. Gruppen mit 1-6 Kohlenstoffatomen sind im allgemeinen bevorzugt.

Der Ausdruck "Alkenyl" bzw. "Alkenyl*" umfasst in dieser Anmeldung geradkettige und verzweigte Alkenylgruppen mit 2-7 Kohlenstoffatomen,insbesondere die geradkettigen Gruppen. Bevorzugte Alkenylgruppen sind C₂-C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele besonders bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfasst in dieser Anmeldung geradkettige Gruppen mit mindestens einem Fluoratom, vorzugsweise einem endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" bzw. "Alkoxy" umfasst in dieser Anmeldung geradkettige Reste der Formel CₙH₂ₙ₊₁-O-(CH₂)ₘ, worin n und m jeweils unabhängig voneinander 1 bis 6 bedeuten. m kann auch 0 bedeuten. Vorzugsweise ist n = 1 und m 1-6 oder m = 0 und n = 1-3.

Der Ausdruck "halogenierter Alkylrest" umfasst vorzugsweise ein- oder mehrfach fluorierte und/oder chlorierte Reste. Perhalogenierte Reste sind eingeschlossen. Besonders bevorzugt sind fluorierte Alkylreste, insbesondere CF₃, CH₂CF₃, CH₂CHF₂, CHF₂, CH₂F, CHFCF₃ und CF₂CHFCF₃.

Falls in den oben- und untenstehenden Formeln R⁰ einen Alkylrest und/oder einen Alkoxyrest bedeutet, so kann dieser geradkettig oder verzweigt sein Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxyoder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradedoxy.

Oxaalkyl bedeutet vorzugsweise geradkettiges 2-Oxapropyl (= Methoxymethyl), 2-(= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6-, oder 7-Oxaoctyl, 2-, 3-, 4-, 5-, 6-, 7- oder 8-Oxanonyl, 2-, 3-, 4-, 5-, 6-, 7-, 8- oder 9-Oxadexyl.

Falls R⁰ einen Alkylrest bedeutet, in dem eine CH₂-Gruppe durch - CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-. 6-, 7- oder Non-8-enyl, Dec-1-, 2-, 3-, 4-, 5-, 6-, 7-, 8- oder Dec-9-enyl. Diese Reste können auch ein- oder mehrfach halogeniert sein.

Falls R⁰ einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

In den oben- und untenstehenden Formeln ist X⁰ vorzugsweise F, Cl oder ein ein- oder mehrfach fluorierter Alkyl- oder Alkoxyrest mit 1, 2 oder 3 C-Atomen oder ein ein- oder mehrfach fluorierter Alkenylrest mit 2 oder 3 C-Atomen. X⁰ ist besonders bevorzugt F, Cl, CF₃, CHF₂, OCF₃, OCHF₂, OCFHCF₃, OCFHCHF₂, OCFHCHF₂, OCF₂CH₃, OCF₂CHF₂, OCF2CHF₂, OCF₂CF₂CHF₂, OCF₂CF₂CH₂F, OCFHCF₂CF_{3,} OCFHCF₂CHF₂, OCH=CF₂, OCF=CF₂, OCF₂CHFCF₃, OCF₂CF₂CF₃, OCF₂CF₂CCIF₂, OCCIFCF₂CF₃, CF=CF₂, CF=CHF, oder CH=CF₂, ganz besonders bevorzugt F oder OCF₃.

Durch geeignete Wahl der Bedeutungen von R⁰ und X⁰ können die Ansprechzeiten, die Schwellenspannung, die Steilheit der Transmissionskennlinien etc. in gewünschter Weise modifiziert werden. Beispielsweise führen 1E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten. Die erfindungsgemäßen Mischungen zeichnen sich insbesondere durch hohe K₁-Werte aus und besitzen somit deutlich schnellere Schaltzeilen als die Mischungen aus dem Stand der Technik.

Das optimale Mengenverhältnis der Verbindungen der oben genannten Formeln hängt weitgehend von den gewünschten Eigenschaften, von der Wahl der Komponenten der oben genannten Formeln und der Wahl weiterer gegebenenfalls vorhandener Komponenten ab.

Geeignete Mengenverhältnisse innerhalb des oben angegebenen Bereichs können von Fall zu Fall leicht ermittelt werden.

Die Gesamtmenge an Verbindungen der oben genannten Formeln in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die gewünschte Verbesserung der Eigenschaften der Mischung ist jedoch in der Regel umso größer je höher die Gesamtkonzentration an Verbindungen der oben genannten Formeln ist.

In einer besonders bevorzugten Ausführungsform enthalten die erfindungsgemäßen Medien Verbindungen der Formel IV bis VIII, worin X⁰ F, OCF₃, OCHF₂, OCH=CF₂, OCF=CF₂ oder OCF₂-CF₂H bedeutet. Eine günstige synergistische Wirkung mit den Verbindungen der Formel I führt. zu besonders vorteilhaften Eigenschaften. Insbesondere Mischungen enthaltend Verbindungen der Formeln I, VI und XI zeichnen sich durch ihre niedrigen Schwellenspannungen aus.

Die einzelnen Verbindungen der oben genannten Formeln und deren Unterformeln, die in den erfindungsgemäßen Medien verwendet werden können, sind entweder bekannt, oder sie können analog zu den bekannten Verbindungen hergestellt werden.

Gegenstand der Erfindung sind auch elektrooptische Anzeigen, wie z. B. TN-, STN-, TFT-, OCB-, IPS-, FFS- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes. Die erzielbaren Kombinationen aus Klärpunkt, Viskosität bei tiefer Temperatur, thermischer und UV-Stabilität und hoher optischer Anisotropie übertreffen bei weitem bisherige Materialien aus dem Stand der Technik.

Die erfindungsgemäßen Mischungen sind insbesondere für mobile Anwendungen und high-Δn-TFT-Anwendungen wie z. B. PDAs, Notebooks, LCD-TV und Monitore geeignet.

Die erfindungsgemäßen Flüssigkristallmischungen ermöglichen es, bei Beibehaltung der nematischen Phase bis -20 °C und bevorzugt bis -30 °C, besonders bevorzugt bis -40 °C, und des Klärpunkts ≥ 70 °C, vorzugsweise ≥75 °C, gleichzeitig Rotationsviskositäten γ₁ von ≤ 100 mPa·s, besonders bevorzugt ≤ 70 mPa·s zu erreichen, wodurch hervorragende MFK-Anzeigen mit schnellen Schaltzeiten erzielt werden können.

Die dielektrische Anisotropie der erfindungsgemäßen Flüssigkristallmischungen Δε ist vorzugsweise ≥ +5, besonders bevorzugt ≥ +10. Die Mischungen sind außerdem durch kleine Operationsspannungen gekennzeichnet. Die Schwellenspannung der erfindungsgemäßen Flüssigkristallmischungen ist vorzugsweise ≤ 1,5 V, insbesondere ≤ 1,2 V.

Die Doppelbrechung Δn der erfindungsgemäßen Flüssigkristallmischungen ist vorzugsweise ≥ 0,10, besonders bevorzugt ≥ 0,11.

Der nematische Phasenbereich der erfindungsgemäßen Flüssigkristallmischungen ist vorzugsweise mindestens 90°, insbesondere mindestens 100° breit. Vorzugsweise erstreckt sich dieser Bereich mindestens von -25 °C bis +70 °C.

Es versteht sich, dass durch geeignete Wahl der Komponenten der erfindungsgemäßen Mischungen auch höhere Klärpunkte (z.B. oberhalb 100 °C) bei höheren Schwellenspannungen oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringen Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften, wie z.B. hohe Steilheit der Kennlinie und geringe Winkelabhängigkeit des Kontrastes (DE-PS 30 22 818) bei gleicher Schwellenspannung wie in einer analogen Anzeige im zweiten Minimum, eine kleinere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyan-verbindungen. Der Fachmann kann durch geeignete Wahl der einzelnen Komponenten und deren Gewichtsanteilen mit einfachen Routine-methoden die für eine vorgegebene Schichtdicke der MFK-Anzeige erforderliche Doppelbrechung einstellen.

Messungen des "Voltage Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, dass erfindungsgemäße Mischungen enthaltend Verbindungen der Formel 1 eine deutlich geringere Abnahme des HR unter UV-Belastung aufweisen als analoge Mischungen enthaltend anstelle den Verbindungen der Formel 1 Cyanophenylcyclohexane der Formel 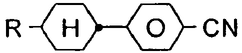 oder Ester der Formel 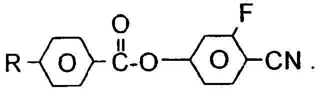 Die LC-Medien sind vorzugsweise zu 99 Gew.-%, besonders bevorzugt zu 100 Gew.-% frei von Benzonitrilderivaten.

Die Lichtstabilität und UV-Stabilität der erfindungsgemäßen Mischungen ist erheblich besser, d.h. sie zeigen eine deutlich kleinere Abnahme des HR unter Licht- bzw. UV-Belastung. Bereits geringe Konzentrationen der Verbindungen (< 10 Gew.%) der Formel I in den Mischungen erhöhen die HR gegenüber Mischungen aus dem Stand der Technik um 6 % und mehr.

Die FK-Medien können auch weitere, dem Fachmann bekannte und in der Literatur beschriebene Zusätze, wie z. B. UV-Stabilisatoren wie Tinuvin^{®} der Fa. Ciba, Antioxidantien, Radikalfänger, Nanopartikel, etc. enthalten. Beispielsweise können 0-15 % pleochroitische Farbstoffe oder chirale Dotierstoffe zugesetzt werden. Geeignete Stabilisatoren und Dotierstoffe werden nachfolgend in den Tabellen C und D genannt.

Die einzelnen Komponenten der oben genannten bevorzugten Ausführungsformen der erfindungsgemäßen FK-Medien sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren.

Es versteht sich für den Fachmann von selbst, daß die erfindungsgemäßen FK-Medien auch Verbindungen enthalten können, worin beispielsweise H, N, O, Cl, F durch die entsprechenden Isotope ersetzt sind.

Die Herstellung der erfindungsgemäß verwendbaren Flüssigkristallmischungen erfolgt in an sich üblicher Weise, beispielsweise indem man eine oder mehrere Verbindungen der Formel I mit einer oder mehreren Verbindungen der Formeln II-XXVIII oder mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Das Verfahren zur Herstellung der erfindungsgemäßen FK-Medien ist ein weiterer Gegenstand der Erfindung.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die folgenden Beispiele erläutern die vorliegende Erfindung. Sie zeigen dem Fachmann jedoch bevorzugte Mischungskonzepte mit bevorzugt einzusetzenden Verbindungen und deren jeweiligen Konzentrationen sowie deren Kombinationen miteinander. Außerdem illustrieren die Beispiele, welche Eigenschaften und Eigenschftskombinationen zugänglich sind.

In der vorliegenden Anmeldung und in den folgenden Beispielen sind die Strukturen der Flüssigkristallverbindungen durch Akronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen; n, m und k sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Akronym für den Grundkörper angegeben. Im Einzelfall folgt getrennt von Akronym für den Grundkörper mit einem Strich ein Code für die Substituenten R^{1*}, R^{2*}, L^{1*} und L^{2*}:

| Code für R^{1*}, R^{2*}, L^{1*}, L^{2*}, L^{3*} | R^{1*} | R^{2*} | L^{1*} | L^{2*} |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | F | H |
| nN.F.F | CₙH₂ₙ₊₁ | CN | F | F |
| nF | CₙH₂ₙ₊₁ | F | H | H |
| nCl | CₙH₂ₙ₊₁ | Cl | H | H |
| nOF | OCₙH₂ₙ₊₁ | F | H | H |
| nF.F | CₙH₂ₙ₊₁ | F | F | H |
| nF.F.F | CₙH₂ₙ₊₁ | F | F | F |
| nOCF₃ | CₙH₂ₙ₊₁ | OCF₃ | H | H |
| nOCF₃.F | CₙH₂ₙ₊₁ | OCF₃ | F | H |
| n-Vm | CₙH₂ₙ₊₁ | -CH=CH-CₘH₂ₘ₊₁ | H | H |
| nV-Vm | CₙH₂ₙ₊₁-CH=CH- | -CH=CH-Cₘ2_{Zm+1} | H | H |

Bevorzugte Mischungskomponenten finden sich in den Tabellen A und B.

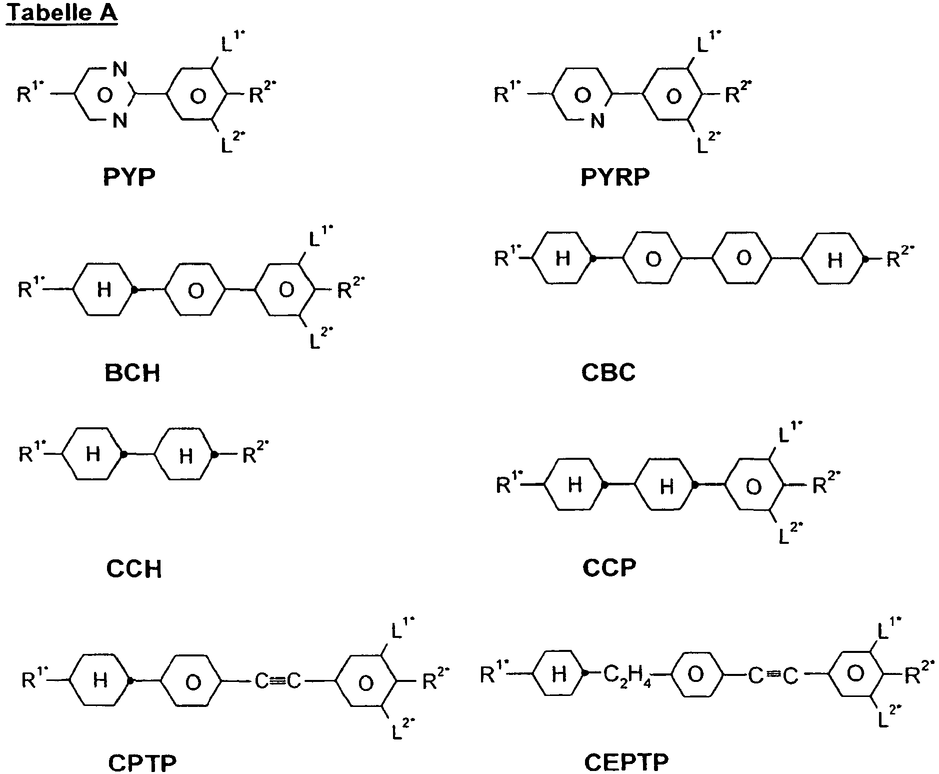

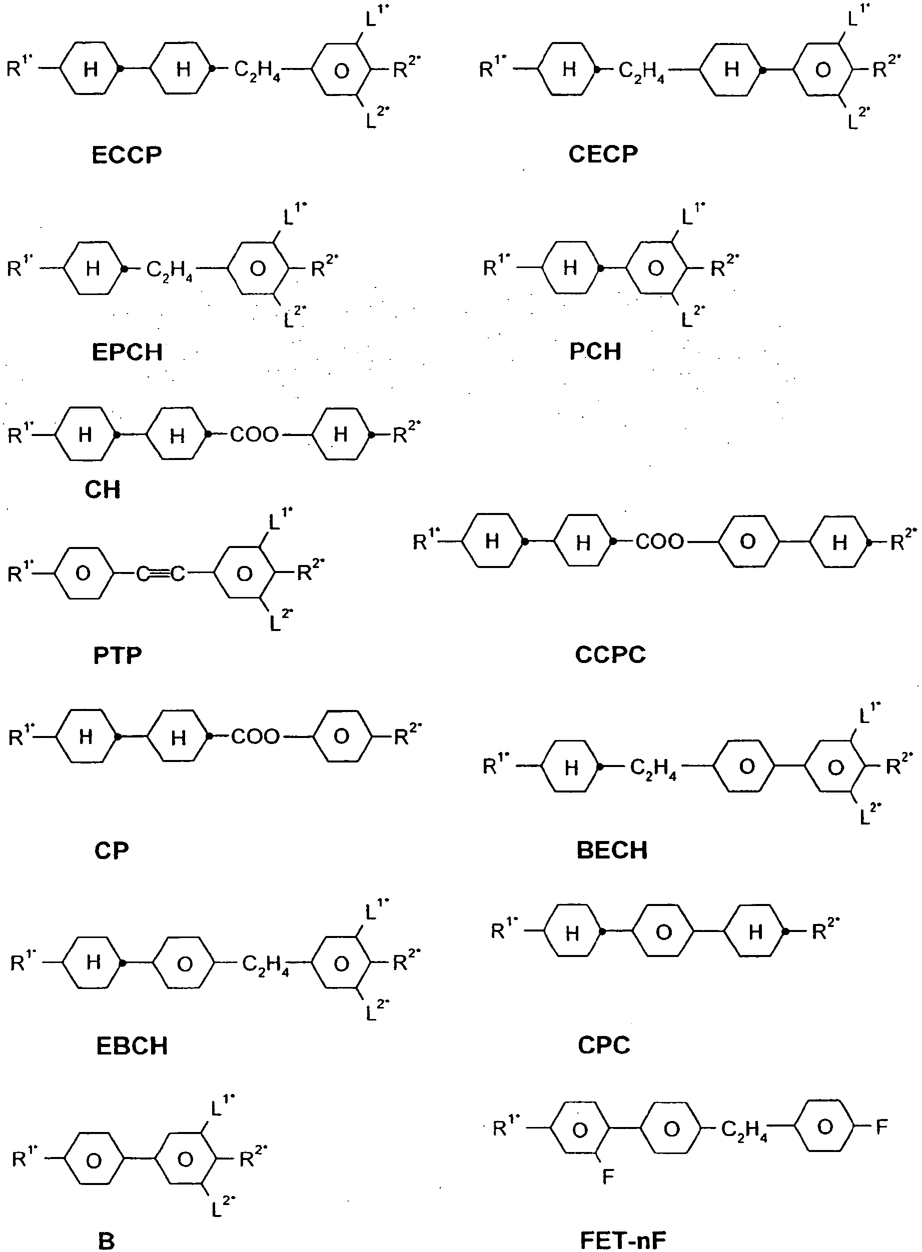

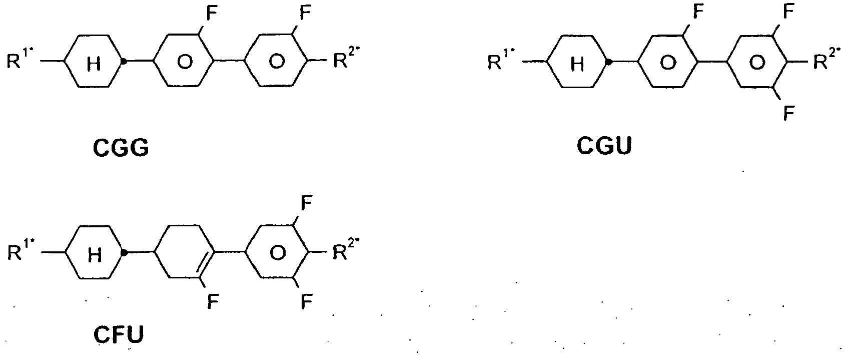

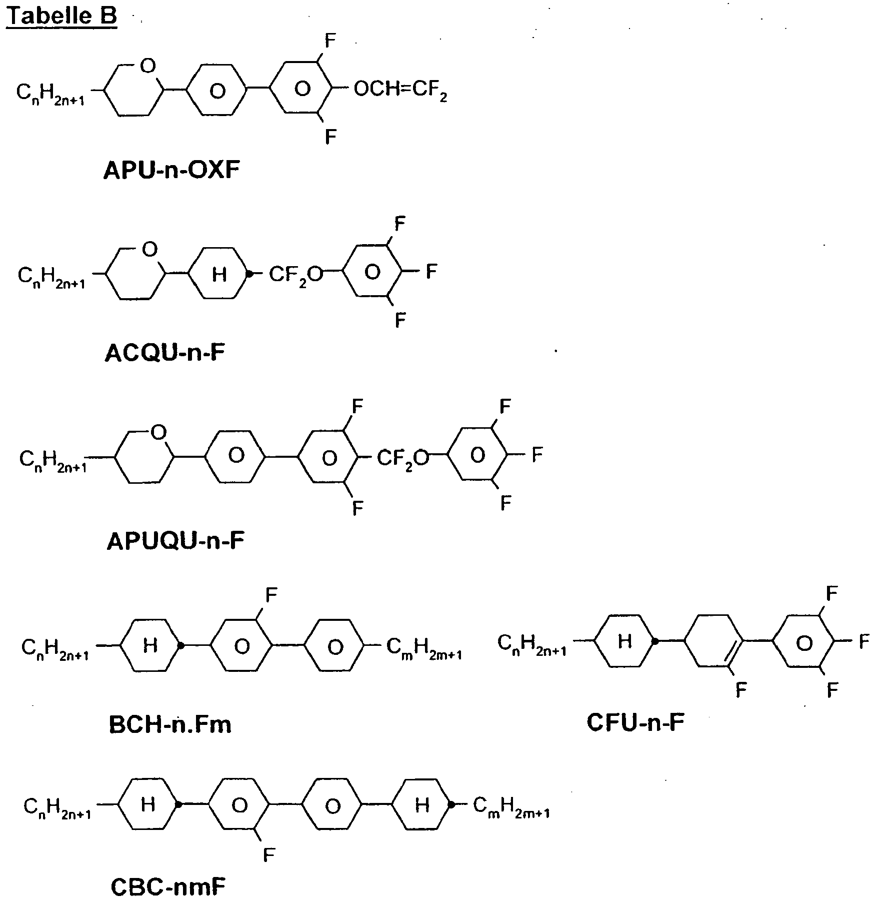

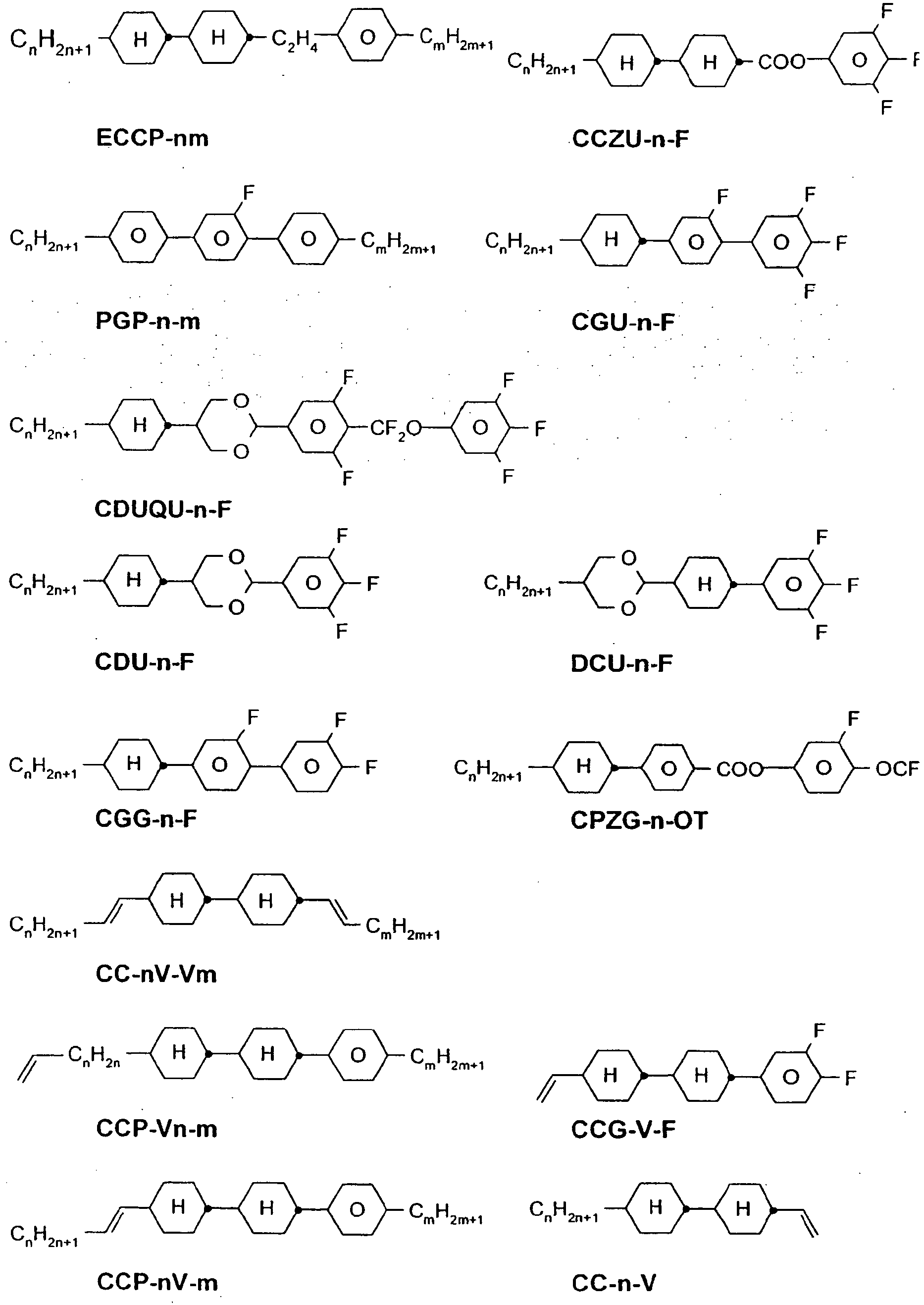

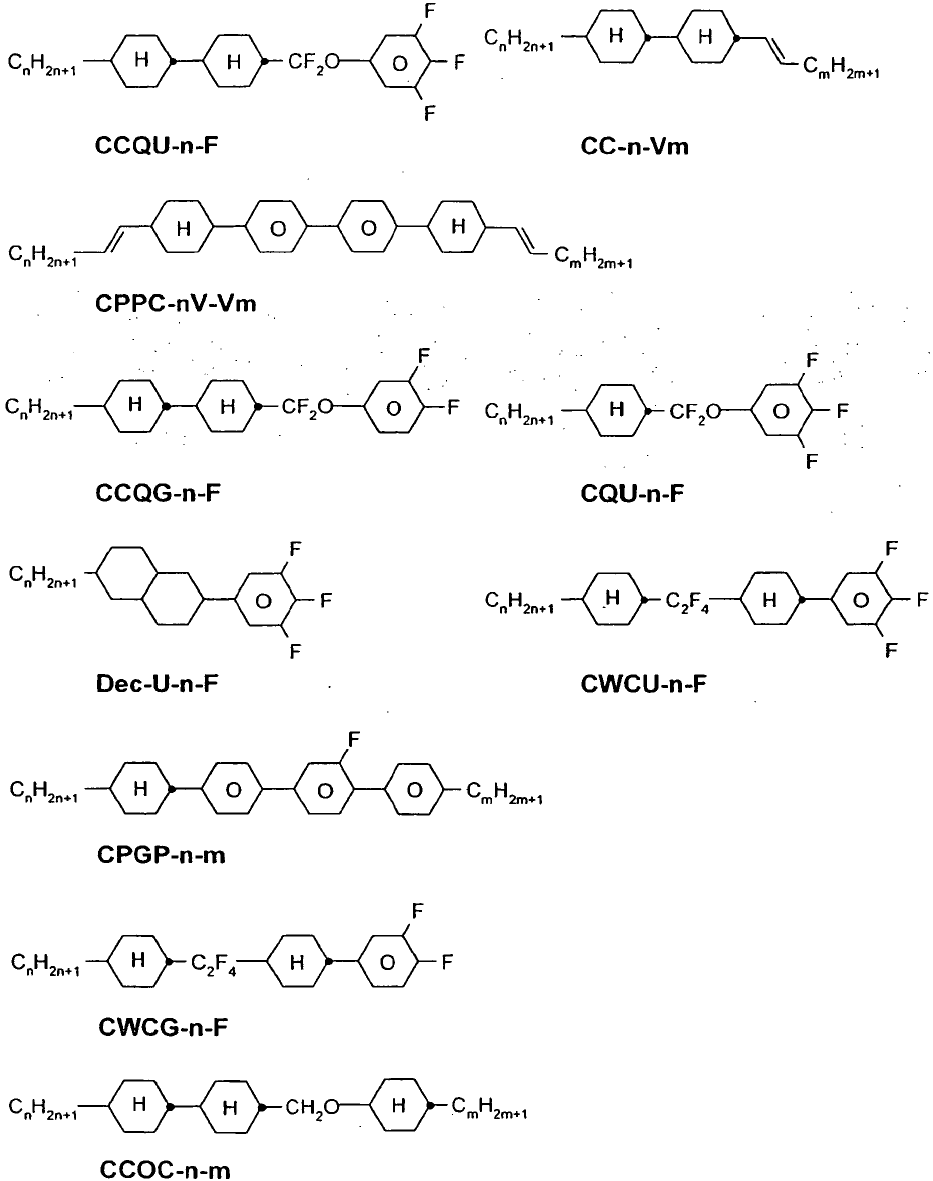

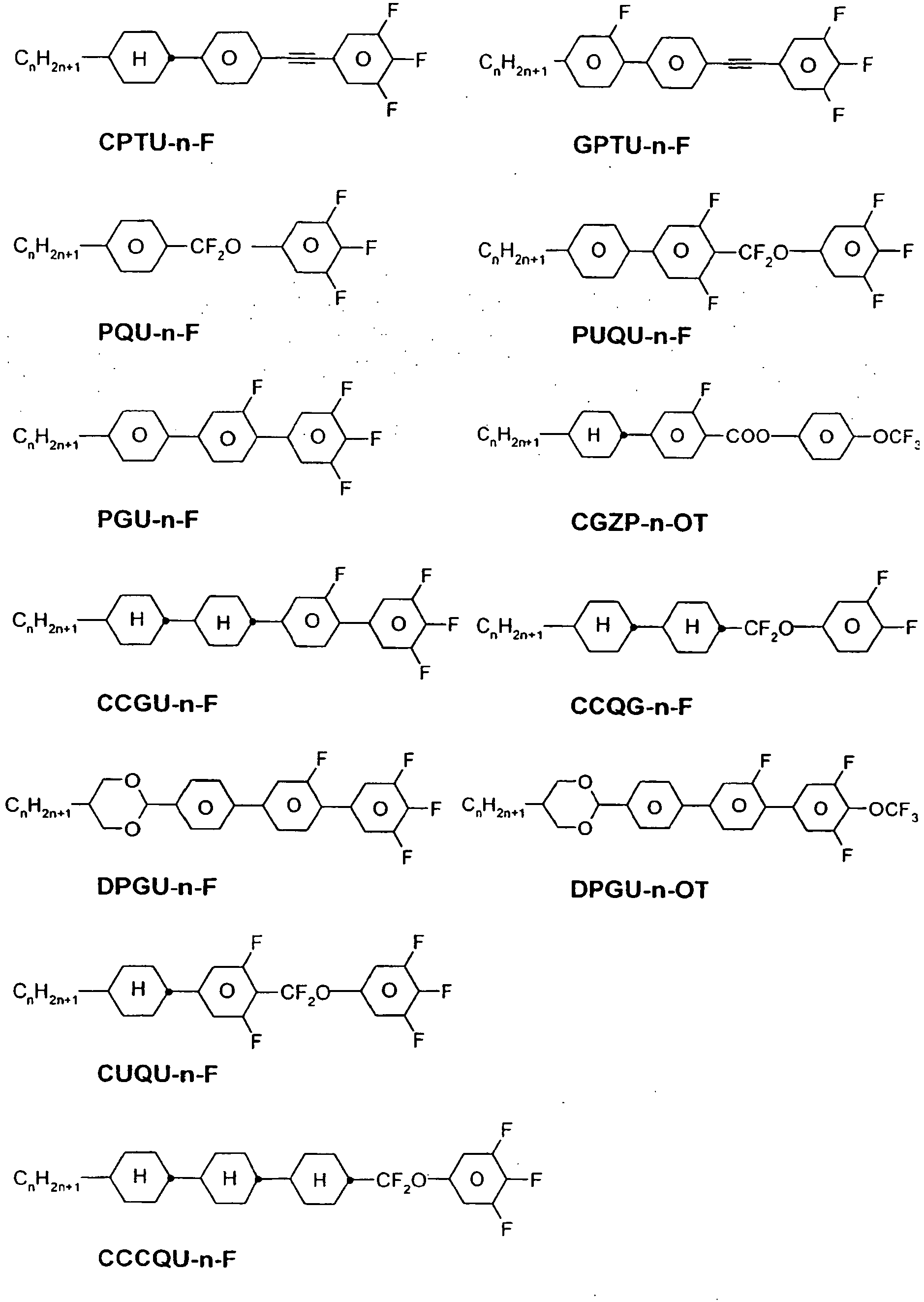

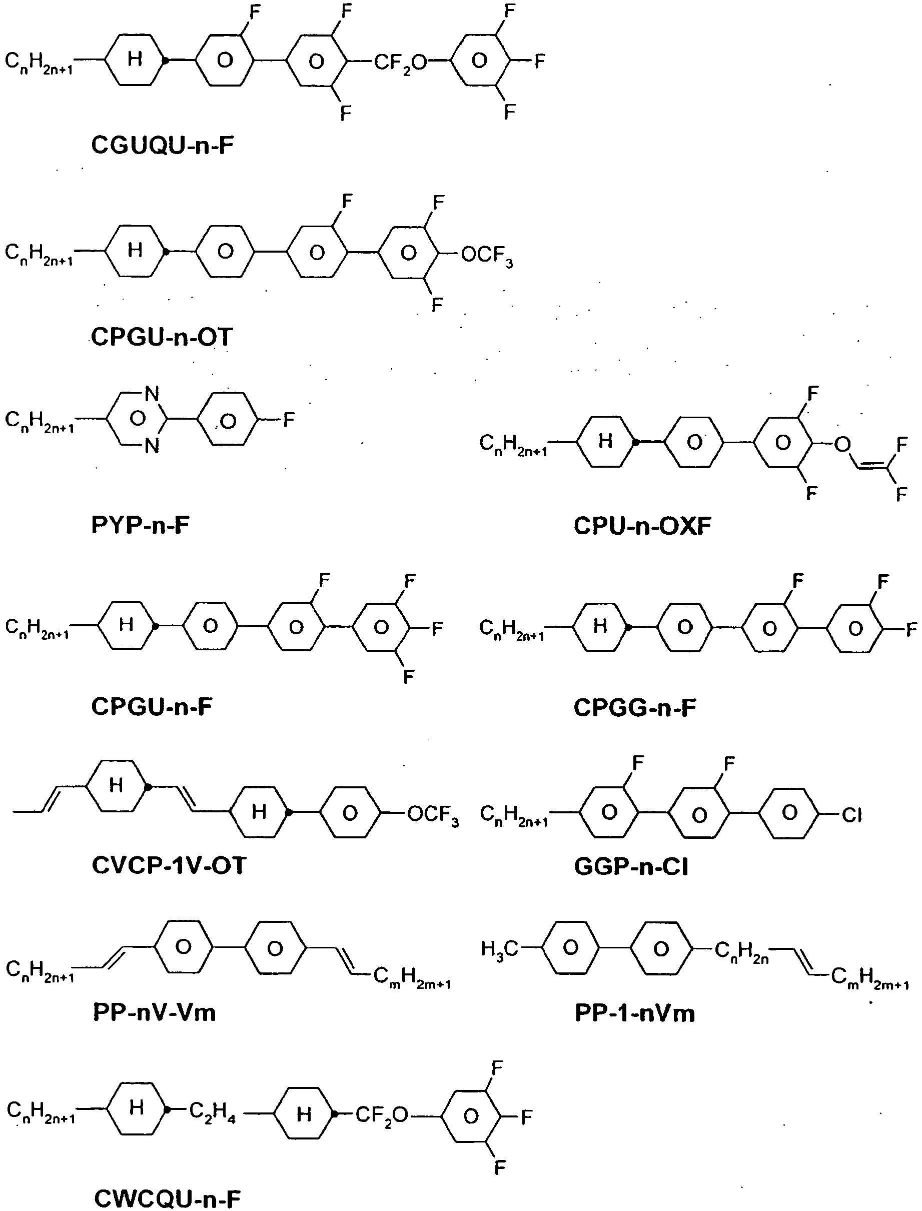

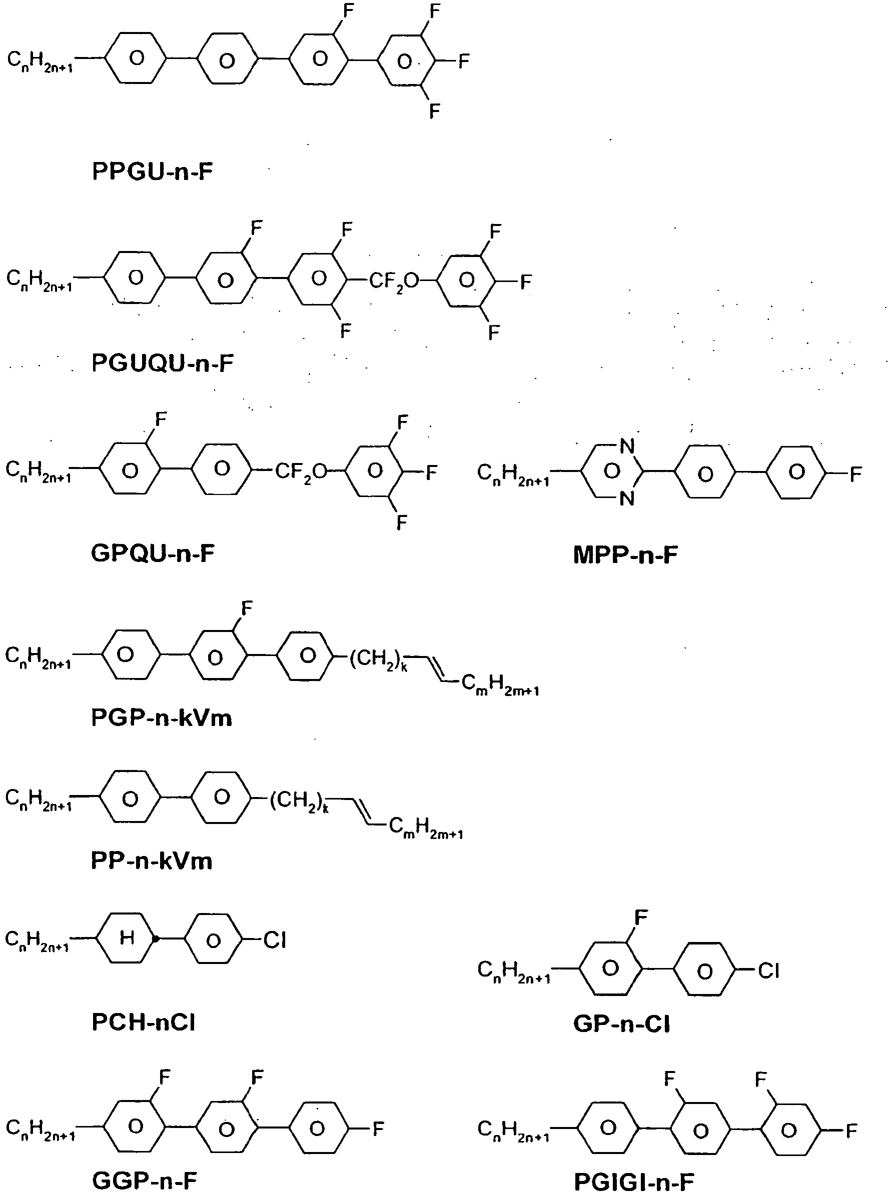

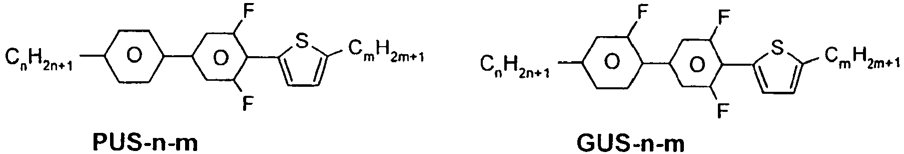

In einer bevorzugten Ausführungsform der vorliegenden Erfindung enthalten die erfindungsgemäßen FK-Medien eine oder mehrere Verbindungen ausgewählt aus der Guppe bestehend aus Verbindungen der Tabelle A und B.

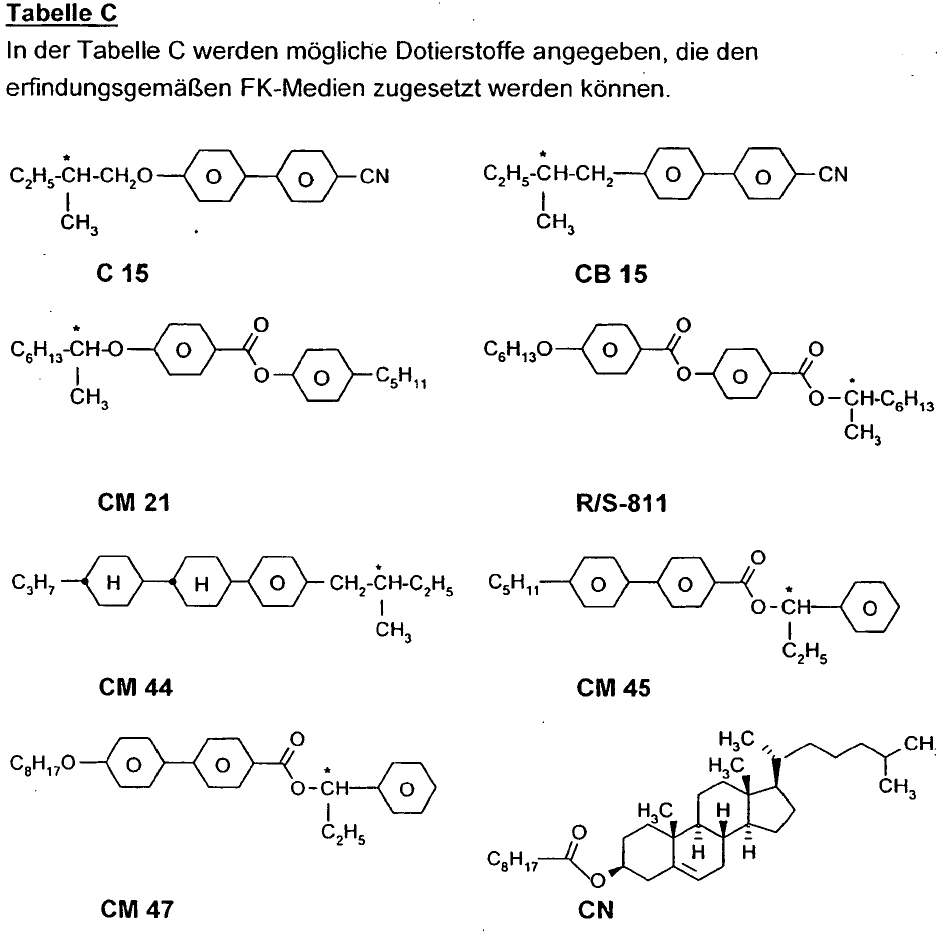

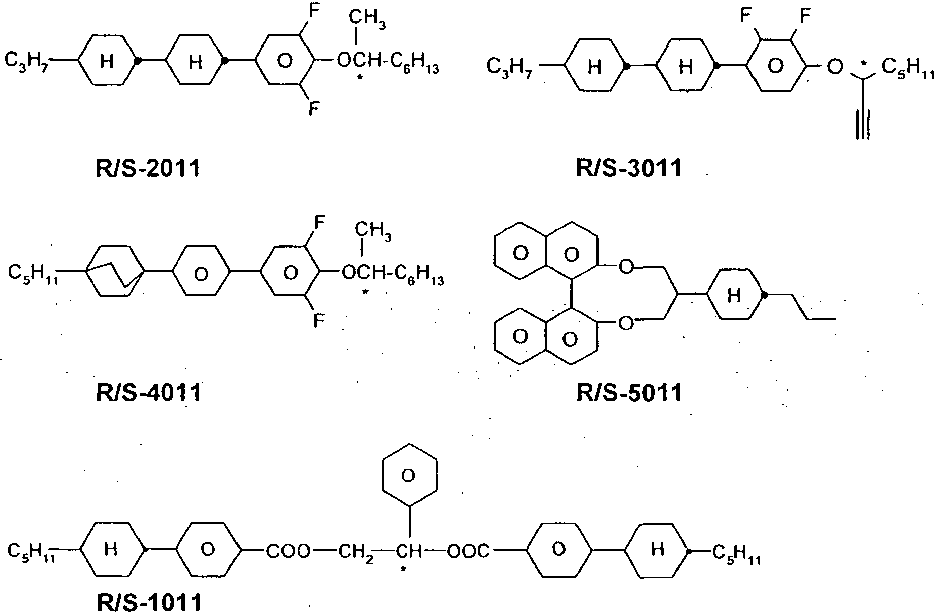

Vorzugsweise enthalten die FK-Medien 0 bis 10 Gew.%, insbesondere 0,01 bis 5 Gew.% und besonders bevorzugt 0,1 bis 3 Gew.% an Dotierstoffen. Vorzugsweise enthalten die FK-Medien einen oder mehrere Dotierstoffe ausgewählt aus der Guppe bestehend aus Verbindungen der Tabelle C.

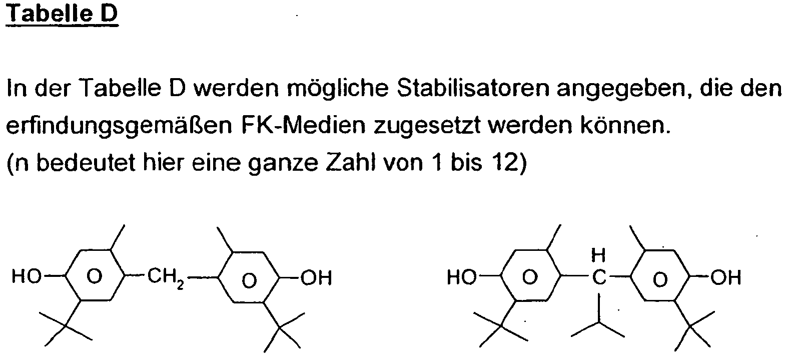

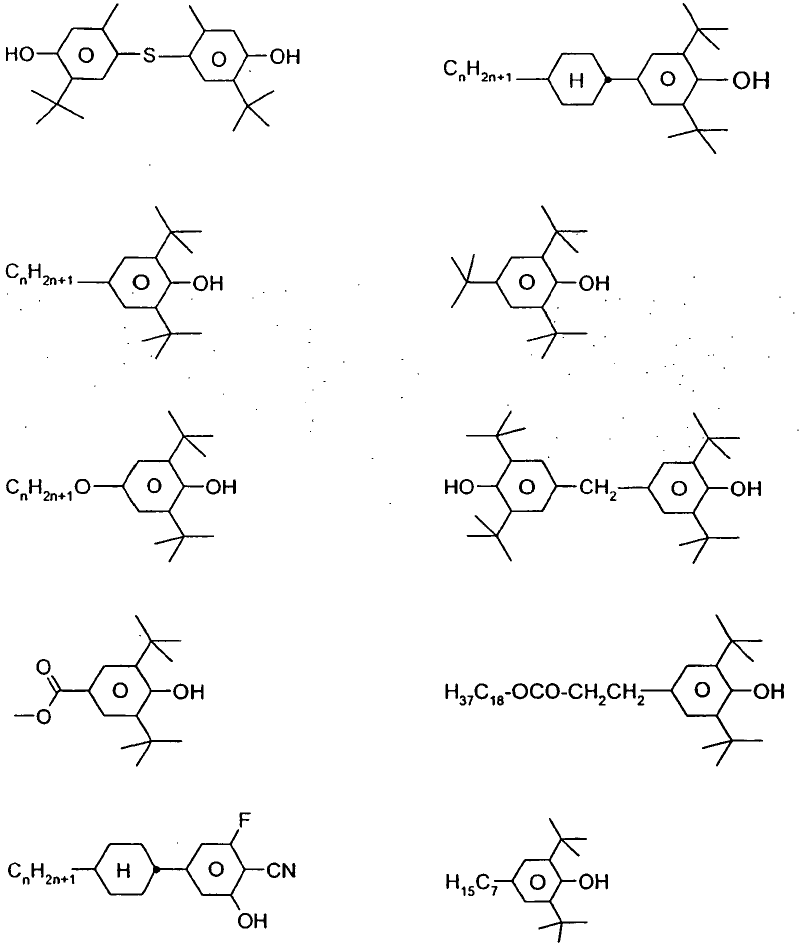

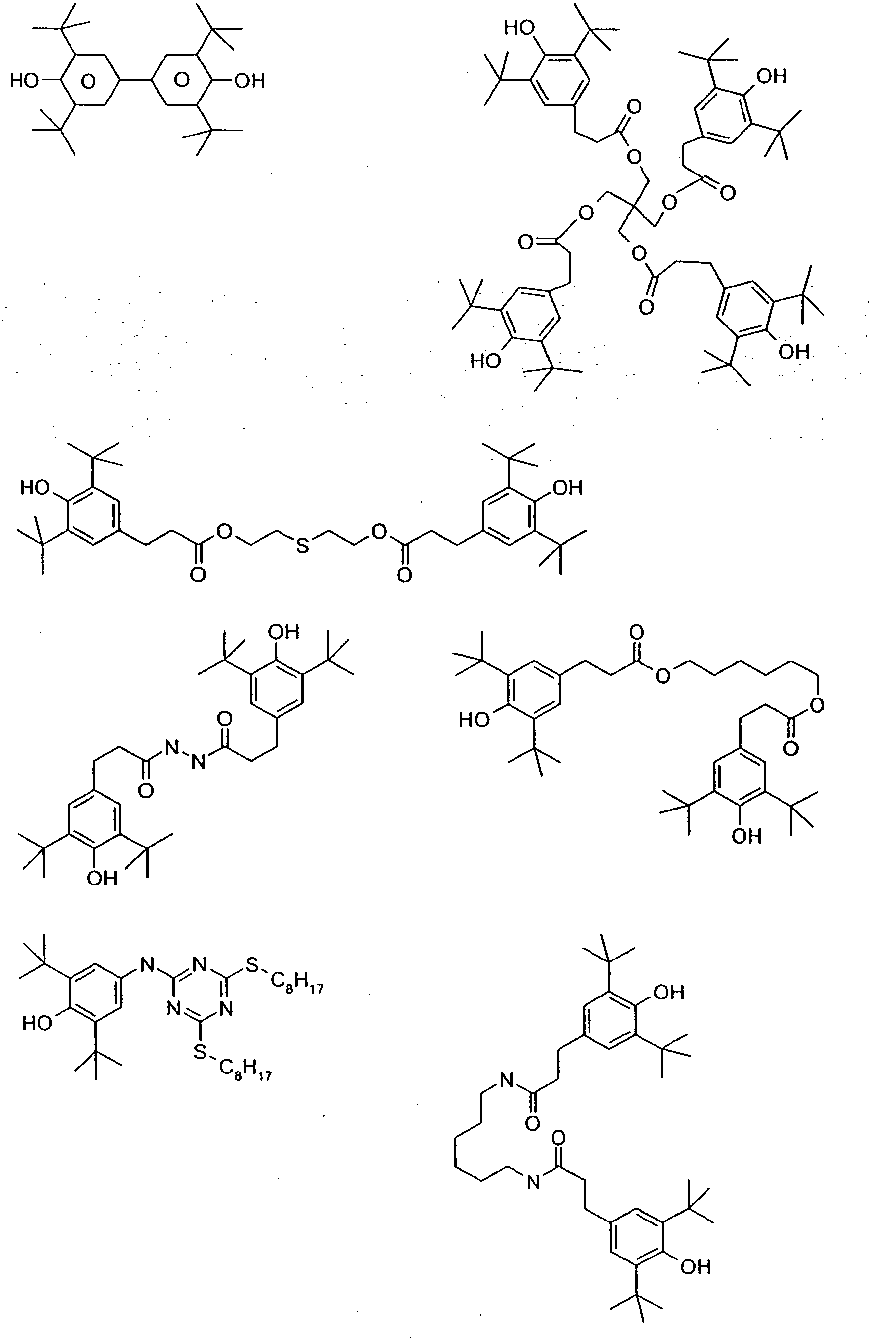

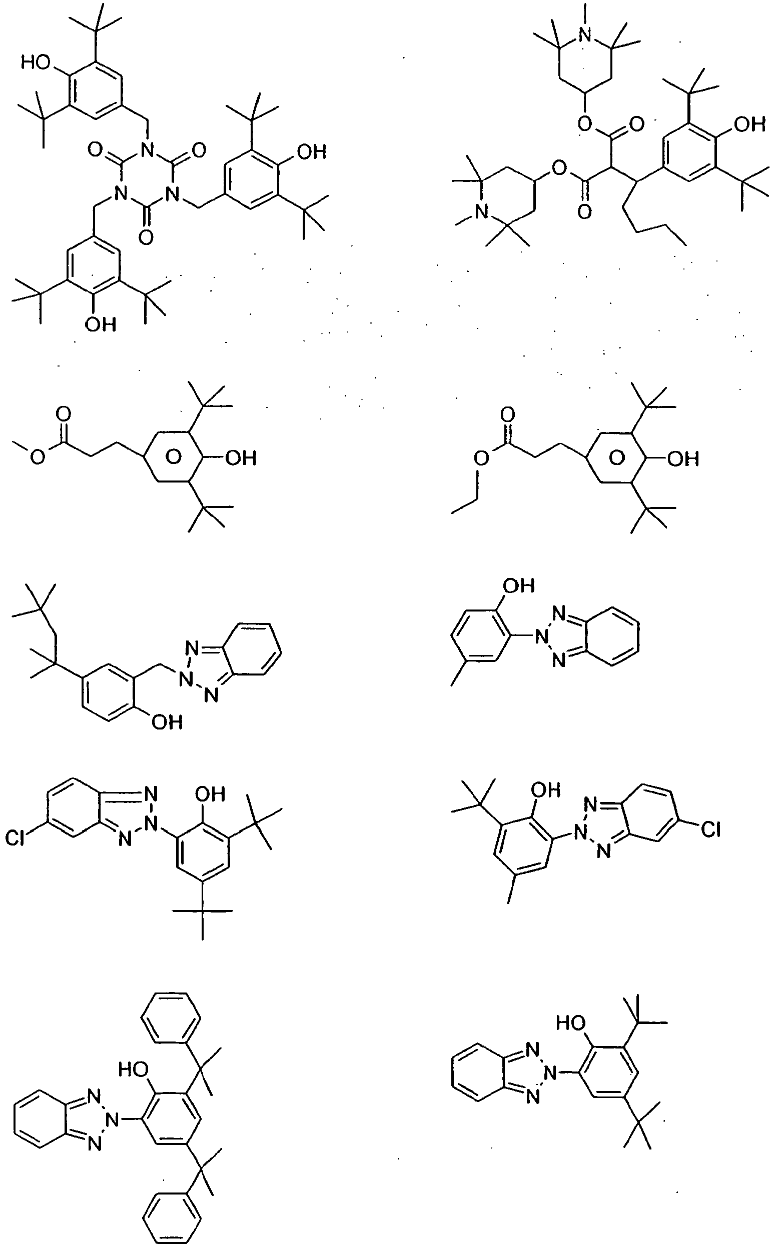

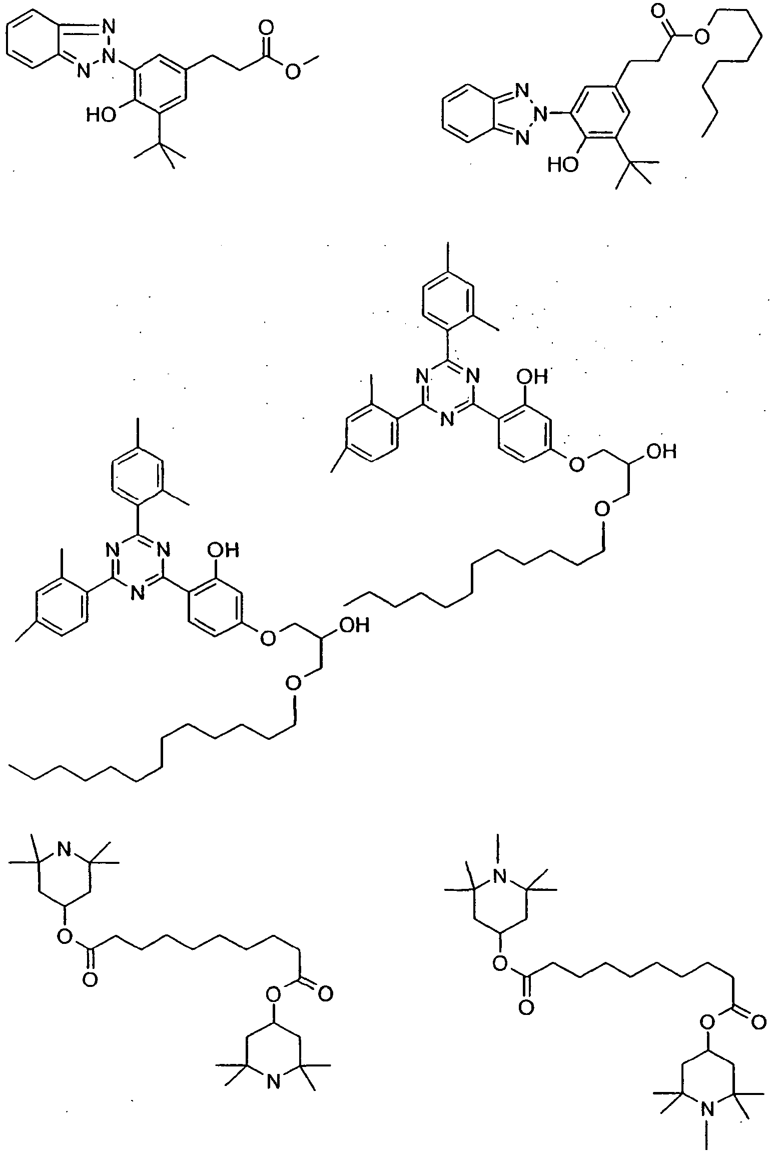

Vorzugsweise enthalten die FK-Medien 0 bis 10 Gew.%, insbesondere 0,01 bis 5 Gew.% und besonders bevorzugt 0,1 bis 3 Gew.% an Stabilisatoren. Vorzugsweise enthalten die FK-Medien einen oder mehrere Stabilisatoren ausgewählt aus der Gruppe bestehend aus Verbindungen der Tabelle D.

Außerdem werden folgende Abkürzungen und Symbole verwendet:
- Vₒ: Schwellenspannung, kapazitiv [V] bei 20°C,
- V₁₀: optische Schwelle für 10 % relativen Kontrast [V] bei 20°C,
- nₑ: außerordentlicher Brechungsindex bei 20°C und 589 nm,
- nₒ: ordentlicher Brechungsindex bei 20°C und 589 nm,
- Δn: optische Anisotropie bei 20°C und 589 nm,
- ε_{⊥}: dielektrische Suszeptibilität senkrecht zum Direktor bei 20°C und 1 kHz,
- ε_{∥}: dielektrische Suszeptibilität parallel zum Direktor bei 20°C und 1 kHz,
- Δε: dielektrische Anisotropie bei 20°C und 1 kHz,
- Kp., T(N,I): Klärpunkt [°C],
- γ₁: Rotationsviskosität bei 20°C [mPa·s],
- K₁: elastische Konstante, "splay"-Deformation bei 20°C [pN],
- K₂: elastische Konstante, "twist"-Deformation bei 20°C [pN],
- K₃: elastische Konstante, "bend"-Deformation bei 20°C [pN],
- LTS: "low temperature stability" (Phase), bestimmt in Testzellen,
- HR₂₀: "voltage holding ratio" bei 20°C [%] und
- HR₁₀₀: "voltage holding ratio" bei 100°C [%].

Soweit nicht explizit anders vermerkt, sind in der vorliegenden Anmeldung alle Konzentrationen in Gewichtsprozent angegeben und beziehen sich auf die entsprechende Gesamtmischung ohne Lösungsmittel.

Soweit nicht explizit anders vermerkt, sind in der vorliegenden Anmeldung alle angegebenen Werte für Temperaturen, wie z. B. der Schmelzpunkt T(C,N), der Übergang von der smektischen (S) zur nematischen (N) Phase T(S,N) und der Klärpunkt T(N,I), in Grad Celsius (°C) angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar.

Alle physikalischen Eigenschaften werden und wurden nach "Merck Liquid Crystals, Physical Properties of Liquid Crystals", Status Nov. 1997, Merck KGaA, Darmstadt, Deutschland bestimmt und gelten für eine Temperatur von 20°C und Δn wird bei 589 nm und Δε bei 1 kHz bestimmt, sofern nicht jeweils explizit anders angegeben.

Die flüssigkristallinen Eigenschaften der Einzelverbindungen werden, soweit nicht anders angegeben, in der nematischen Wirtsmischung ZLI-4792 (kommerziell erhältlich von Merck KGaA, Darmstadt) bei einer Konzentration von 10% bestimmt.

"Raumtemperatur" bedeutet 20°C, falls nicht anders angegeben.

Der Begriff "Schwellenspannung" bezieht sich für die vorliegende Erfindung auf die kapazitive Schwelle (V₀), auch Freedericksz-Schwelle genannt, sofern nicht explizit anders angegeben. In den Beispielen kann auch, wie allgemein üblich, die optische Schwelle für 10 % relativen Kontrast (V₁₀) angegeben werden.

Die zur Messung der kapazitiven Schwellenspanung Vₒ sowie für V₁₀ verwendeten Testzellen sind aufgebaut aus Substraten bestehend aus Natriumglas (Sodalime Glas), die mit Polyimidorientierungsschichten (Durimid 32 mit Verdünner (70 % NMP + 30 % Xylol) im Verhältnis 1:4) der Firma Arch Chemicals beschichtet sind, welche antiparallel zueinander gerieben sind und einen Oberflächentilt von quasi 0 Grad aufweisen. Die Fläche der durchsichtigen, nahezu quadratischen Elektroden aus ITO, beträgt 1 cm². Zur Bestimmung der kapazitiven Schwellenspannung wird ein handelsübliches hochauflösendes LCR-Meter (z.B. LCR-Meter 4284A der Firma Hewlett Packard) eingesetzt.

### Beispiel 1: 2-(3,5-Difluor-4'-propyl-biphenyl-4-yl)-5-methyl-thiophen (PUS-3-1)

Die erfindungsgemäße Verbindung 2-(3,5-Difluor-4'-propyl-biphenyl-4-yl)-5-methyl-thiophen (PUS-3-1) wird hergestellt wie nachfolgend beschrieben:

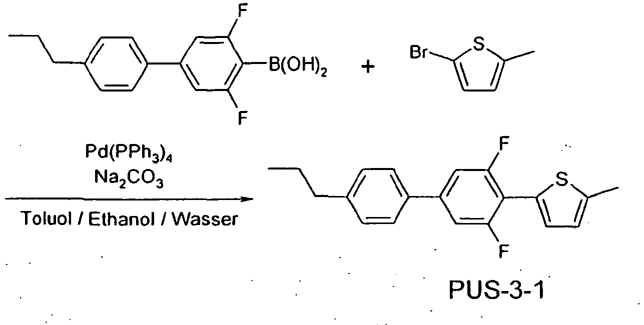

Eine Mischung aus 5,50 g (31,0 mmol) 2-Brom-5-methylthiophen, 9,0 g (32,6 mmol) 4'-Propyl-3,5-difluor-4-biphenylboronsäure, 1,83 g (1,58 mmol) Tetrakis-(triphenylphosphin)palladium(O) und 36 ml 2 N Natriumcarbonatlsg. in 90 ml Toluol/Ethanol (1 : 2) wird 16 h zum Rückfluss erhitzt. Nach dem Abkühlen wird die organische Phase abgetrennt, und die wässrige Phase wird mehrfach mit Toluol extrahiert. Die vereinigten organischen Phasen werden mit Wasser und ges. Natriumchloridlsg. gewaschen. Die Lösung wird mit Natriumsulfat getrocknet und vollständig konzentriert. Der Rückstand wird säulenchromatographisch (SiO₂, n-Heptan : Toluol = 9 : 1) gereinigt. Weitere Reinigung erfolgt durch Umkristallisation aus Ethanol und n-Heptan; 2-(3,5-Difluor-4'-propyl-biphenyl-4-yl)-5-methyl-thiophen (PUS-3-1) wird als farbloser Feststoff (Schmp. 75 °C) erhalten.
**¹H-NMR** (300 MHz, CHCl₃): δ = 7,49 (d, 2H, J = 8,4 Hz, H*ₐᵣₒₘ.*),7,39 (d, 1H, J = 3,6 Hz, H*ₐᵣₒₘ*.), 7,28-7,16 (m, 4H, H*ₐᵣₒₘ*.), 6,82-6,80 (m, 1H, H_{*arom*.}), 2,63 (t, 2H, J = 7,5 Hz, H₃CCH₂**CH₂**-), 2,54 (d, 3H, J = 0,6 Hz, **CH₃**), 1,74-1,61 (m, 2H, H₃C**CH₂**CH₂-), 0,97 (t, 3H, J = 7,5 Hz, H₃CCH₂CH₂-).
**¹⁹F-NMR** (376 MHz, CHC1₃): δ = -110,5 (d, 2F, J = 10.9 Hz).
**MS (EI):** m/z(%) = 328 (100, M⁺), 299 (79, [M - C₂H₅]⁺)
Δε = +3,1
Δn = 0,3055
γ_{I} = 102 mPa·s
K 75N 107 I

Beispiel 2: 2-(3,5-Difluor-4'-propyl-biphenyl-4-yl)-5-ethyl-thiophen (PUS-3-2) Die erfindungsgemäße Verbindung 2-(3,5-Difluor-4'-propyl-biphenyl-4-yl)-5-ethyl-thiophen (PUS-3-2) wird hergestellt wie nachfolgend beschrieben: 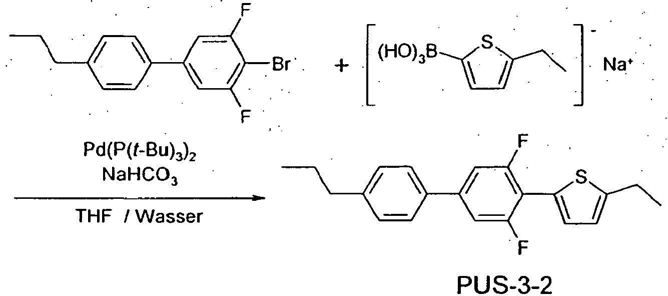 17,0 g (54,6 mmol) 4-Brom-3,5-difluor-4'-propyl-biphenyl, 16,1 g (82,1 mmol) 2-Ethylthiophen-5-hydroxyboronsäurenatriumsalz und 9,2 g (0,11 mol) Natriumhydrogencarbonat werden zusammen mit 700 mg (1,37 mmol) Bis(tri-*tert*-butylphosphin)palladium(O) in 250 ml THF-WasserGemisch (2 : 1) refluxiert. Nach 22 h wird die Mischung mit MTBE verdünnt, und die organische Phase wird abgetrennt. Die wässrige Phase wird mehrfach mit MTBE extrahiert, und die vereinigten organischen Phasen werden mit Wasser und gesättigter Natriumchloridlösung gewaschen. Die Lösung wird mit Natriumsulfat getrocknet und vollständig konzentriert. Das Rohprodukt wird säulenchromatographisch (SiO₂, n-Heptan) gereinigt. Die weitere Reinigung erfolgt durch Umkristallisation aus Ethanol und n-Heptan. 2-(3,5-Difluor-4'-propyl-biphenyl-4-yl)-5-ethyl-thiophen (PUS-3-2) wird als farbloser Feststoff (Schmp. 45 °C) erhalten.
**¹H-NMR** (400 MHz, CHCl₃): δ = 7,49 (d, 2H, J = 8,4 Hz, H_{*arom*.}),7,41 (d, 1H, J = 3,6 Hz, H*ₐᵣₒₘ*.), 7,28-7,17 (m, 4H, H_{*arom*.}), 6,85-6,83 (m, 1H, H_{*arom*.}), 2,90 (q, 2H, J = 7,5 Hz, **CH₂**CH₃), 2,63 (t, 2H, J = 7,6 Hz, H₃CCH₂**CH₂**-), 1,74-1,61 (m, 2H, H₃C**CH₂**CH₂-), 1,36 (t, 3H, J = 7,5 Hz, CH₂**CH₃**), 0,97 (t, 3H, J = 7,6 Hz, **H₃C**CH₂CH₂-).
**¹⁹F-NMR** (376 MHz, CHCl₃): δ = -110,5 (d, 2F, J = 11.0 Hz).
**MS (EI):** m/z(%) = 342 (100, M⁺), 327 (80, [M - CH₃]⁺).
Δε = +3,8
Δn = 0,2939
γ_{I} = 76 mPa·s
K 45 N 90 I

### Beispiel 3: 2-(3,5-Difluor-4'-propyl-biphenyl-4-yl)-5-propyl-thiophen (PUS-3-3)

Die erfindungsgemäße Verbindung 2-(3,5-Difluor-4'-propyl-biphenyl-4-yl)-5-propyl-thiophen (PUS-3-3) wird hergestellt wie nachfolgend beschrieben: 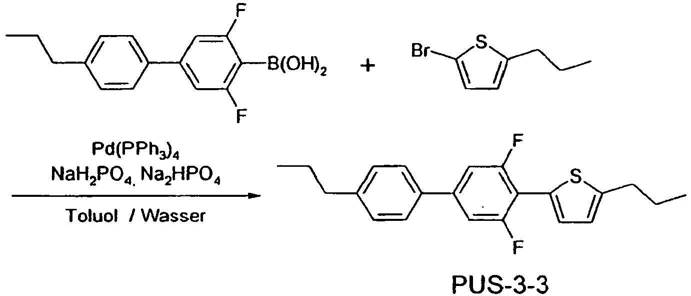 5,0 g (24,4 mmol) 2-Brom-5-propylthiophen, 7,40 g (26,8 mmol) 4'-Propyl-3,5-difluor-4-biphenylboronsäure und 2,82 g (2,44 mmol) Tetrakis-(triphenylphosphin)palladium(0) werden in 160 ml Toluol vorgelegt. Eine Lösung von 5,85 g (48,8 mmol) Natriumdihydrogenphosphat und 13,8 g (97,5 mmol) di-Natriumhydrogenphosphat-dodecahydrat in 50 ml Wasser wird zugegeben, und die Mischung wird 19 h zum Rückfluß erhitzt. Nach dem Abkühlen wird die organische Phase abgetrennt, und die wässrige Phase wird mehrfach mit Toluol extrahiert. Die vereinigten organischen Phasen werden mit Wasser und ges. Natriumchloridlsg. gewaschen. Die Lösung wird mit Natriumsulfat getrocknet und vollständig konzentriert. Der Rückstand wird säulenchromatographisch (SiO₂, n-Heptan) gereinigt. Weitere Reinigung erfolgt durch Umkristallisation aus Ethanol und *n*-Heptan; 2-(3,5-Difluor-4'-propyl-biphenyl-4-yl)-5-propyl-thiophen (PUS-3-3) wird als farbloser Feststoff (Schmp. 64 °C) erhalten.
**¹H-NMR** (400 MHz, CHCl₃): δ = 7,49 (d, 2H, J = 8,1 Hz, H_{*arom*.}),7,42 (d, 1H, J = 3,6 Hz, H_{*arom*.}), 7,28-7,17 (m, 4H, H_{*arom*.}), 6,85-6,83 (m, 1H, H_{*arom*.}), 2,84 (t, 2H, J = 7,6 Hz, **-CH₂**CH₂CH₃), 2,63 (t, 2H, J = 7,6 Hz, H₃CCH₂CH₂-), 1,81-1,63 (m, 4H, H₃CCH₂CH₂-, -CH₂**CH₂**CH₃), 1,01 (t, 3H, J = 7,6 Hz, -CH₂CH₂CH₃), 0,97 (t, 3H, J = 7,6 Hz, H₃CCH₂CH₂-). **¹⁹F-NMR** (376 MHz, CHCl₃): δ = -110,5 (d, 2F, J = 10.9 Hz).
**MS (EI):** m/z(%) = 358 (67, M⁺), 327 (100, [M - C₂H₅]⁺).
Δε = +4,2
Δn = 0,2831
y_{I} = 67 mPa·s
K 64 N 86 I

### Beispiel 4: 2-(3,5-Difluor-4'-ethyl-biphenyl-4-yl)-5-ethyl-thiophen (PUS-2-2)

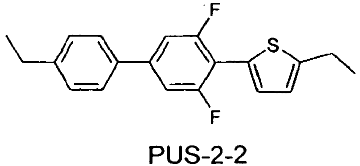

Die erfindungsgemäße Verbindung 2-(3,5-Difluor-4'-ethyl-biphenyl-4-yl)-5-ethyl-thiophen (PUS-2-2) wird analog zu Beispiel 3 aus 4'-Ethyl-3,5-difluor-4-biphenylboronsäure und 2-Brom-5-ethylthiophen hergestellt.
**¹H-NMR** (400 MHz, CHCl₃): δ = 7,49 (d, 2H, J = 8,1 Hz, H_{*arom*.}), 7.42 (d, 1H, J = 3,6 Hz, H*ₐᵣₒₘ*.), 7,28-7,17 (m, 4H, H_{*arom*.}), 6,85-6,83 (m, 1H, H_{*arom*,}), 2,90 (q, 2H, J = 7,4 Hz, **-CH₂**CH₃), 2,69 (q, 2H, J = 7,4 Hz, H₃C**CH₂**-), 1,36 (t, 3H, J = 7,6 Hz, -CH₂**CH₃**), 1,28 (t, 3H, J = 7,6 Hz, **H₃C**CH₂-).
**¹⁹F-NMR** (376 MHz, CHCl₃): ö = -110,5 (d, 2F, J = 10.9 Hz).
**MS (EI):** m/z(%) = 328 (98, M⁺), 313 (100, [M - CH₃]⁺).
Δε = +5,4
Δn = 0,2969
γ_{I} = 58 mPa·s
K 48 N 62 I

### Beispiel 5: 2-(3,5-Difluor-4'-ethyl-biphenyl-4-yl)-5-propyl-thiophen (PUS-2-3)

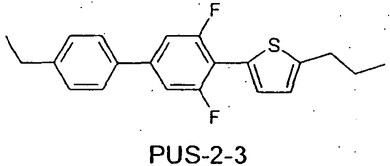

Die erfindungsgemäße Verbindung 2-(3,5-Difluor-4'-ethyl-biphenyl-4-yl)-5-propyl-thiophen (PUS-2-3) wird analog zu Beispiel 3 aus 4'-Ethyl-3,5-difluor-4-biphenylboronsäure und 2-Brom-5-propylthiophen hergestellt.
**¹H-NMR** (400 MHz, CHCl₃): δ = 7,49 (d, 2H, J = 8,1 Hz, H_{*arom*.}),7,42 (d, 1H, J = 3,6 Hz, H_{*arom*.}), 7,28-7,17 (m, 4H, H_{*arom*.}), 6,85-6,83 (m, 1H, H_{*arom*.}), 2,84 (t, 2H, J = 7,5 Hz, **-CH₂**CH₂CH₃), 2,70 (q, 2H, J = 7,4 Hz, H₃CCH₂-), 1,81-1,71 (m, 2H, -CH₂**CH**₂CH₃), 1,27 (t, 3H, J = 7,5 Hz, -CH₂CH₂**CH₃**), 1,01 (t, 3H, J = 7,4 Hz, **H₃C**CH₂-).
**¹⁹F-NMR** (376 MHz, CHCl₃): ö = -110,5 (d, 2F, J = 10.9 Hz).
**MS (EI):** m/z(%) = 342 (58, M⁺), 313 (100, [M - C₂H₅]⁺).
Δε = +3,3
Δn = 0,2829
γ₁ = 61 mPa·s
K 50 N 61 I

### Beispiel 6: 2-(4'-Ethyl-2,6-difluor-biphenyl-4-yl)-5-propyl-thiophen (SUP-3-2)

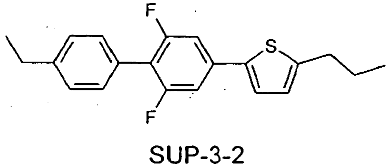

Die erfindungsgemäße Verbindung 2-(4'-Ethyl-2,6-difluor-biphenyl-4-yl)-5-propyl-thiophen (SUP-3-2) wird analog zu Beispiel 1 aus 4'-Ethyl-2,6-difluor-4-biphenylboronsäure und 2-Brom-5-propylthiophen hergestellt.
**¹H-NMR** (400 MHz, CHCl₃): δ = 7,41 (d, 2H, J = 8,2 Hz, H_{*arom*.}),7,29 (d, 2H, J = 8,2 Hz, H_{*arom*.}), 7,19-7,12 (m, 3H, H_{*arom*.}), 6,76 (d, 1H, J = 3,4 Hz, H_{*arom*.}), 2,80 (t, 2H, J = 7,6 Hz, **-CH₂**CH₂CH₃), 2,70 (q, 2H, J = 7,6 Hz, H₃C**CH₂**-), 1,78-1,69 (m, 2H, -CH₂**CH₂**CH₃), 1,28 (t, 3H, J = 7,6 Hz, -CH₂CH₂**CH₃**), 1,00 (t, 3H, J = 7,6 Hz, **H₃C**CH₂-).
**¹⁹F-NMR** (376 MHz, CHCl₃): δ = -114,5 (d, 2F, J = 8,3 Hz).
**MS (EI):** m/z(%) = 342 (61, M⁺), 313 (100, [M - C₂H₅]⁺).
Δε = +5,5
Δn = 0,2332
γ_{I} = 110 mPa·s
K 80 I

### Beispiel 7: 2-(4'-Ethyl-2,6-difluor-biphenyl-4-yl)-5-ethyl-thiophen (SUP-2-2)

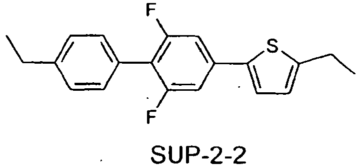

Die erfindungsgemäße Verbindung 2-(4'-Ethyl-2,6-difluor-biphenyl-4-yl)-5-ethyl-thiophen (SUP-2-2) wird analog zu Beispiel 1 aus 4'-Ethyl-2,6-difluor-4-biphenylboronsäure und 2-Brom-5-etyhlthiophen hergestellt.
**¹H-NMR** (400 MHz, CHCl₃): δ = 7,41 (d, 2H, J = 8,1 Hz, H_{*arom*.}), 7,29 (d, 2H, J = 8,1 Hz, H_{*arom*.}), 7,19-7,13 (m, 3H, H_{*arom*.}), 6,77 (d, 1H, J = 3,4 Hz, H_{*arom*.}), 2,87 (q, 2H, J = 7,5 Hz, **-CH₂**CH₃), 2,71 (q, 2H, J = 7,5 Hz, H₃C**CH₂**-), 1,34 (t, 3H, J = 7,5 Hz, -CH₂**CH₃**), 1,28 (t, 3H, J = 7,6 Hz, -CH₂**CH₃**).
**¹⁹F-NMR** (376 MHz, CHCl₃): δ = -114,5 (d, 2F, J = 8,3 Hz).
**MS (EI):** m/z(%) = 328 (100, M⁺), 313 (89, [M - CH₃⁺).
Δε = +6,4
Δn = 0,3021
γ_{I} = 1 06 mPa·s
K 106 I

### Beispiel 8: 2-[2,6-Difluor-4-(4-propyl-cyclohexyl)-phenyl]-5-ethyl-thiophen (CUS-3-2)

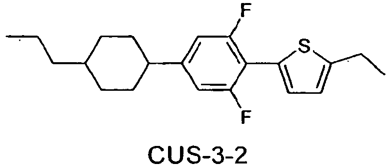

Die erfindungsgemäße Verbindung 2-[2,6-Difluor-4-(4-propyl-cyclohexyl)-phenyl]-5-ethyl-thiophen ("CUT-3-2") wird analog zu Beispiel 3 aus 2,5-Difluor-4-(4-propyl-cyclohexyl)-benzolboronsäure und 2-Brom-5-etyhlthiophen hergestellt.
**¹H-NMR** (400 MHz, CHCl₃): δ = 7,31 (d, 1H, J = 3,6 Hz, H_{*arom*.}), 6,84-6,79 (m, 3H, H_{*arom*.}), 2,88 (dq, 2H, J = 7,5 Hz, J = 0,8 Hz, -CH₂CH₃), 2,48-2,40 (m, 1H, H_{*aliphat*.}), 1,93-1,84 (m, 4H, H_{*aliphat*.}), 1,46-1,18 (m, 10H, H*_{aliphat.}*), 1,10-0,98 (m, 2H, H*_{aliphat.}*), 0,90 (t, 3H, J = 7,5 Hz, -CH₂CH₃).
**¹⁹F-NMR** (376 MHz, CHCl₃). δ = -111,5 (d, 2F, J = 10,0 Hz).
**MS (EI):** m/z(%) = 348 (100, M⁺), 333 (67, [M - CH₃⁺).
Δε = +3,0
Δn = 0,1719
γ₁ = 107 mPa·s
K 34 N 97 I

### Beispiel 9: 2-(3,5-Difluor-4'-propyl-biphenyl-4-yl)-5-vinyl-thiophen (PUS-3-V)

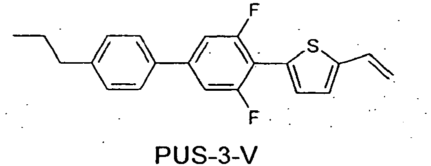

Die erfindungsgemäße Verbindung 2-(3,5-Difluor-4'-propyl-biphenyl-4-yl)-5-vinyl-thiophen (PUS-2-V) wird analog zu Beispiel 3 aus 4'-Propyl-3,5-difluor-4-biphenylboronsäure und 2-Brom-5-vinylthiophen hergestellt.
Δε = +6,5
Δn = 0,349
γₗ = 163 mPa·s
K 69 N 111 I

### Vergleichsbeispiel 1: 2-(2-Fluor-4'-pentyl-biphenyl-4-yl)-5-hexyl-thiophen

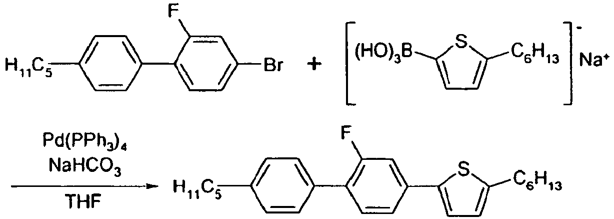

Eine Mischung aus 3,30 g (13,1 mmol) 5-Hexylthiophen-2-yl-hydroxyboronsäurenatriumsalz, 4,20 g (13,1 mmol) 4-Brom-2-fluor-4'-pentyl-biphenyl, 2,2 g (26,2 mmol) Natriumhydrogencarbonat und 0,70 g (0,61 mmol) Tetrakis-(triphenylphosphin)palladium(0) in THF/Wasser (1:1) wird 6 h zum Rückfluß erhitzt. Die Mischung wird mit MTBE verdünnt, und die organische Phase wird abgetrennt. Die wässrige Phase wird mit MTBE extrahiert. Die vereinigten organischen Phasen werden mit ges.

Natriumchloridlsg. gewaschen und mit Natriumsulfat getrocknet. Die Lösung wird vollständig konzentriert, und der Rückstand wird säulenchromatographisch (SiO₂, *n*-Heptan) gereinigt. Die weitere Reinigung erfolgt durch Umkristallisation aus Methanol/Pentan (5 : 1). 2-(2-Fluor-4'-pentyl-biphenyl-4-yl)-5-hexyl-thiophen wird als farbloser Feststoff mit einem Schmp. von 44 °C erhalten.
**¹H-NMR** (400 MHz, CHCl₃): δ = 7,47 (dd, 2H, J = 8,6 Hz, J = 1,7 Hz, H_{*arom*.}), 7,42-7,35 (m, 2H, H*ₐᵣₒₘ*.), 7,31-7,30 (d, 1H, J = 12,0 Hz, H_{*arom*.}), 7,26 (d, 2H, J = 8,4 Hz, H_{*arom*.}), 7,17 (d, 1H, J = 3,3 Hz, H_{*arom*.}), 6,76 (d, 1H, J = 3,3 Hz, H_{*arom*.}), 2,82 (t, 2H, J = 7,6 Hz, -CH₂(CH₂)₄CH₃), 2,68 (t, 2H, J = 7,8 Hz, H₃C(CH₂)₃**CH₂**-), 1,75-1,60 (m, 4H, -**(CH₂)**-), 1,44-1,27 (m, 10H, -**(CH₂)**-), 0,94-0,86 (m, 6H, **H₃C**(CH₂)₃CH₂-, -CH₂(CH₂)₄**CH₃**). **¹⁹F-NMR** (282 MHz, CHCl₃): δ = 118,0 (dd, 1 F, J = 12,0 Hz, J = 7,6 Hz).
**MS (EI):** m/z(%) = 408 (100, M⁺), 337 (77, [M - Pent]⁺), 280 (21, [M - Pent - Bu]⁺).
Δε = +3,2
Δn = 0,2311
γ_{I} = 153 mPa·s
K 44 Sm (6) SmB (38) SmC 68 SmA 75 N 94 I

### Vergleichsbeispiel 2: 2-Butyl-5-(3-fluor-4'-hexyloxy-biphenyl-4-yl)-thiophen

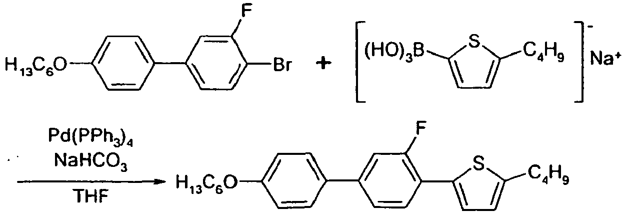

Eine Mischung aus 4,48 g (20,0 mmol) 5-Butylthiophen-2-yl-hydroxyboronsäurenatriumsalz, 7,0 g (19,9 mmol) 4-Brom-3-fluor-4'-hexyloxy-biphenyl, 3,36 g (40,0 mmol) Natriumhydrogencarbonat und 1,1 g (0,95 mmol) Tetrakis-(triphenylphosphin)palladium(0) in 90 ml THF/Wasser (2:1) wird 19 h zum Rückfluß erhitzt. Die Mischung wird mit MTBE verdünnt, und die organische Phase wird abgetrennt. Die wässrige Phase wird mit MTBE extrahiert. Die vereinigten organischen Phasen werden mit ges. Natriumchloridlsg. gewaschen und mit Natriumsulfat getrocknet. Die Lösung wird vollständig konzentriert, und der Rückstand wird säulenchromatographisch (SiO₂, n-Heptan : Toluol = 9 : 1) gereinigt. Die weitere Reinigung erfolgt durch Umkristallisation aus Ethanol. 2-Butyl-5-(3-fluor-4'-hexyloxy-biphenyl-4-yl)-thiophen wird als farbloses Wachs mit einem Schmp. von 66 °C erhalten.
**¹H-NMR** (300 MHz, CHCl₃): δ = 7,62-7,57 (m, 1H, H_{*arom*.}), 7,52 (d, 2H, J = 8,2 Hz, H_{*arom*.}), 7,35-7,28 (m, 3H, H*_{arom.}*), 7,96 (d, 2H, J = 8,2 Hz, H_{*arom*.}), 6,80-6,78 (m, 1H, H_{*arom*.}), 3,99 (t, 2H, J = 6,1 Hz, O**CH₂**(CH₂)₄CH₃), 2,85 (t, 2H, J = 7,2 Hz, **CH₂**(CH₂)₂CH₃), 1,85-1,65 (m, 4H, H_{*aliph*.}), 1,54-1,31 (8H, H_{*aliph*.}), 0,99-0,89 (m, 6H, 2 × CH₃).
**¹⁹F-NMR** (282 MHz, CHCl₃): δ = 114,2 (dd, 1F, J = 12,7 Hz, J = 8,2 Hz).
**MS (EI):** m/z(%) = 410 (100, M⁺), 367 (31, [M - Propyl]⁺), 283 (51, [M - Propyl - Hexyl]⁺).
Δε = +8,9
Δn = 0,2749
γ_{I} = 262 mPa·s
K 66 SmE 89 SmC 131 SmA 144 N 146 I

### Vergleichsbeispiel 3: 2-(3-Fluor-4-pentyl-phenyl)-5-p-tolyl-thiophen Synthese von 2-(3-Fluor-4-pentyl-phenyl)-thiophen

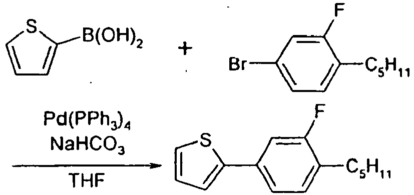

Eine Mischung aus 40,0 g (0,31 mol) Thiophen-2-yl-boronsäure, 80,0 g (0,32 mol) 4-Brom-2-fluor-1-pentyl-benzol, 18,0 g (15,6 mmol) Tetrakis-(triphenylphosphin)palladium(0) und 400 ml 2M Natriuhydrogencarbonatlsg. in 1000 ml Ethanol/Toluol =3 : 2 wird 19 h auf 80 °C erhitzt. Die Mischung wird mit Toluol und Wasser versetzt, und die organische Phase wird abgetrennt. Die wässrige Phase wird mit Toluol extrahiert und die vereinigten organischen Phasen werden mit ges. Natriumchloridlsg. gewaschen. Die Lösung wird mit Natriumsulfat getrocknet und vollständig konzentriert. Der Rückstand wird säulenchromatographisch (SiO₂, n-Heptan) gereinigt. 2-(3-Fluoro-4-pentyl-phenyl)-thiophen wird als farblose Flüssigkeit erhalten.

### Synthese von 2-Brom-5-(3-fluor-4-pentyl-phenyl)-thiophen

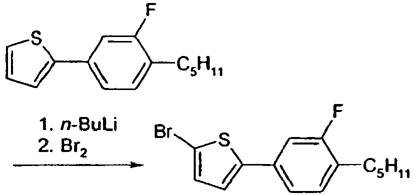

20,0 g (80,5 mmol) 2-(3-Fluoro-4-pentyl-phenyl)-thiophen werden in 200 ml THF vorgelegt, und die Lösung wird im Temperaturbereich von 0-5 °C mit 50,0 ml *n*-BuLi (79,6 mmol, 15% Lsg. in Hexan) tropfenweise versetzt. Nach 1 h bei 0 °C wird der Ansatz auf -70 °C abgekühlt, und 4,1 ml (80,0 mmol) Brom werden zudosiert. Nach beendeter Zugabe wird 1 h bei 0 °C gerührt. Der Ansatz wird mit MTBE verdünnt und nacheinander mit 2N HCl, 10% Natriuhydrogensulfitösg., ges. Natriumhydrogencarbonatlsg. und ges. Natriumchloridlsg. gewaschen. Die Lösung wird mit Natriumsulfat getrocknet und vollständig konzentriert. Das Rohprodukt wird säulenchromatographisch (SiO₂, *n*-Heptan) gereinigt. 2-Brom-5-(3-fluor-4-pentyl-phenyl)-thiophen wird als gelbliche Flüssigkeit erhalten.

### Synthese von 2-(3-Fluor-4-pentyl-phenyl)-5-p-tolyl-thiophen

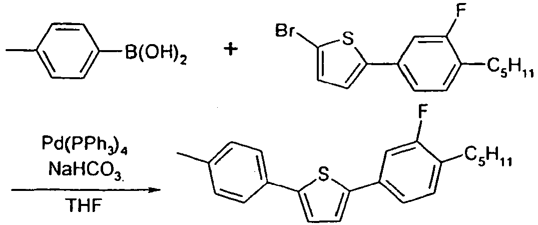

Eine Mischung aus 3,54 g (26,0 mmol) p-Tolylboronsäure, 8,50 g (26,0 mmol) 2-Brom-5-(3-fluor-4-pentyl-phenyl)-thiophen, 1,50 g (1,30 mmol) Tetrakis-(triphenylphosphin)palladium(0) und 33 ml 2M Natriuhydrogencarbonatlsg. in 80 ml Ethanol/Toluol = 3 : 2 wird 20 h auf 80 °C erhitzt. Die Mischung wird mit Toluol und Wasser versetzt, und die organische Phase wird abgetrennt. Die wässrige Phase wird mit Toluol extrahiert und die vereinigten organischen Phasen werden mit ges. Natriumchloridlsg. gewaschen. Die Lösung wird mit Natriumsulfat getrocknet und vollständig konzentriert. Der Rückstand wird säulenchromatographisch (SiO₂, Toluol) gereinigt. Die weitere Reinigung erfolgt durch Umkristallisation aus Ethanol und *n*-Heptan. 2-(3-Fluor-4-pentyl-phenyl)-5-*p*-tolyl-thiophen wird als Feststoff mit einem Schmp. von 115 °C erhalten.
**¹H-NMR** (300 MHz, CHCl₃): δ = 7,53-7,49 (m, 2H, H_{*arom*.}), 7,32-7,37 (m, 2H, H_{*arom*.}), 7,24-7,13 (m, 5H, H_{*arom*.}), 2,63 (t, 2H, J = 7,7 Hz, - CH₂(CH₂)₃CH₃), 2,36 (s, 3H, Me), 1,67-1,56 (m, 2H, H*ₐₗᵢₚₕ*.), 1,39-1,29 (m, 4H, H_{*aliph*.}), 0,90 (t, 3H, J = 6,9 Hz, -CH₂(CH₂)₃**CH₃**).
**¹⁹F-NMR** (282 MHz, CHCl₃): δ = 118,6 (dd, 1F, J = 11,0 Hz, J = 8,2 Hz).
**MS (EI):** m/z(%) = 338 (92, M⁺), 281 (100, [M - Butyl]⁺).
Δε = +3,7
Δn = 0,1877
γ_{I} = 196 mPa·s
K 115 N 119 I

### Mischungsbeispiele

### Vergleichsbeispiel M1

Eine nematische FK-Mischung wird wie folgt formuliert

| | | | |
|---|---|---|---|
| PUQU-3-F | 5,0 % | Kp. | + 75,5 °C |
| PGUQU-3-F | 7,0 % | Δn | 0,1036 |
| APUQU-2-F | 8,0 % | Δε | +7,4 |
| APUQU-3-F | 8,0 % | ε_{∥} | 10,6 |
| CC-3-V | 47,0 % | K₃/K₁ | 1,10 |
| CC-3-V1 | 6,0 % | γ_{I} | 57 mPa·s |
| CCP-V-1 | 10,0 % | V₀[V] | 1,36 |
| PGP-2-3 | 4,0 % | | |
| PGP-2-4 | 5,0 % | | |

### Mischungsbeispiel 1

Eine erfindungsgemäße nematische FK-Mischung wird wie folgt formuliert

| | | | |
|---|---|---|---|
| PUQU-3-F | 5,0 % | Kp. | + 74,5 °C |
| PGUQU-3-F | 7,0 % | Δn | 0,1026 |
| APUQU-2-F | 8,0 % | Δε | +7,6 |
| APUQU-3-F | 8,0 % | ε_{∥} | 10,7 |
| CC-3-V | 47,0 % | K₃/K₁ | 1,07 |
| CC-3-V1 | 7,0 % | γ_{I} | 55 mPa·s |
| CCP-V-1 | 11,0 % | V₀[V] | 1,36 |
| PUS-2-2 | 7,0 % | | |

Die Mischung weist gegenüber Vergleichsmischung M1 eine geringere Rotationsviskosität auf, bei nahezu unveränderten Werten des Klärpunkts, der dielektrischen Anisotropie, der Doppelbrechung und der Schwellenspannung.

### Mischungsbeispiel 2

Eine erfindungsgemäße nematische FK-Mischung wird wie folgt formuliert

| | | | |
|---|---|---|---|
| PUQU-3-F | 6,0 % | Kp. [°C] | + 74,0 |
| PGUQU-3-F | 6,0 % | Δn | 0,1001 |
| APUQU-2-F | 8,0 % | Δε | +7,3 |
| APUQU-3-F | 8,0 % | ε_{∥} | 10,5 |
| CC-3-V | 48,0 % | K₃/K₁ | 1,08 |
| CC-3-V1 | 6,0 % | γ_{I} [mPa·s] | 53 |
| CCP-V-1 | 12,0 % | V₀[V] | 1,36 |
| PUS-2-2 | 6,0 % | | |

Die Mischung weist gegenüber Vergleichsmischung M1 eine geringere Rotationsviskosität auf, bei nahezu unveränderten Werten des Klärpunkts, der dielektrischen Anisotropie, der Doppelbrechung und der Schwellenspannung.

### Vergleichsbeispiel M2

Eine nematische FK-Mischung wird wie folgt formuliert

| | | | |
|---|---|---|---|
| PUQU-3-F | 9,0 % | Kp. [°C] | + 75,5 |
| CPGU-3-OT | 9,0 % | Δn | 0,1351 |
| PUQU-2-F | 14,0 % | Δε | +8,0 |
| CC-3-V | 41,5 % | ε_{∥} | 11,3 |
| CCP-V-1 | 4,5 % | γ_{I} [mPa·s ] | 63 |
| PP-1-2V1 | 5,0 % | V₀[V] | 1,53 |
| PGP-2-2V | 17,0 % | | |

### Mischungsbeispiel 3

Eine erfindungsgemäße nematische FK-Mischung wird wie folgt formuliert

| | | | |
|---|---|---|---|
| PUQU-3-F | 9,0 % | Kp. [°C] | + 74,0 |
| CPGU-3-OT | 9,0 % | Δn | 0,132 |
| PUQU-2-F | 14,0 % | Δε | +7,9 |
| CC-3-V | 43,5 % | ε_{∥} | 11,2 |
| CCP-V-1 | 4,5 % | γ_{I} [mPa·s] | 61 |
| PP-1-2V1 | 3,0 % | V₀[V] 1,50 | |
| PGP-2-2V | 11,0 % | | |
| PUS-3-2 | 6,0% | | |

Die Mischung weist gegenüber Vergleichsmischung M2 eine geringere Rotationsviskosität und Schwellenspannung auf, bei nahezu unveränderten Werten des Klärpunkts, der dielektrischen Anisotropie und der Doppelbrechung.

### Mischungsbeispiel 4

Eine erfindungsgemäße nematische FK-Mischung wird wie folgt formuliert

| | | | |
|---|---|---|---|
| PUQU-3-F | 8,0 % | Kp. [°C] | + 74,0 |
| CPGU-3-OT | 5,5 % | Δn | 0,132 |
| PUQU-2-F | 15,0 % | Δε | +7,2 |
| CC-3-V | 44,0 % | ε_{∥} | 10,4 |
| CCP-V-1 | 7,0 % | γ_{I} [mPa·s | ] 58 |
| PGP-2-2V | 3,0 % | V₀[V] | 1,56 |
| CPGP-4-3 | 1,5 % | | |
| PUS-3-2 | 16,0 % | | |

Die Mischung weist gegenüber Vergleichsmischung M2 eine geringere Rotationsviskosität auf, bei nahezu unveränderten Werten des Klärpunkts, der dielektrischen Anisotropie, der Doppelbrechung und der Schwellenspannung.

Weitere Kombinationen der Ausführungsformen und Varianten der Erfindung ergeben sich aus den folgenden Ansprüchen.

## Patentansprüche

1. Verbindungen der Formel I 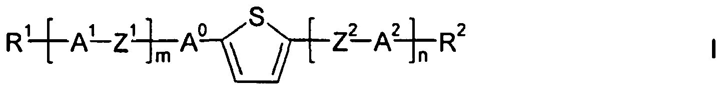 worin die einzelnen Reste folgende Bedeutung besitzen
R¹ und R² H, F, Cl, Br, -CN, -SCN, -NCS, SF₅ oder geradkettiges oder verzweigtes Alkyl mit 1 bis 12 C-Atomen, worin auch eine oder mehrere nicht benachbarte CH₂-Gruppen jeweils unabhängig voneinander durch -CH=CH-, -C≡C-, -O-, -CO-, -CO-O-, -O-CO-, -O-CO-O- so ersetzt sein können, dass O-Atome nicht direkt miteinander verknüpft sind, und worin auch ein oder mehrere H-Atome durch F, Cl oder Br ersetzt sein können, oder P-Sp-,
P eine polymerisierbare Gruppe,
Sp eine Abstandsgruppe oder eine Einfachbindung,
A⁰ 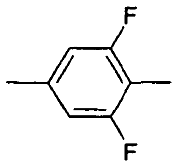
A¹ und A² jeweils unabhängig voneinander Phenylen-1,4-diyl, worin auch eine oder zwei CH-Gruppen durch N ersetzt sein können und ein oder mehrere H-Atome durch Halogen, CN, CH₃, CHF₂, CH₂F, OCH₃, OCHF₂ oder OCF₃ ersetzt sein können, Cyclohexan-1,4-diyl, worin auch eine oder zwei nicht benachbarte CH₂ -Gruppen unabhängig voneinander durch O und/oder S ersetzt sein können, und ein oder mehrere H-Atome durch F ersetzt sein können, Cyclohexen-1,4-diyl, Bicyclo[1.1.1]pentan-1,3-diyl, Bicyclo[2.2.2]octan-1,4-diyl, Spiro[3.3]heptan-2,6-diyl, Tetrahydropyran-2,5-diyl, oder 1,3-Dioxan-2,5-diyl,
Z¹ und Z² jeweils unabhängig voneinander -CF₂O-, -OCF₂-, -CH₂O-, -OCH₂-, -CO-O-, -O-CO-, -C₂H₄- -C₂F₄₋, - CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- oder eine Einfachbindung,
m und n jeweils unabhängig voneinander 0, 1, 2 oder 3.

2. Verbindungen nach Anspruch 1, **dadurch gekennzeichnet, dass** sie ausgewählt aus der Gruppe bestehend aus folgenden Formeln sind: 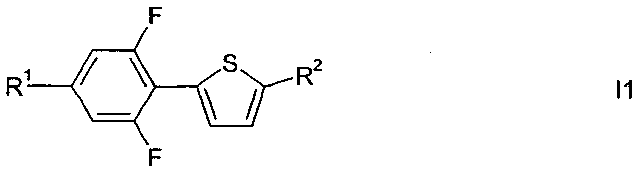 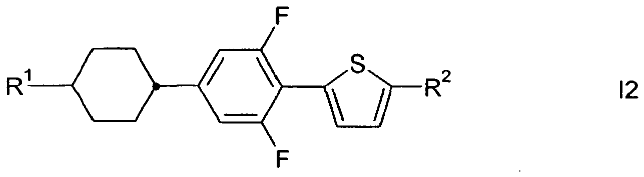 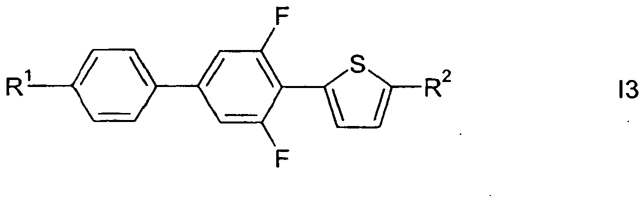 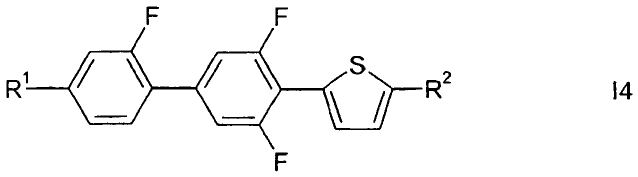 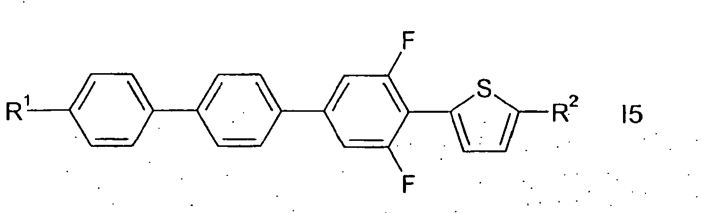 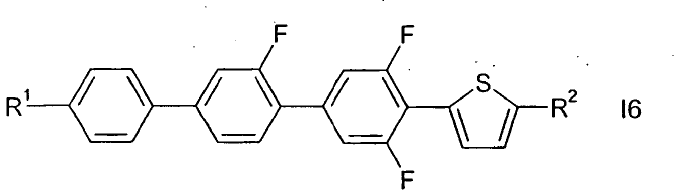 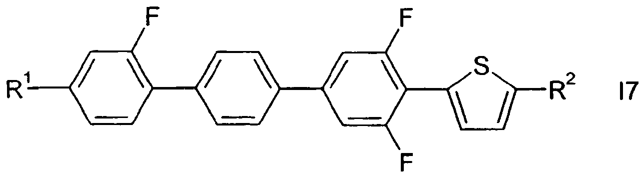 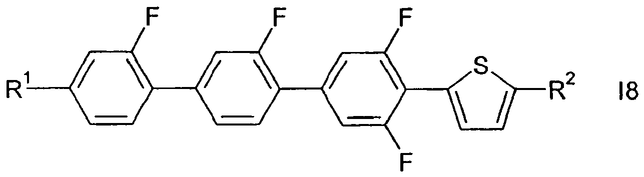 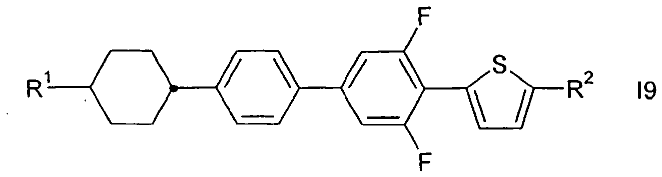 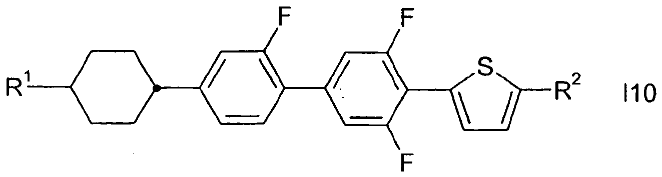 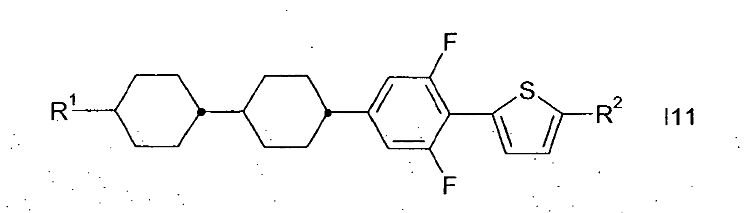 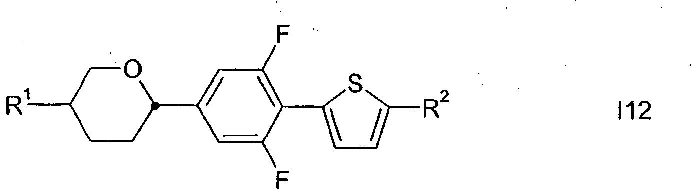 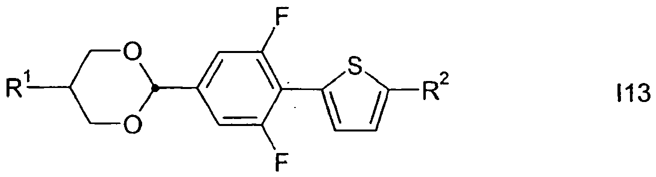 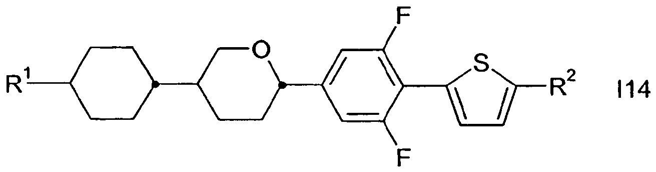 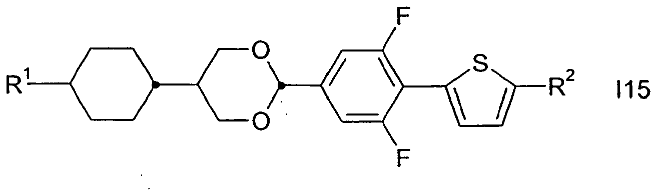 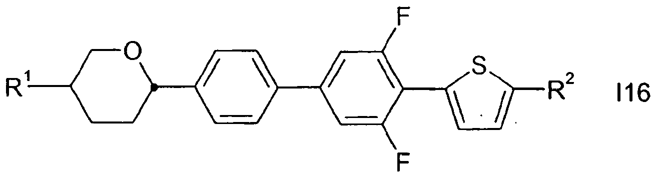 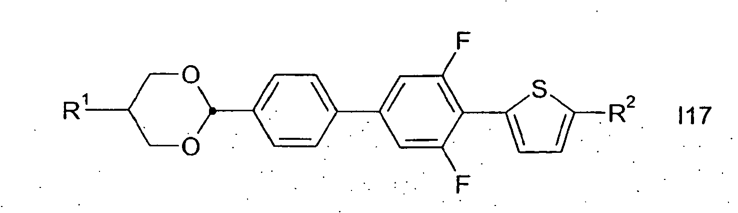 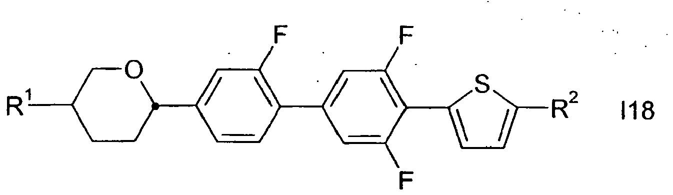 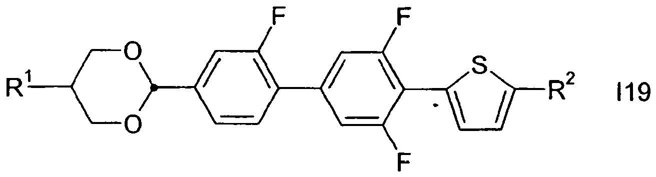 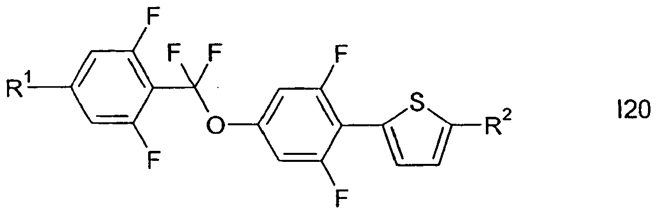 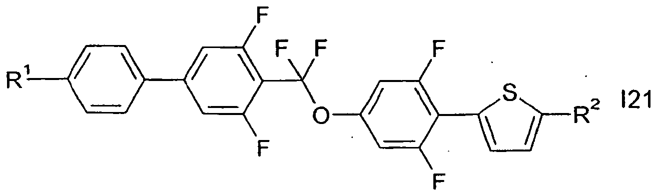 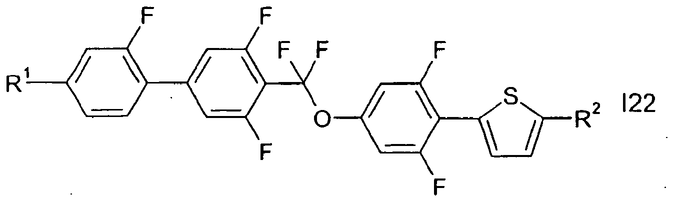 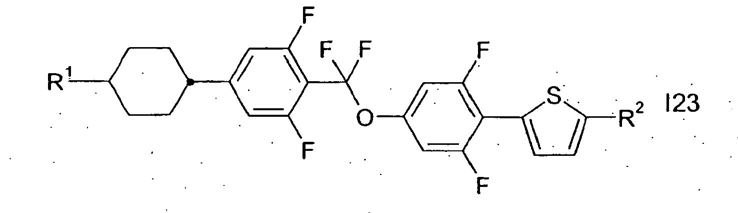 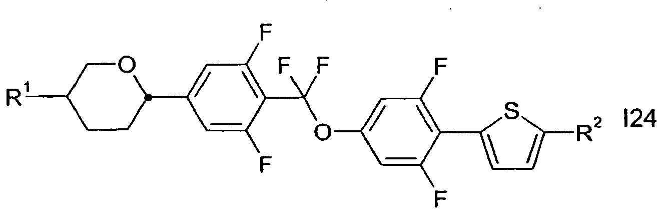 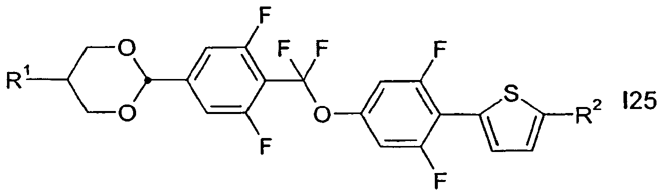 worin R¹ und R² die in Anspruch 1 angegebenen Bedeutungen besitzen.

3. Verbindungen nach Anspruch 1 oder 2, **dadurch gekennzeichnet, dass** in Formel I n 0, m 0 oder 1, und Z¹ eine Einfachbindung bedeuten.

4. FK-Medium, welches bei Raumtemperatur eine nematische Phase aufweist, enthaltend eine oder mehrere Verbindungen nach einem oder mehreren der Ansprüche 1 bis 3.

5. FK-Medium nach Anspruch 4, **dadurch gekennzeichnet, dass** es zusätzliche eine oder mehrere Verbindungen der Formel II und/oder III enthält: 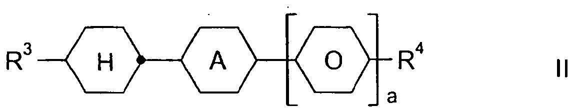 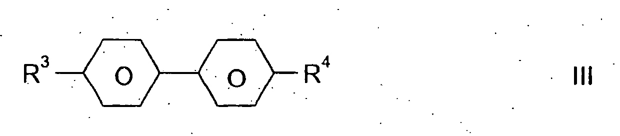 worin
A 1,4-Phenylen oder trans-1,4-Cyclohexylen bedeutet,
a 0 oder 1 ist,
R³ Alkenyl mit 2 bis 9 C-Atomen bedeutet, und
R⁴ Alkyl mit 1 bis 12 C-Atomen, wobei auch eine oder zwei nicht benachbarte CH₂-Gruppen durch -O-, -CH=CH-, -CO-, -OCO- oder -COO- so ersetzt sein können, dass O-Atome nicht direkt miteinander verknüpft sind, und vorzugsweise Alkyl mit 1 bis 12 C-Atomen oder Alkenyl mit 2 bis 9 C-Atomen bedeutet.

6. FK-Medium nach Anspruch 4 oder 5, **dadurch gekennzeichnet, dass** es zusätzlich eine oder mehrere Verbindungen ausgewählt aus der Gruppe bestehend aus folgenden Formeln enthält 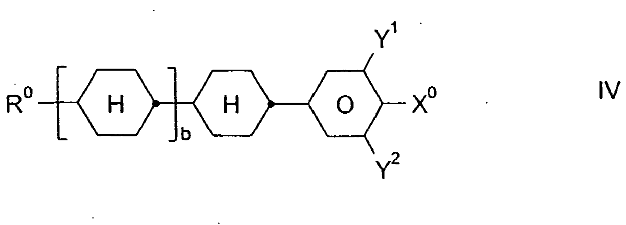 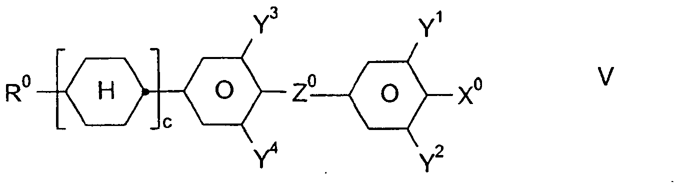 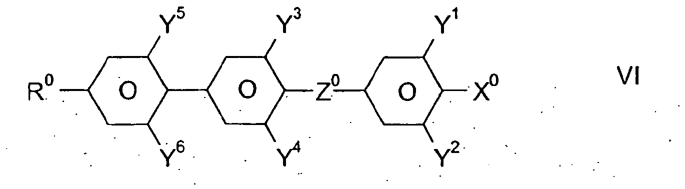 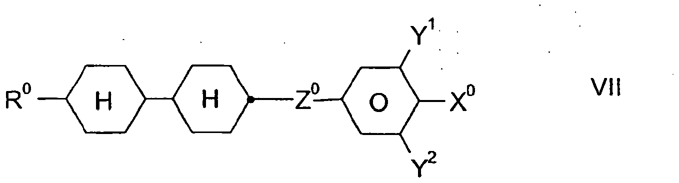 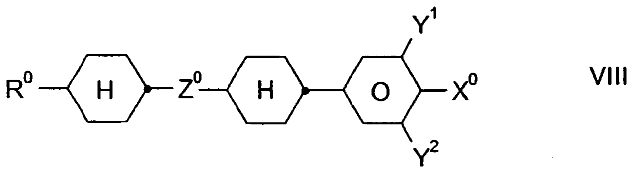 worin
R⁰ einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH- , 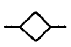 , 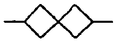 , -CO-O- oder -O-CO- so ersetzt sein können, dass O-Atome nicht direkt miteinander verknüpft sind, und worin auch ein oder mehrere H-Atome durch Halogen ersetzt sein können,
X⁰ F, Cl, CN, SF₅, SCN, NCS, einen halogenierten Alkylrest, halogenierten Alkenylrest, halogenierten Alkoxyrest oder halogenierten Alkenyloxyrest mit jeweils bis zu 6 C-Atomen,
Y¹⁻⁶ jeweils unabhängig voneinander H oder F,
Z⁰ -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- oder -OCF₂-, in den Formeln V und VI auch eine Einfachbindung, und
b und c jeweils unabhängig voneinander 0 oder 1
bedeuten.

7. FK-Medium nach einem oder mehreren der Ansprüche 4 bis 6, **dadurch gekennzeichnet, dass** es zusätzlich eine oder mehrere Verbindungen ausgewählt aus der Gruppe bestehend aus folgenden Formeln enthält 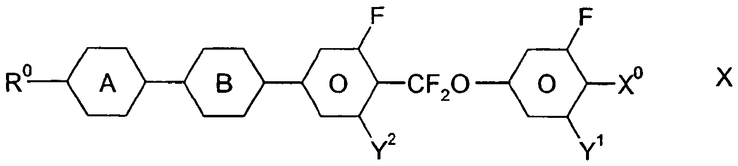 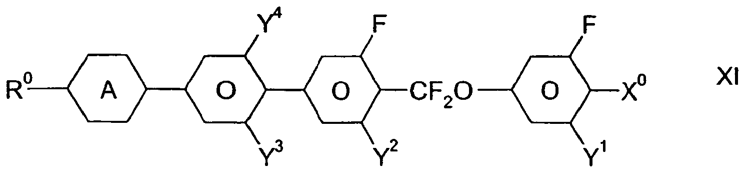 worin R⁰, X⁰ und Y¹⁻⁴ die in Anspruch 4 angegebenen Bedeutungen besitzen, und 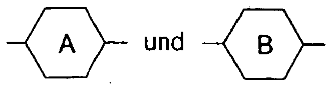 jeweils unabhängig voneinander 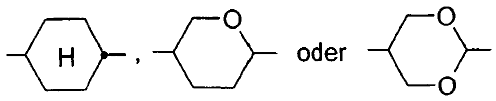 bedeuten.

8. FK-Medium nach einem oder mehreren der Ansprüche 4 bis 7, **dadurch gekennzeichnet, dass** es zusätzlich eine oder mehrere Verbindungen der folgenden Formel enthält: 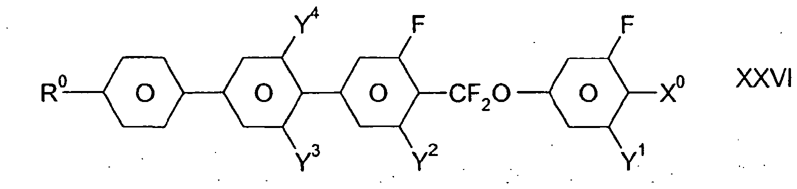 worin R⁰, X⁰ und Y¹⁻⁴ die in Anspruch 7 angegebenen Bedeutungen besitzen.

9. Verfahren zur Herstellung von Verbindungen der Formel I gemäß einem oder mehreren der Ansprüche 1 bis 3, **dadurch gekennzeichnet, dass** man in einer Palladium-vermittelten Suzuki-Kreuzkupplung ein substituiertes Bromthiophen **2** 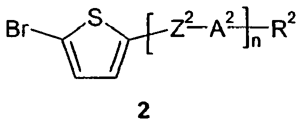 mit einer Boronsäure **1** 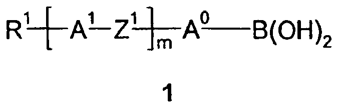 oder einem entsprechenden Boronsäureester umsetzt,
oder eine Verbindung vom Typ **10** 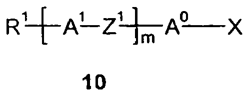 mit einer Thiophenboronsäure oder einem entsprechenden Thiophen-hydroxyboronsäuresalz **11** 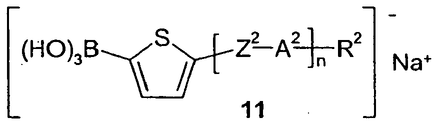 umsetzt, wobei R^{1,2}, A^{0,1,2}, Z^{1,2}, m und n die in Anspruch 7 angegebene Bedeutung besitzen und X Br oder I bedeutet.

10. FK-Anzeige enthaltend ein FK-Medium oder eine oder mehrere Verbindungen nach einem oder mehreren der Ansprüche 1 bis 8.

11. FK-Anzeige nach Anspruch 10, **dadurch gekennzeichnet, dass** sie eine MFK-, TN-, STN- oder IPS-Anzeige ist.

## Claims

1. Compounds of the formula I 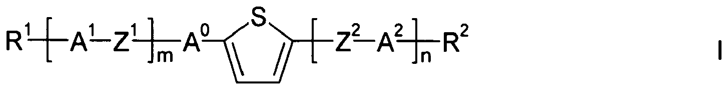 in which the individual radicals have the following meaning
R¹ and R² denote H, F, Cl, Br, -CN, -SCN, -NCS, SF₅ or straight-chain or branched alkyl having 1 to 12 C atoms, in which, in addition, one or more non-adjacent CH₂ groups may each be replaced, independently of one another, by -CH=CH-, -C≡C-, -O-, -CO-, -CO-O-, -O-CO-, -O-CO-O- in such a way that O atoms are not linked directly to one another, and in which, in addition, one or more H atoms may be replaced by F, Cl or Br, or P-Sp-,
P denotes a polymerisable group,
Sp denotes a spacer group or a single bond,
A⁰ denotes 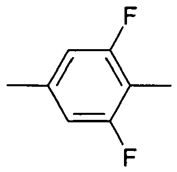
A¹ and A² each, independently of one another, denote phenylene-1,4-diyl, in which, in addition, one or two CH groups may be replaced by N and one or more H atoms may be replaced by halogen, CN, CH₃, CHF₂, CH₂F, OCH₃, OCHF₂ or OCF₃, cyclohexane-1,4-diyl, in which, in addition, one or two non-adjacent CH₂ groups may be replaced, independently of one another, by O and/or S, and one or more H atoms may be replaced by F, cyclohexene-1,4-diyl, bicyclo[1.1.1]pentane-1,3-diyl, bicyclo[2.2.2]octane-1,4-diyl, spiro[3.3]heptane-2,6-diyl, tetrahydropyran-2,5-diyl or 1,3-dioxane-2,5-diyl,
Z¹ and Z² each, independently of one another, denote -CF₂O-, -OCF₂-, -CH₂O-, -OCH₂-, -CO-O-, -O-CO-, -C₂H₄-, -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- or a single bond,
m and n each, independently of one another, denote 0, 1, 2 or 3.

2. Compounds according to Claim 1, **characterised in that** they are selected from the group consisting of the following formulae: 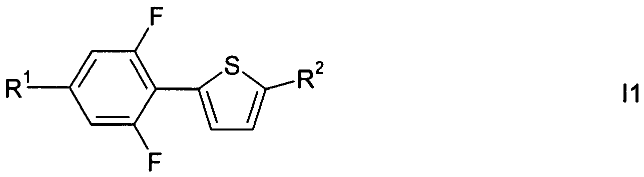 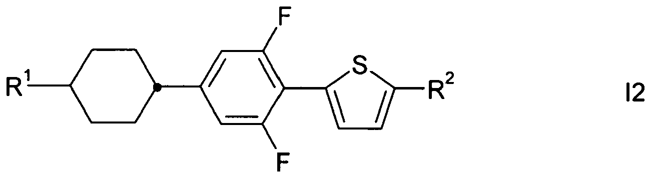 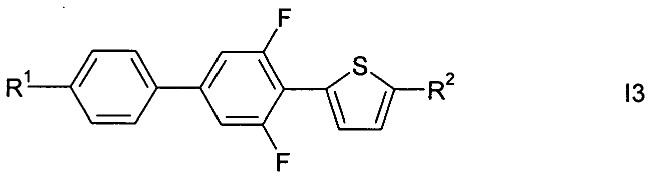 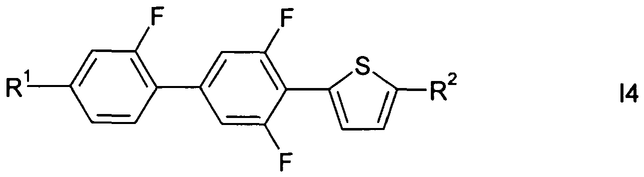 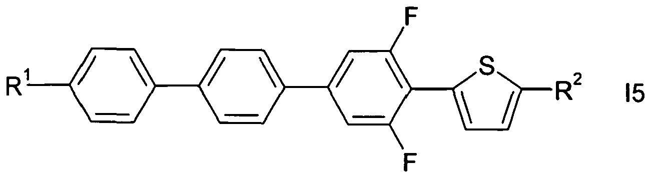 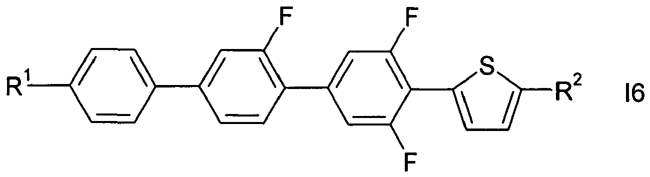 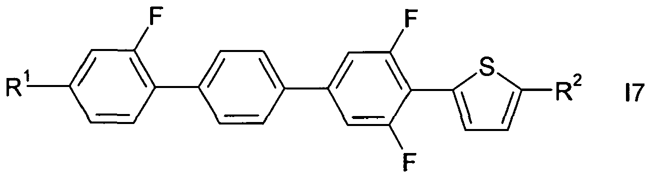 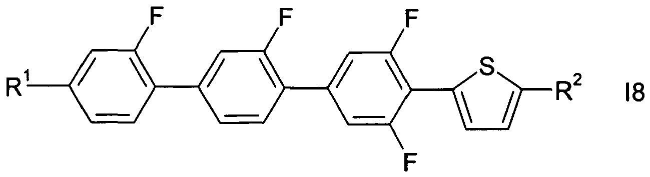 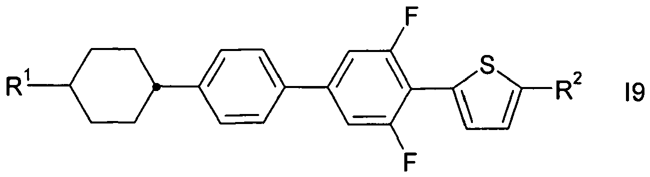 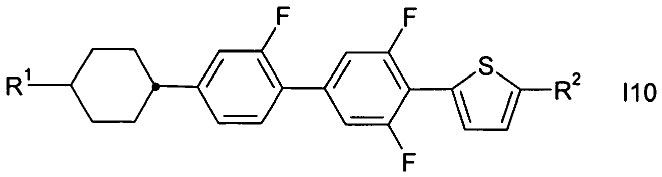 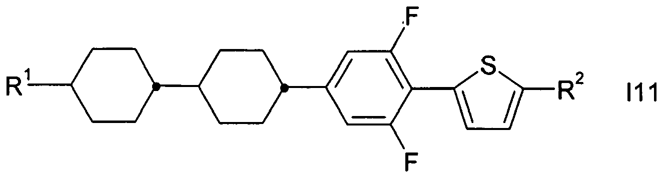 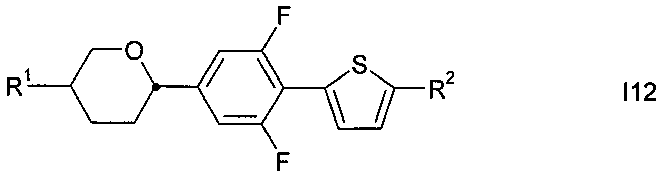 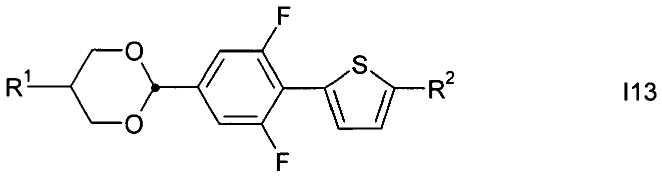 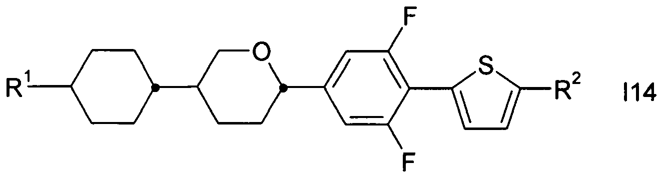 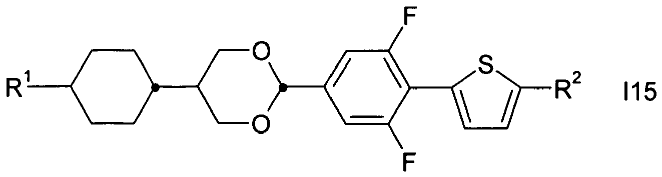 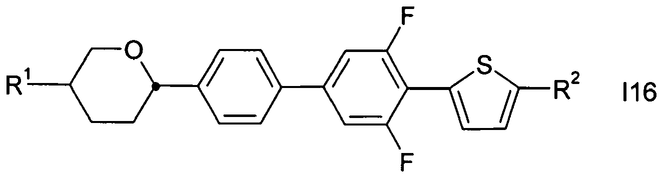 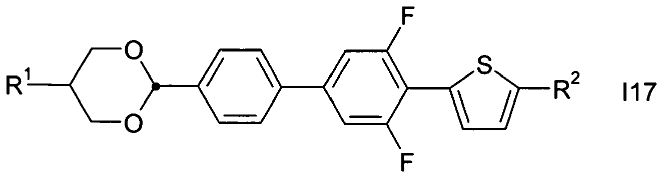 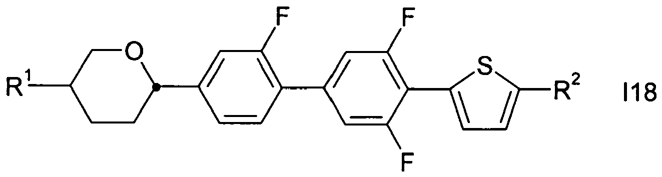 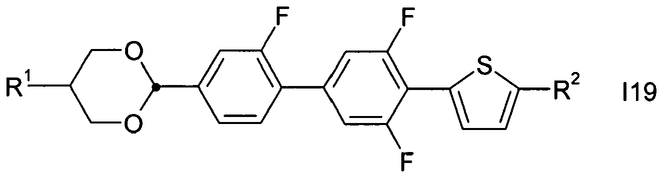 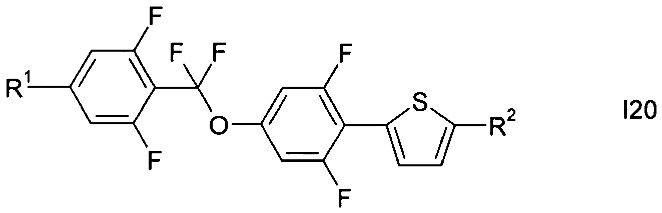 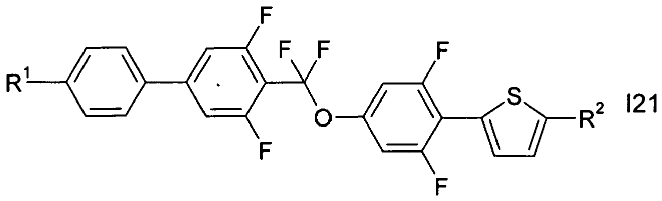 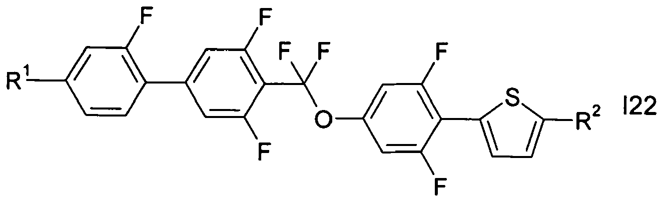 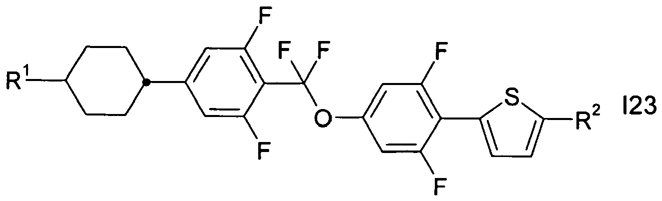 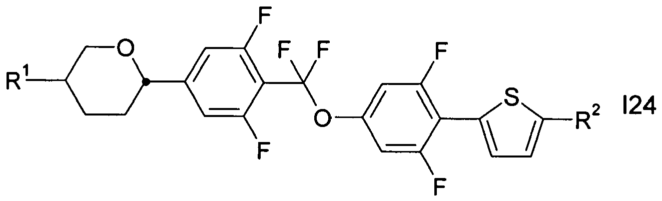 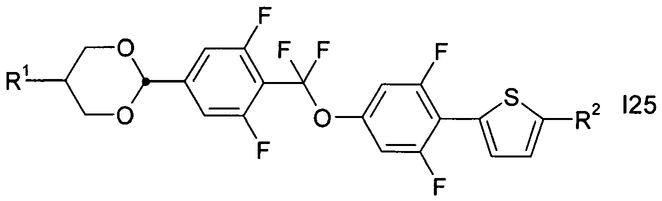 in which R¹ and R² have the meanings indicated in Claim 1.

3. Compounds according to Claim 1 or 2, **characterised in that**, in formula I, n denotes 0, m denotes 0 or 1, and Z¹ denotes a single bond.

4. LC medium which has a nematic phase at room temperature, comprising one or more compounds according to one or more of Claims 1 to 3.

5. LC medium according to Claim 4, **characterised in that** it additionally comprises one or more compounds of the formula II and/or III: 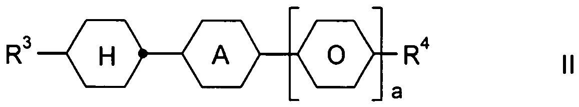 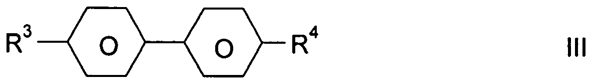 in which
A denotes 1,4-phenylene or trans-1,4-cyclohexylene,
a is 0 or 1,
R³ denotes alkenyl having 2 to 9 C atoms, and
R⁴ denotes alkyl having 1 to 12 C atoms, where, in addition, one or two non-adjacent CH₂ groups may be replaced by -O-, -CH=CH-, -CO-, -OCO- or -COO- in such a way that O atoms are not linked directly to one another, and preferably denotes alkyl having 1 to 12 C atoms or alkenyl having 2 to 9 C atoms.

6. LC medium according to Claim 4 or 5, **characterised in that** it additionally comprises one or more compounds selected from the group consisting of the following formulae: 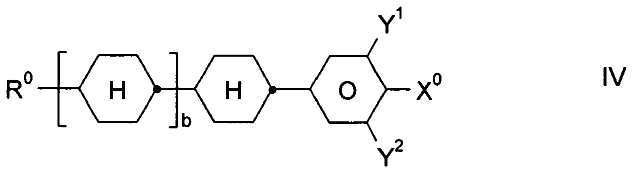 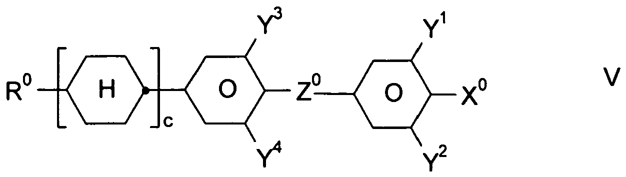 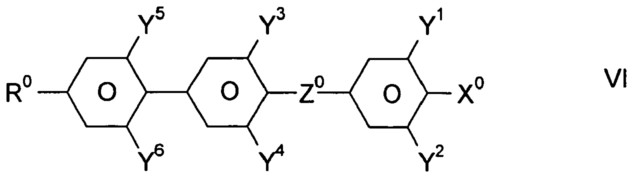 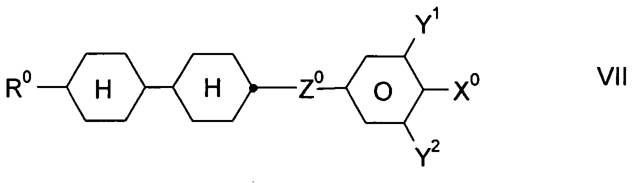 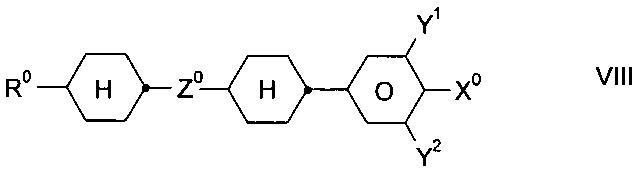 in which
R⁰ denotes an alkyl or alkoxy radical having 1 to 15 C atoms, where, in addition, one or more CH₂ groups in these radicals may each be replaced, independently of one another, by -C≡C-, -CF₂O-, -CH=CH-, 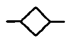 , 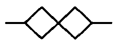 , -O-, -CO-O- or -O-CO- in such a way that O atoms are not linked directly to one another, and in which, in addition, one or more H atoms may be replaced by halogen,
X⁰ denotes F, Cl, CN, SF₅, SCN, NCS, a halogenated alkyl radical, halogenated alkenyl radical, halogenated alkoxy radical or halogenated alkenyloxy radical, each having up to 6 C atoms,
Y¹⁻⁶ each, independently of one another, denote H or F,
Z⁰ denotes -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- or -OCF₂-, in the formulae V and VI also a single bond, and
b and c each, independently of one another, denote 0 or 1.

7. LC medium according to one or more of Claims 4 to 6, **characterised in that** it additionally comprises one or more compounds selected from the group consisting of the following formulae: 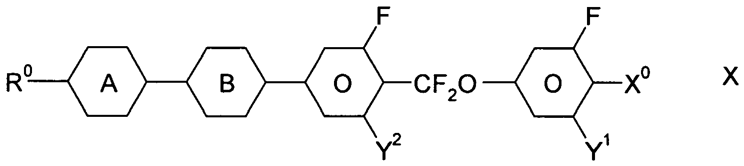 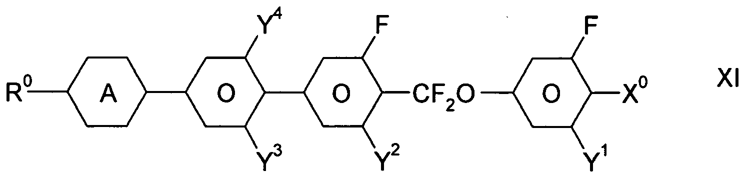 in which R⁰, X⁰ and Y¹⁻⁴ have the meanings indicated in Claim 4, and 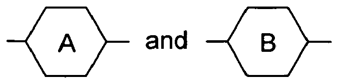 each, independently of one another, denote

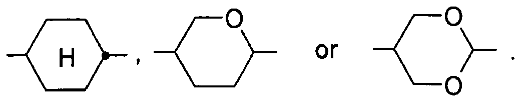

8. LC medium according to one or more of Claims 4 to 7, **characterised in that** it additionally comprises one or more compounds of the following formula: 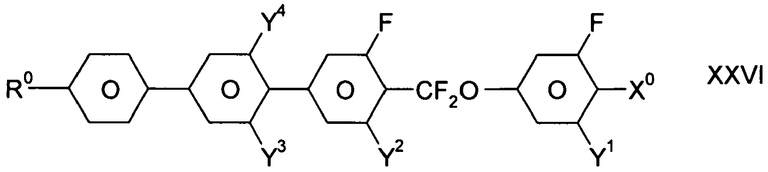 in which R⁰, X⁰ and Y¹⁻⁴ have the meanings indicated in Claim 7.

9. Process for the preparation of compounds of the formula I according to one or more of Claims 1 to 3, **characterised in that**, in a palladium-promoted Suzuki cross-coupling, a substituted bromothiophene **2** 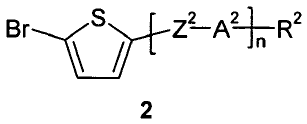 is reacted with a boronic acid **1** 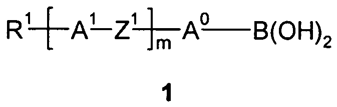 or a corresponding boronic acid ester, or a compound of type **10** 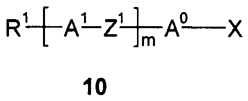 is reacted with a thiopheneboronic acid or a corresponding thiophenehydroxyboronic acid salt **11** 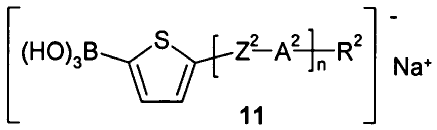 where R^{1,2}, A^{0,1,2}, Z^{1,2}, m and n have the meaning indicated in Claim 7, and X denotes Br or I.

10. LC display containing an LC medium or one or more compounds according to one or more of Claims 1 to 8.

11. LC display according to Claim 10, **characterised in that** it is an MLC, TN, STN or IPS display.

## Revendications

1. Composés de la formule I 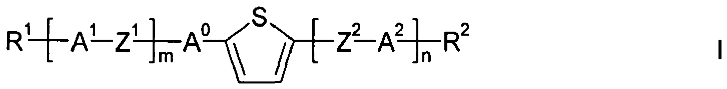 dans laquelle les radicaux individuels présentent la signification qui suit :
R¹ et R² représentent H, F, Cl, Br, -CN, -SCN, -NCS, SF₅ ou alkyle en chaîne droite ou ramifié comportant 1 à 12 atomes de C, où, en outre, un ou plusieurs groupes CH₂ non adjacents peuvent chacun être remplacés, indépendamment les un des autres, par -CH=CH-, -C≡C-, -O-, -CO-, -CO-O-, -O-CO-, -O-CO-O- de telle sorte que des atomes de O ne soient pas liés directement les uns aux autres, et où, en outre, un ou plusieurs atomes de H peuvent être remplacés par F, Cl ou Br, ou P-Sp-,
P représente un groupe polymérisable,
Sp représente un groupe d'espaceur ou une liaison simple,
A⁰ représente 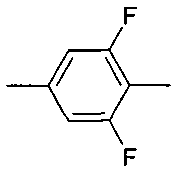
A¹ et A² représentent, chacun indépendamment de l'autre, phénylène-1,4-diyle, où, en outre, un ou deux groupes CH peuvent être remplacés par N et un ou plusieurs atomes de H peuvent être remplacés par halogène, CN, CH₃, CHF₂, CH₂F, OCH₃, OCHF₂ ou OCF₃, cyclohexane-1,4-diyle, où, en outre, un ou deux groupes CH₂ non adjacents peuvent être remplacés, indépendamment l'un de l'autre, par des atomes de O et/ou de S, et un ou plusieurs atomes de H peuvent être remplacés par F, cyclohexène-1,4-diyle, bicyclo[1.1.1]-pentane-1,3-diyle, bicyclo[2.2.2]octane-1,4-diyle, spiro-[3.3]heptane-2,6-diyle, tétrahydropyran-2,5-diyle ou 1,3-dioxane-2,5-diyle,
Z¹ et Z² représentent, chacun indépendamment de l'autre, -CF₂O-, -OCF₂-, -CH₂O-, -OCH₂-, -CO-O-, -O-CO-, -C₂H₄-, -C₂F₄-, -CF₂CH₂-, -CH₂CF₂-, -CFHCFH-, -CFHCH₂-, -CH₂CFH-, -CF₂CFH-, -CFHCF₂-, -CH=CH-, -CF=CH-, -CH=CF-, -CF=CF-, -C≡C- ou une liaison simple,
m et n représentent, chacun indépendamment de l'autre, 0, 1, 2 ou 3.

2. Composés selon la revendication 1, **caractérisés en ce qu'**ils sont choisis parmi le groupe constitué par les formules qui suivent : 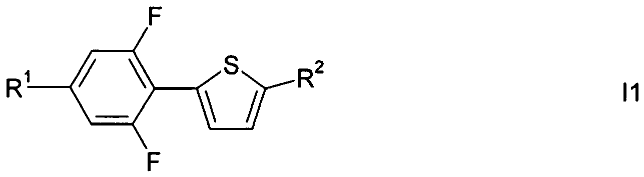 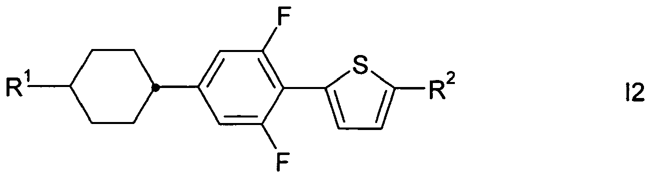 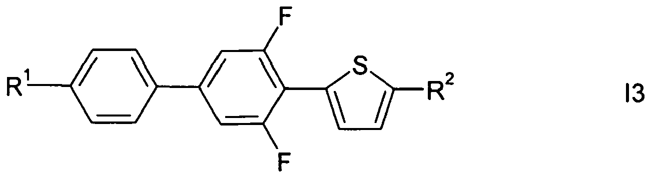 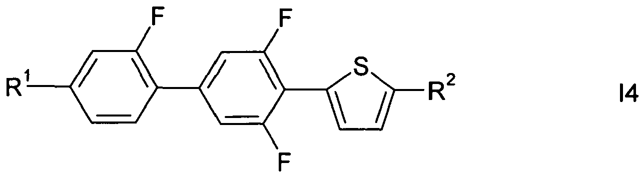 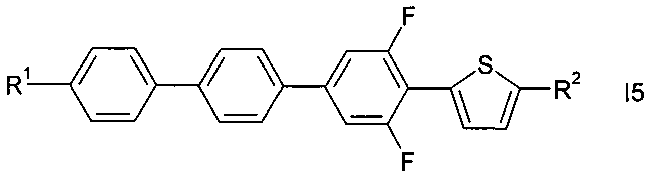 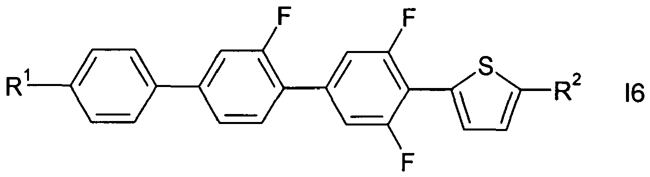 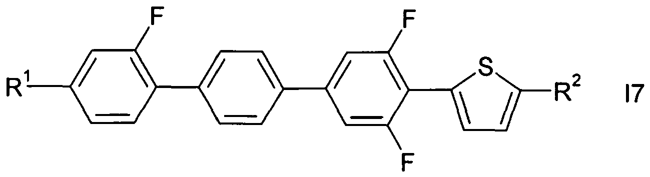 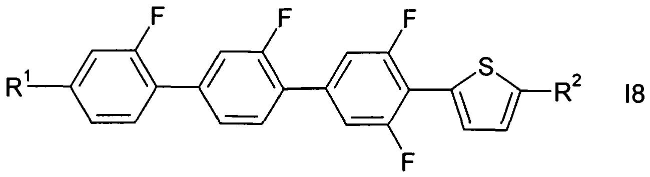 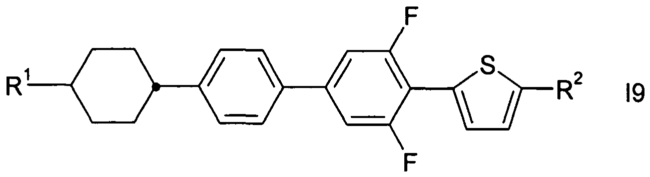 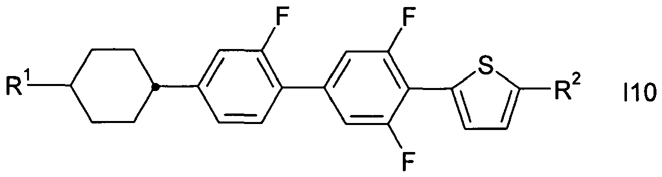 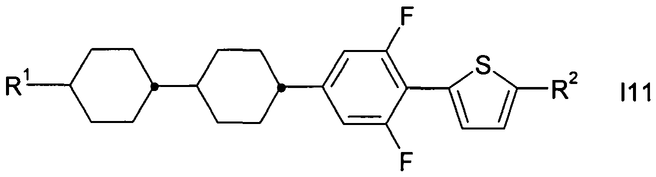 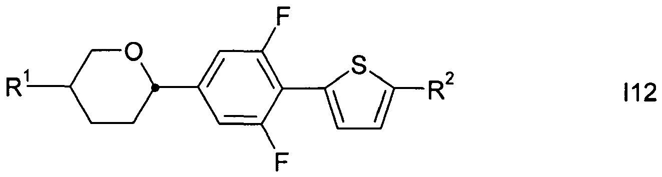 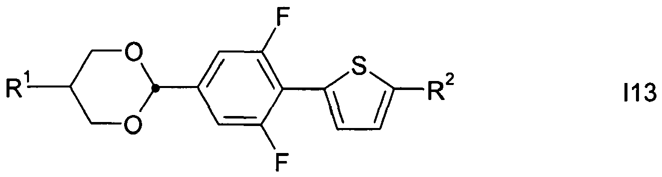 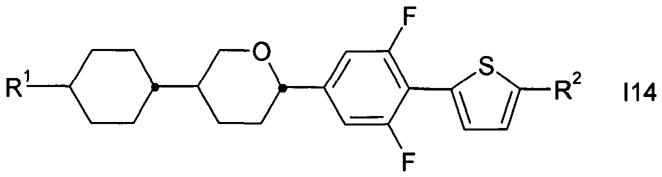 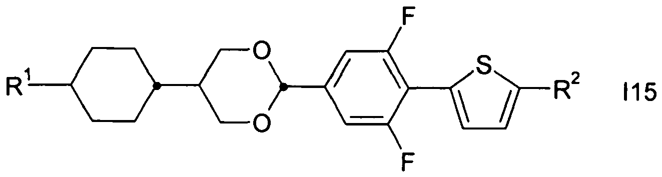 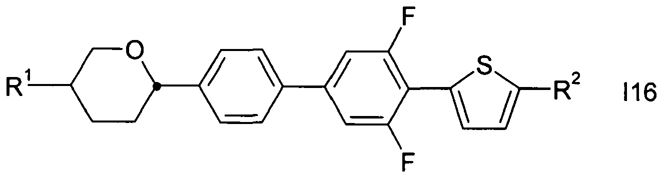 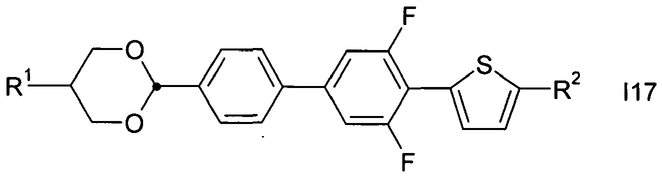 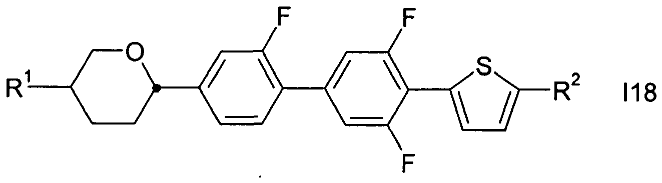 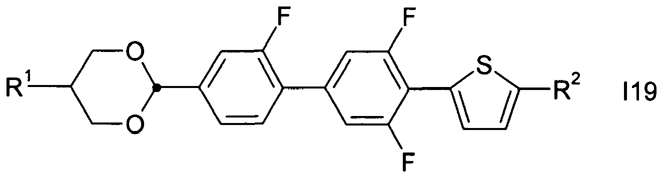 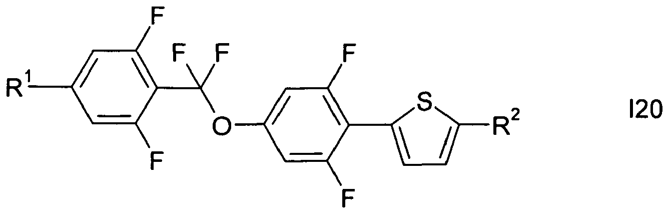 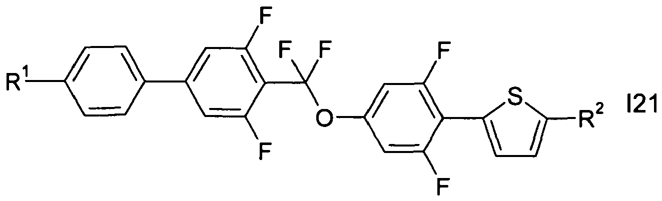 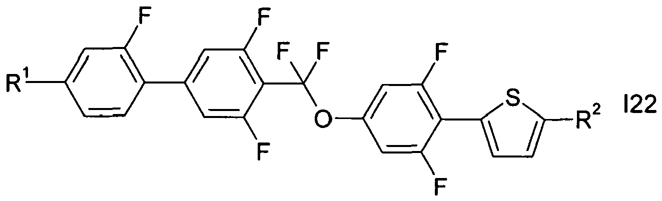 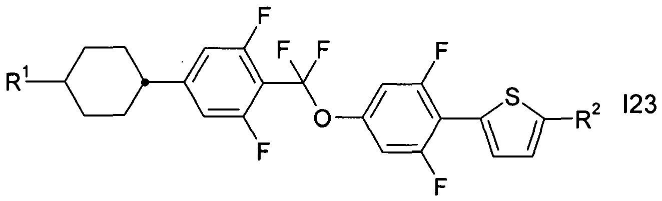 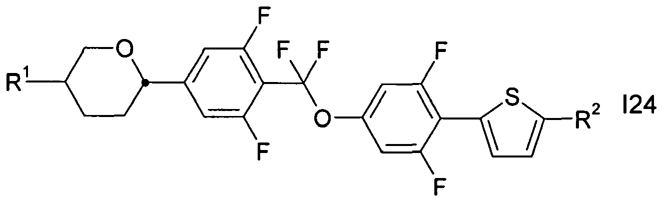 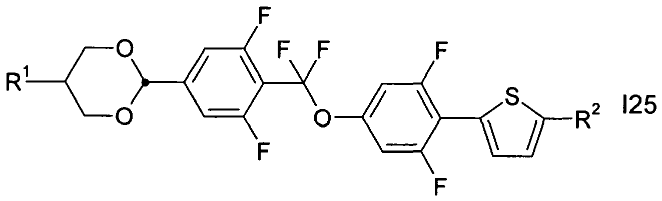 dans lesquelles R¹ et R² présentent les significations indiquées selon la revendication 1.

3. Composés selon la revendication 1 ou 2, **caractérisés en ce que**, dans la formule I, n représente 0, m représente 0 ou 1, et Z¹ représente une liaison simple.

4. Milieu LC qui présente une phase nématique à température ambiante, comprenant un ou plusieurs composés selon une ou plusieurs des revendications 1 à 3.

5. Milieu LC selon la revendication 4, **caractérisé en ce qu'**il comprend additionnellement un ou plusieurs composés de la formule II et/ou III 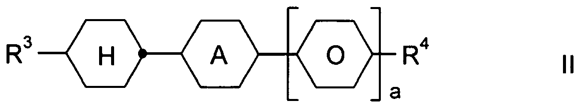 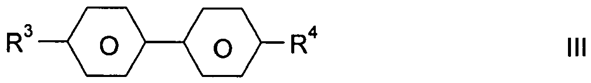 dans lesquelles
A représente 1,4-phénylène ou trans-1,4-cyclohexylène,
a est 0 ou 1,
R³ représente alkényle comportant 2 à 9 atomes de C, et
R⁴ représente alkyle comportant 1 à 12 atomes de C, où, en outre, un ou deux groupes CH₂ non adjacents peuvent être remplacés par -O-, -CH=CH-, -CO-, -OCO- ou -COO- de telle sorte que des atomes de O ne soient pas liés directement les uns aux autres, et de façon préférable, représente alkyle comportant 1 à 12 atomes de C ou alkényle comportant 2 à 9 atomes de C.

6. Milieu LC selon la revendication 4 ou 5, **caractérisé en ce qu'**il comprend additionnellement un ou plusieurs composés choisis parmi le groupe constitué par les formules qui suivent : 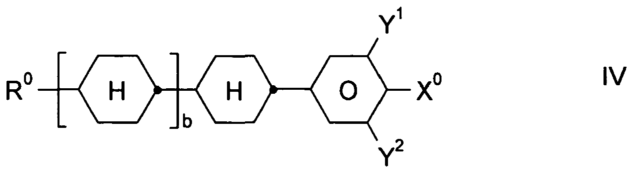 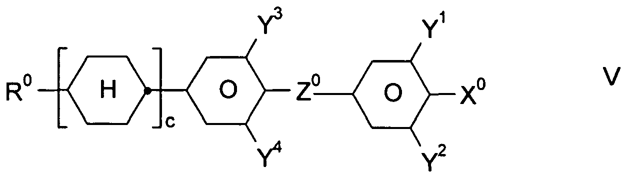 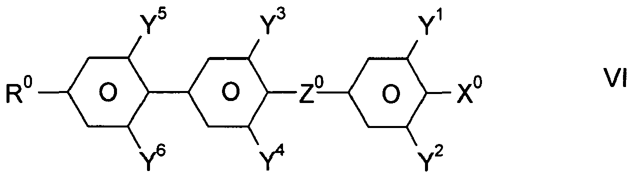 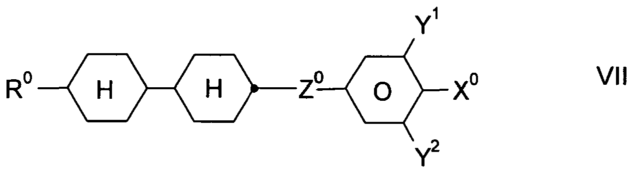 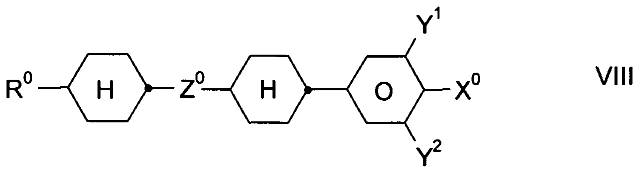 dans lesquelles
R⁰ représente un radical alkyle ou alcoxy comportant 1 à 15 atomes de C, où, en outre, un ou plusieurs groupes CH₂ dans ces radicaux peuvent chacun être remplacés, indépendamment les uns des autres, par -C≡C-, -CF₂O-, -CH=CH-, 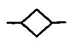 , 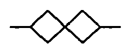 , -O-, -CO-O- ou -O-CO- de telle sorte que des atomes de O ne soient pas liés directement les uns aux autres, et où, en outre, un ou plusieurs atomes de H peuvent être remplacés par halogène,
X⁰ représente F, Cl, CN, SF₅, SCN, NCS, un radical alkyle halogéné, un radical alkényle halogéné, un radical alcoxy halogéné ou un radical alkényloxy halogéné, chacun comportant jusqu'à 6 atomes de C,
Y¹⁻⁶ représentent, chacun indépendamment des autres, H ou F,
Z⁰ représente -C₂H₄-, -(CH₂)₄-, -CH=CH-, -CF=CF-, -C₂F₄-, -CH₂CF₂-, -CF₂CH₂-, -CH₂O-, -OCH₂-, -COO-, -CF₂O- ou -OCF₂-, dans les formules V et VI également une liaison simple, et
b et c représentent, chacun indépendamment de l'autre, 0 ou 1.

7. Milieu LC selon une ou plusieurs des revendications 4 à 6, **caractérisé en ce qu'**il comprend additionnellement un ou plusieurs composés choisis parmi le groupe constitué par les formules qui suivent : 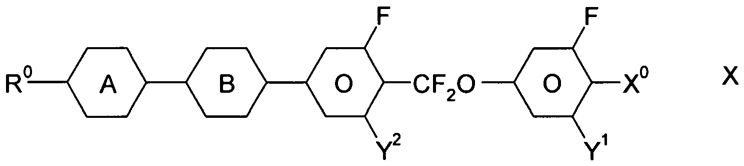 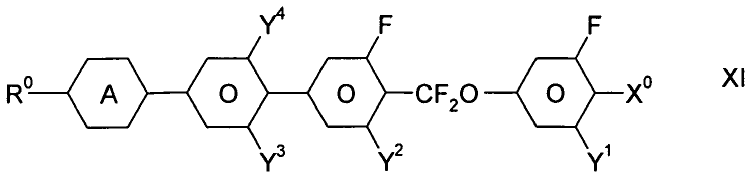 dans lesquelles R⁰, X⁰ et Y¹⁻⁴ présentent les significations indiquées selon la revendication 4, et 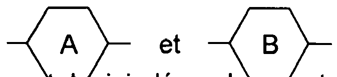 représentent, chacun indépendamment de inindépendamment de l'autre one another, indé

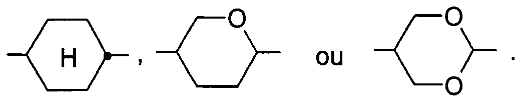

8. Milieu LC selon une ou plusieurs des revendications 4 à 7, **caractérisé en ce qu'**il comprend additionnellement un ou plusieurs composés de la formule qui suit : 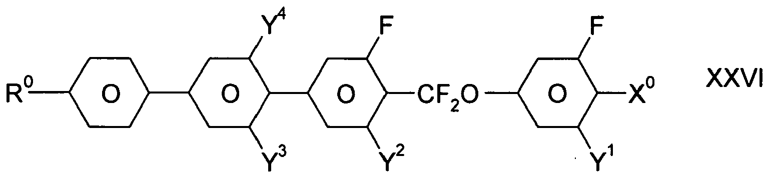 dans laquelle R⁰, X⁰ et Y¹⁻⁴ présentent les significations indiquées selon la revendication 7.

9. Procédé pour la préparation de composés de la formule I selon une ou plusieurs des revendications 1 à 3, **caractérisé en ce que**, au niveau d'un couplage de Suzuki favorisé par palladium, un bromothiophène substitué **2** 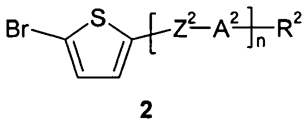 est amené à réagir avec un acide boronique **1** 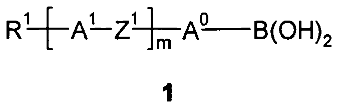 ou un ester d'acide boronique correspondant, ou un composé de type **10** 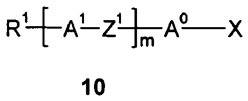 est amené à réagir avec un acide thiophèneboronique ou un sel d'acide thiophènehydroxyboronique correspondant **11** 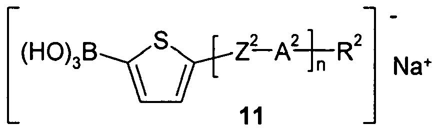 où R^{1,2}, A^{0,1,2}, Z^{1,2}, m et n présentent la signification indiquée selon la revendication 7, et X représente Br ou I.

10. Affichage LC contenant un milieu LC ou un ou plusieurs composés selon une ou plusieurs des revendications 1 à 8.

11. Affichage LC selon la revendication 10, **caractérisé en ce qu'**il s'agit d'un affichage MLC, TN, STN ou IPS.
